# EUROPEAN PATENT SPECIFICATION

(11) **EP 4 561 996 B1**
(45) Date of publication and mention of the grant of the patent: **24.06.2026**
(21) Application number: 23749045.3
(22) Date of filing: 28.07.2023
(51) Int. Cl.: C07D 295/10, C07D 487/08, C07D 413/12, A61K 31/495, A61K 31/496, A61P 25/00, A61P 25/04, A61P 25/16, A61P 25/28, A61P 35/00

(54) **[4-[5-(METHYLSULFONYL)-PHENYL]PIPERAZIN-1-YL]-(PHENYL)METHANONE DERIVATIVES AND SIMILAR COMPOUNDS WITH GFR ALPHA 1-RET ACTIVITY AS NEUROPROTECTIVE AGENTS FOR THE TREATMENT OF NEUROLOGICAL DISORDERS**
[4-[5-(METHYLSULFONYL)-PHENYL]PIPERAZIN-1-YL]-(PHENYL)METHANON-DERIVATE UND ÄHNLICHE VERBINDUNGEN MIT GFR ALPHA 1-RET AKTIVITÄT ALS NEUROPROTEKTIVE WIRKSTOFFE ZUR BEHANDLUNG VON NEUROLOGISCHEN STÖRUNGEN
DÉRIVÉS DE [4-[5-(MÉTHYLSULFONYL)-PHÉNYL]PIPÉRAZIN-1-YL]-(PHÉNYL)MÉTHANONE ET COMPOSÉS SIMILAIRES AVEC ACTIVITÉ GFR ALPHA 1-RET EN TANT QU'AGENTS NEUROPROTECTIVES POUR LE TRAITEMENT DE TROUBLES NEUROLOGIQUES

(30) Priority: 28.07.2022 EP 22187648
(43) Date of publication of application: 04.06.2025
(73) Proprietor: Genecode, 10151 Tallinn (EE)
(72) Inventor: CHARVIN, Delphine, 01170 GEX (FR); MIGNANI, Serge, 92290 CHATENAY-MALABRY (FR); MIEGE, Frédéric, 69003 LYON (FR); RODESCHINI, Vincent, 69780 MIONS (FR); RIBEILL, Yves, DURHAM, NC 27701 (US)
(74) Representative: Icosa
(86) International application number: PCT/EP2023/070973
(87) International publication number: WO 2024/023287

(56) References cited:
- WO-A2-2011/070177

## Description

### FIELD OF INVENTION

The present invention relates to novel polycyclic sulfones. The compounds of the invention are useful as neuroprotective and/or neurorestorative agents, in particular for use in the treatment of neurological disorders.

### BACKGROUND OF INVENTION

Neurological disorders (NDs) are heterogeneous diseases affecting the autonomic, peripheral and central nervous system of the body. Amongst the Central Nervous System (CNS) diseases, Alzheimer's disease (AD), Parkinson's disease (PD), Huntington's disease (HD), Amyotrophic Lateral Sclerosis (ALS), dementia, stroke, head trauma, brain tumor, pain and epilepsy are always the most challenging diseases to be addressed. Compounds actives for treating CNS might also be relevant to other diseases, for example diseases of the peripheral nervous system, eyes, spinal cord and enteric system.

The incidence of NDs is expected to increase dramatically in the 21^{st} century, in particular due to increased life expectancy and demographic changes. Some of these diseases are characterized by age-related gradual decline in neurological functions. In medicine, neurological diseases are the world's important and common cause of disability-adjusted life years, or years of healthy life lost due to death or disability. CNS diseases represent the largest and fastest growing therapeutic domain of unmet medical need, and it is being recognized as a global public health challenge and become a major global health priority. Adequate neurological diagnosis represents an immense challenge, and patients are more concerned about the development of new effective treatments to treat pathophysiology or symptoms. Neurological disorders affect millions of people worldwide and cause permanent damage. They are progressive diseases with symptoms that can degenerate overtime. Although there is generally no definitive cure, supporting treatments exist. The goal of these treatments is mainly to reduce symptoms and preserve the quality of life of the patient as long as possible.

Neurons are postmitotic cells that must live for a lifetime. While young neurons have proper functioning of self-healing protective mechanisms, aging or external or internal insults disturb them, eventually leading to neurodegeneration. These external/internal hazards are traumatic injuries or excitotoxic compounds, reactive oxygen species (ROS), protein aggregates, and other toxic molecules. Fortunately, cells have an intrinsic machinery that blocks death by activating resilience mechanisms or promoting regeneration pathways. Dysfunctionality or insufficiency of these self-healing mechanisms has also been described in neurodegenerative diseases.

Among natural self-healing agents, the glial cell line-derived neurotrophic factor (GDNF) acts as a potent neurotrophic factor, promoting survival in different neuronal populations such as spinal motor neurons, retinal cells, central noradrenergic neurons, or sympathetic neurons, among others. Likewise, GDNF (and other proteins of the GDNF family of neurotrophic factors such as neurturin, artemin and persephin) acts as a powerful trophic factor favoring, not only the survival and plasticity, but also the proliferation, differentiation, and protection of dopaminergic neurons, as well as the synthesis of dopamine and dopaminergic transmission in the developing and adult brain. GDNF can promote neuroprotection through MAP kinase/ERK, Src kinase and PI3 kinase/AKT pathways by inducing several neuroprotective signaling cascade, including the activation of the transcription factor Elk1 through the activation of the GFRα1-RET receptor complex.

The field of applications for these proteins is vast. Pre-clinical and clinical trials have been carried out to evaluate the effect of neurotrophic factors of the GDNF family for the prevention, treatment or management of Parkinson's disease, chronic pain, Alzheimer's disease, amyotrophic lateral sclerosis, neuropathy, depression, stroke, and these proteins were even suggested as male contraceptives. However, the clinical application of GDNF is hampered by its poor pharmacokinetic properties, the fact that it does not cross the blood-brain barrier and thus the necessity for intracranial delivery via stereotaxic surgery, varying biological activity, and high price.

Blood-brain barrier penetrating small-molecule compounds that target the GDNF receptor complex and mimic GDNF biological effects in neurons may be an avenue to overcome these issues and translate to greater efficacy in the clinic. Superior tissue penetration of such compounds could promote survival in all affected neuronal pathways.

WO 2011/070177 A2 (BALTIC TECHNOLOGY DEV LTD) discloses polycyclic compounds that are sulfonamide derivatives. Said compounds modulate GFRα1-RET activity and are for use in the treatment of neurological or neurodegenerative disorders.

It was surprisingly found out by the Applicants that novel polycyclic sulfones of formula (I) showed potent GFRα1-RET activity in a luciferase assay, thereby opening the way to overcoming the limitations of available therapeutic solutions.

### SUMMARY

An object of the present invention is a compound of formula (I) 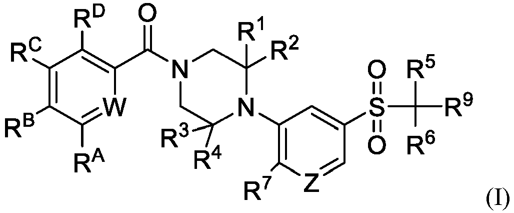 or a pharmaceutically acceptable salt and/or solvate thereof;
wherein **W, R^{A}-R^{D}, R¹-R⁴, Z, R⁵-R⁷** and **R⁹** are as defined hereinafter and/or as defined in the claims.

According to one embodiment, the compound is selected from the compounds of **Table 1** herein, and pharmaceutically acceptable salts and/or solvates thereof.

Another object of the present invention is a pharmaceutical composition comprising a compound according to the invention and at least one pharmaceutically acceptable carrier.

Another object of the present invention is a compound according to the invention or a pharmaceutical composition according to the invention for use as a medicament. According to one embodiment, the compound or the pharmaceutical composition is for use in the treatment of a neurological disorder.

Another object of the present invention is a process for manufacturing a compound according to the invention.

### DEFINITIONS

In the present invention, the following terms have the following meanings:

### Chemical definitions

Where chemical substituents are combinations of chemical groups, the point of attachment of the substituent to the molecule is by the last chemical group recited on the right of the name of the substituent. For example, an arylalkyl substituent is linked to the rest of the molecule through the alkyl moiety and it may by represented as follows: "aryl-alkyl-".

Unless otherwise indicated, the compounds were named using BIOVIA Draw 2021 (Dassault, France).

The definitions herein referring to the optional or mandatory substitution of a specified group apply both to the substituted group considered as such or to the same group comprised in another chemical moiety, which can both be substituted as set forth herein. For example, "***R^{x}** represents hydrogen, (C₁-C₈) alkyl, (C₁-C₈) alkyl-O- or cycloalkyl-(C₁-C₈) alkyl-NH-; wherein the alkyl is optionally substituted by at least one F"* means that any alkyl group present in the structure of **R^{x}** may optionally substituted by at least one F, including said (C₁-C₈) alkyl as such (*e.g.,* CF₃), the alkyl comprised in said (C₁-C₈) alkyl-O- (*e.g.,* OCF₃) and the alkyl comprised in said cycloalkyl-(C₁-C₈) alkyl-NH- (*e.g.*, cyclopropyl-CH₂-CHF-CH₂-NH-).

**"Alkoxy"** refers to an alkyl-O- group.

**"Alkyl"** refers to a saturated linear or branched hydrocarbon chain, typically comprising from 1 to 16 carbon atoms, preferably from 1 to 12 carbon atoms, more preferably from 1 to 8 carbon atoms, furthermore preferably from 1 to 6 carbon atoms. Alkyl groups may be monovalent or polyvalent (*i.e.,* **"alkylene"** groups, which are divalent alkyl groups, are encompassed in "alkyl" definition). Non-limiting examples of alkyl groups include methyl, ethyl, *n*-propyl, *i*-propyl, *n*-butyl, *i*-butyl, *s*-butyl and *t*-butyl, pentyl and its isomers (*e.g., n*-pentyl, *iso*-pentyl), and hexyl and its isomers (*e.g., n*-hexyl, *iso*-hexyl). Particular examples of alkyl groups include methyl, ethyl, *n*-propyl, *i*-propyl, *n*-butyl, *s*-butyl and *t*-butyl (including methylene, ethylene, *n*-propylene, *n*-butylene and *n*-butylene).

**"Amine"** refers to derivatives of ammonia (NH₃), wherein one or more hydrogen atoms have been replaced by a substituent such as, for example, alkyl or aryl. **"Amino"** refers to the -NH₂ group.

**"Aryl"** refers to a cyclic, polyunsaturated, aromatic hydrocarbyl group comprising at least one aromatic ring and comprising from 5 to 12 carbon atoms, preferably from 6 to 10 carbon atoms. Aryl groups may be monovalent or polyvalent (*e.g.,* divalent). Aryl groups may have a single ring (*e.g.,* phenyl) or multiple aromatic rings fused together (*e.g.*, naphthyl) or linked covalently. The aromatic ring may optionally include one to two additional rings (either cycloalkyl, heterocycloalkyl or heteroaryl) fused thereto. This definition of "aryl" encompasses the partially hydrogenated derivatives of the carbocyclic systems enumerated herein, as long as at least one ring is aromatic. Aryls may optionally be substituted by at least one group such as, for example, halogen (*e.g.,* F or Cl), (C₁-C₈) alkyl (*e.g.,* methyl) or nitrile (CN). Non-limiting examples of aryl groups include phenyl, biphenyl, biphenylenyl, 5- or 6-tetralinyl, naphthalen-1- or -2-yl, 4-, 5-, 6 or 7-indenyl, 1- 2-, 3-, 4- or 5-acenaphthylenyl, 3-, 4- or 5-acenaphthenyl, 1- or 2-pentalenyl, 4- or 5-indanyl, 5-, 6-, 7- or 8-tetrahydronaphthyl, 1,2,3,4-tetrahydronaphthyl, 1,4-dihydronaphthyl, and 1-, 2-, 3-, 4- or 5-pyrenyl. A particular example of aryl group is phenyl.

**"Benzylidene"** refers to a phenyl group bond to a moiety through an *exo* carbon-carbon double bound, *i.e.,* =CH-Ph bond to a carbon atom. The moiety is typically cyclic such as, for example, an heterocycloalkyl.

**"Cycloalkyl"** refers to a cyclic alkyl group, typically comprising from 3 to 15 carbon atoms, preferably from 3 to 12 carbon atoms, more preferably from 3 to 8 carbon atoms, further more preferably from 3 to 6 carbon atoms. Cycloalkyl groups may be monovalent or polyvalent (*e.g*., divalent). This definition of "cycloalkyl" encompasses polycyclic cycloalkyls (*e.g.*, bicycles) and bridged cycloalkyl structures, including cycles bound together through one atom ("spiro") or through two atoms. This definition of "cycloalkyl" encompasses cycloalkyls including a cyclic alkyl group substituted by at least one non-cyclic alkyl, such as, for example, a (C₁-C₈) alkyl (preferably, (C₁-C₄) alkyl, (*e.g.,* methyl). Non-limiting examples of cycloalkyl groups include cyclopropyl, cyclobutyl, cyclopentyl, cyclohexyl, cycoheptyl, cyclooctanyl, cyclononanyl, cyclodecanyl, norbornyl, adamantyl, bicyclo[2.2.2]octanyl, bicyclo[4.4.0]decanyl, bicyclo[3.2.1]octanyl, bicyclo[3.3.1]nonanyl, bicyclo[2.1.1]hexane, 2,3-dihydro-1H-indenyl, 1,2,3,4-tetrahydronaphthalenyl, decahydronaphthalenyl, 1,2,3,4-tetrahydronaphthalenyl, and octahydropentalenyl.

"**Cₓ-C_{y}**" or "**(Cₓ-C_{y})**" preceding the name of a group means that the group comprises from x to y carbon atoms, in accordance to common terminology in the chemistry field.

**"Halogen"** refers to a fluorine, chlorine, bromine or iodine atom.

**"Heteroalkyl"** refers to an alkyl group as defined herein, wherein one or more carbon atoms are replaced by a heteroatom selected from oxygen, nitrogen and sulfur, and wherein the resulting heteroalkyl group comprises at least one carbon atom. In heteroalkyl groups, the heteroatoms are bound along the alkyl chain only to carbon atoms, *i.e.,* each heteroatom is separated from any other heteroatom by at least one carbon atom, typically by at least two carbon atoms. Heteroalkyl groups may be monovalent or polyvalent (*e.g*., divalent). The nitrogen and sulfur heteroatoms may optionally be oxidized and the nitrogen heteroatoms may optionally be quaternized (*e.g.,* sulfur may be oxidized as SO or SO₂). Heteroalkyl groups may further include one or more =O and/or =S groups. In one embodiment, at least two carbon atoms are replaced by a heteroatom. In one embodiment, the heteroalkyl is bound to another group or molecule through a carbon atom, *i.e.,* the binding atom is not selected among the heteroatoms included therein. In one embodiment, the heteroalkyl is bound to another group or molecule through one of the heteroatoms included therein. When substituted by one or more other group(s), an heteroalkyl may be substituted either through a carbon atom or through a heteroatom (*e.g.,* nitrogen), unless otherwise specified. Non-limiting examples of heteroalkyl include alkoxy, ethers and polyethers (*e.g.,* polyethylene glycol), secondary and tertiary amines and polyamines, thioethers and polythioethers, and combinations thereof.

**"Heteroaryl"** refers to aromatic rings or aromatic ring systems comprising from 5 to 15 carbon atoms, preferably from 4 to 12 carbon atoms, more preferably from 3 to 10 carbon atoms, having one or two rings that are fused together or linked covalently, wherein at least one ring is aromatic, and wherein one or more carbon atoms in one or more of these rings is replaced by oxygen, nitrogen and/or sulfur atoms. Heteroaryl groups may be monovalent or polyvalent (*e.g.,* divalent). The nitrogen and sulfur heteroatoms may optionally be oxidized and the nitrogen heteroatoms may optionally be quaternized (*e.g.,* the heteroatom is substituted by oxo (=O) for sulfur atom or (→O) for nitrogen atom). This definition of "heteroaryl" encompasses the partially hydrogenated derivatives of the carbocyclic systems enumerated herein, as well as ring systems including one or more fused non-aromatic cycloalkyl and/or heterocycloalkyl ring(s), as long as at least one ring is aromatic. In one embodiment, the heteroaryl is bound to another group or molecule through a carbon atom, *i.e.,* the binding atom is not selected among the heteroatoms included therein. In one embodiment, the heteroaryl is bound to another group or molecule through one of the heteroatoms included therein. When substituted by one or more other group(s), an heteroaryl may be substituted either through a carbon atom or through a heteroatom (*e.g.,* nitrogen), unless otherwise specified. Heteroaryls may optionally be substituted by at least one group such as, for example, halogen (*e.g.,* F or Cl), (C₁-C₈) alkyl (preferably, (C₁-C₄) alkyl, *e.g*., methyl) or nitrile (CN). Non-limiting examples of heteroaryl groups include pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, tetrazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-l(6H)-yl, 2-oxopyridin-l(2H)-yl, 6-oxo-pyridazin-l(6H)-yl, 2-oxopyridin-l(2H)-yl, 1,3-benzodioxolyl, quinolinyl, isoquinolinyl, cinnolinyl, quinazolinyl and quinoxalinyl. Non-limiting examples of heteroaryl groups comprising at least one fused non-aromatic ring include 2,3-dihydrobenzofuranyl, benzo*[d]*[1,3]dioxolyl, indolinyl, 2,3-dihydrobenzo*[b]*[1,4]dioxinyl, 3,4-dihydro-2*H*-benzo*[b]*[1,4]oxazinyl, 1,2,3,4-tetrahydroquinoxaline, 3,4-dihydro-*2H*-benzo[b][1,4]thiazine and 2,3-dihydrobenzo[b][1,4]oxathiine.

**"Heteroarylidene"** refers to a heteroaryl group bond to a moiety through an *exo* carbon-carbon double bound, *i.e.,* =CH-heteroaryl bond to a carbon atom. The moiety is typically cyclic such as, for example, an heterocycloalkyl.

**"Heterocycloalkyl"** refers to a cyclic heteroalkyl group, typically comprising from 2 to 15 carbon atoms, preferably from 2 to 11 carbon atoms, more preferably from 2 to 7 carbon atoms, furthermore preferably from 2 to 6 carbon atoms. Heterocycloalkyl groups may be monovalent or polyvalent (*e.g*., divalent). Heterocycloalkyl groups are typically 3- to 7-membered, preferably 5- or 6-membered. Heterocycloalkyl are typically monocyclic or bicyclic, preferably monocyclic. This definition encompasses polycyclic heterocycloalkyls (*e.g.*, bicycles) and bridged heterocycloalkyl structures, including cycles bound together through one atom ("spiro") or through two atoms. The nitrogen and sulfur heteroatoms may optionally be oxidized and the nitrogen heteroatoms may optionally be quaternized (*e.g*., the heteroatom is substituted by oxo (=O) for sulfur atom or (→O) for nitrogen atom). In one embodiment, the heterocycloalkyl is bound to another group or molecule through a carbon atom, *i.e.,* the binding atom is not selected among the heteroatoms included therein. In one embodiment, the heterocycloalkyl is bound to another group or molecule through one of the heteroatoms included therein. When substituted by one or more other group(s), an heterocycloalkyl may be substituted either through a carbon atom or through a heteroatom (*e.g.,* nitrogen), unless otherwise specified. Heterocycloalkyls may optionally be substituted by at least one group such as, for example, halogen (*e.g.,* F or Cl), (C₁-C₈) alkyl (preferably, (C₁-C₄) alkyl, *e.g.,* methyl), nitrile (CN) or =O. Non-limiting examples of heterocycloalkyl include aziridine, pyrrolidine, piperidine, piperazine, morpholine, thiomorpholine, azepane, azocane, octahydro-1*H*-isoindole, decahydroisoquinoline, tetrahydrofuran, tetrahydropyran, tetrahydroisoquinoline (*e.g.*, 1,2,3,4-tetrahydroisoquiline), hexahydropyridazine, hexahydropyrazine, hexahydropyrimidine, decahydroquinoline, octahydropyrrolo[3,4-c]pyrrole, isoindoline, 1,2,3,4-tetrahydroquinoline and oxetane.

**"Prodrug"** refers to a pharmacologically acceptable derivative of a therapeutic agent (*e.g.,* a compound according to the invention) whose *in vivo* biotransformation product is the therapeutic agent (active drug). Prodrugs are typically characterized by increased bioavailability and are readily metabolized *in vivo* into the active compounds. Non-limiting examples of prodrugs include amide prodrugs and carboxylic acid ester prodrugs.

**"Solvate"** refers to molecular complex comprising a compound along with stoichiometric or sub-stoichiometric amounts of one or more molecules of one or more solvents, typically the solvent is a pharmaceutically acceptable solvent such as, for example, ethanol. The term **"hydrate"** refers to a solvate when the solvent is water (H₂O).

**"Ylidene"** refers to CH group involved in an *exo* carbon-carbon double bound with another moiety. The moiety is typically cyclic such as, for example, an heterocycloalkyl.

### General definitions

**"About"** is used herein to mean approximately, roughly, around, or in the region of. The term "about" preceding a figure means plus or less 10 % of the value of the figure. When the term "about" is used in conjunction with a numerical range, it modifies that range by extending the boundaries above and below the numerical values set forth by 10%.

**"Administration",** or a variant thereof (*e.g.,* **"administering"),** means providing a therapeutic agent alone or as part of a pharmaceutically acceptable composition, to the patient in whom/which the condition, symptom, or disease is to be treated.

**"Comprise"** or a variant thereof (*e.g*., "comprises", "comprising") is used herein according to common patent application drafting terminology. Hence, "comprise" preceded by an object and followed by a constituent means that the presence of a constituent in the object is required (typically as a component of a composition), but without excluding the presence of any further constituent(s) in the object. Moreover, any occurrence of "comprise" or a variant thereof herein also encompasses narrower expression "substantially consist of", further narrower expression "consist of" and any variants thereof (*e.g.*, "consists of", "consisting of"), and may be replaced thereby, unless otherwise stated.

**"GDNF family receptor alpha-1",** "**GFRα1**", or "**GDNFRα1**", also named "RET ligand 1" or "TGF-beta-related neurotrophic factor receptor 1", is a protein from the GDNFR family that acts as a receptor for GDNF. It mediates the GDNF-induced autophosphorylation and activation of the RET receptor. In humans, GFRα1 is encoded by the *GFRA1* gene. An exemplary amino acid sequence of human GFRα1 is given in SEQ ID NO: 1, in which amino acid residues 1-24 correspond to the signal peptide and amino acid residues 430-465 correspond to the propeptide which is removed in mature form.

| |
|---|
| **SEQ ID NO: 1** |
| MFLATLYFALPLLDLLLSAEVSGGDRLDCVKASDQCLKEQSCSTKYRTLRQCV AGKETNFSLASGLEAKDECRSAMEALKQKSLYNCRCKRGMKKEKNCLRIYWS MYQSLQGNDLLEDSPYEPVNSRLSDIFRVVPFISDVFQQVEHIPKGNNCLDAAK ACNLDDICKKYRSAYITPCTTSVSNDVCNRRKCHKALRQFFDKVPAKHSYGML FCSCRDIACTERRRQTIVPVCSYEEREKPNCLNLQDSCKTNYICRSRLADFFTNC QPESRSVSSCLKENYADCLLAYSGLIGTVMTPNYIDSSSLSVAPWCDCSNSGND LEECLKFLNFFKDNTCLKNAIQAFGNGSDVTVWQPAFPVQTTTATTTTALRVK NKPLGPAGSENEIPTHVLPPCANLQAQKLKSNVSGNTHLCISNGNYEKEGLGAS SHITTKSMAAPPSCGLSPLLVLVVTALSTLLSLTETS |

**"Human"** refers to a male or female human subject at any stage of development, including neonate, infant, juvenile, adolescent and adult.

**"Neuroprotective"** refers to the protection of a neuronal cell from insults, events, or conditions that would normally result in a loss of neuronal cell's functions, and ultimately, neuronal cell death. Such insults, events, or conditions include, without limitation, neuronal stress, for instance, caused by hypoxia or ischemia; traumatic injuries; and exposure to toxic molecules, for instance, to abnormal misfolded proteins, protein aggregates, excitotoxins, reactive oxygen species, endoplasmic reticulum stressors, mitochondrial stressors, Golgi apparatus antagonists and the like. The term also characterizes the detectable biological activity of a compound in reducing the amount or level of neuronal cell's loss of functions and/or neuronal cell death.

**"Neurorestorative"** refers to the restoration or rescue of a neuronal cell and in particular of its functions, from the effect of an insult, event, or condition that would normally result in a loss of neuronal cell's functions if not in neuronal cell death.

**"Patient"** refers to a subject who/which is awaiting the receipt of, or is receiving medical care or was/is/will be the object of a medical procedure, or is monitored for the development of the targeted disease or condition, such as, for example, a neurological disorder.

**"Pharmaceutically acceptable"** means that the ingredients of a composition are compatible with each other and not deleterious to the subject to which/whom it is administered.

**"Pharmaceutically acceptable carrier"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, *e.g.,* FDA Office or EMA. Examples of pharmaceutically acceptable carriers include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

**"Pharmaceutical composition"** refers to a composition comprising at least one therapeutic agent (*e.g.,* a compound according to the present invention) and at least one pharmaceutically acceptable carrier.

**"Proto-oncogene tyrosine-protein kinase receptor Ret",** or in short **"RET",** also named "cadherin family member 12", is a receptor tyrosine kinase involved in numerous cellular mechanisms including cell proliferation, neuronal navigation, cell migration, and cell differentiation. RET is activated upon (i) binding of a neurotrophic factor of the GDNF family (*e.g.*, GDNF, neurturin, artemin or persephin) to a receptor of the GDNFR family (*e.g.*, GFRα1, GFRα2, GFRα3 or GFRα4), then (ii) complex formation between RET and the receptor of the GDNFR family, (iii) dimerization and (iv) trans-autophosphorylation. An exemplary amino acid sequence of human RET is given in SEQ ID NO: 2, in which amino acid residues 1-28 correspond to the signal peptide.

| |
|---|
| **SEQ ID NO: 2** |
| |

**"Selected from"** is used herein according to common patent application drafting terminology, to introduce a list of elements among which one or more item(s) is (are) selected. Any occurrence of "selected from" in the specification may be replaced by "selected from the group comprising or consisting of" and reciprocally without changing the meaning thereof.

**"Subject"** refers to an animal, typically a warm-blooded animal, preferably a mammal, more preferably a primate, furthermore preferably a human. In one embodiment, the subject is a **"patient"** as defined herein. In one embodiment, the subject is affected, preferably is diagnosed, with a disease. In one embodiment, the subject is at risk of developing a disease. Examples of risks factor include, but are not limited to, genetic predisposition, or familial history of the disease.

**"Therapeutic agent", "active pharmaceutical ingredient"** and **"active ingredient"** refer to a compound for therapeutic use and relating to health. Especially, a therapeutic agent (*e.g.,* a compound according to the present invention) may be indicated for treating a disease (*e.g.,* a neurological disorder). An active ingredient may also be indicated for improving the therapeutic activity of another therapeutic agent.

**"Therapeutically effective amount"** (in short **"effective amount")** refers to the amount of a therapeutic agent (*e.g.,* a compound according to the present invention) that is sufficient to achieve the desired therapeutic, prophylactic or preventative effect in the patient to which/whom it is administered, without causing significant negative or adverse side effects to said patient. A therapeutically effective amount may be administered prior to the onset of the disease for a prophylactic or preventive action. Alternatively, or additionally, the therapeutically effective amount may be administered after initiation of the disease for a therapeutic action.

**"Treating", "treatment"** or **"alleviation"** refers to both therapeutic treatment and prophylactic or preventative measures, wherein the object is to prevent or slow down (lessen) the targeted pathologic condition or disorder (herein a **"disease")** (*e.g.,* a neurological disorder). Those in need of treatment include those already with the disease as well as those prone to have the disease or those in whom the condition or disease is to be prevented. A patient is successfully "treated" for a disease if, after receiving a therapeutic amount of a therapeutic agent (*e.g.,* a compound according to the present invention), the patient shows observable and/or measurable reduction in or absence of one or more of the following: reduction in the number of pathogens; reduction in the percent of total cells that are pathogenic; and/or relief to some extent, one or more of the symptoms associated with the specific disease; reduced morbidity and mortality, and improvement in quality of life issues. The above parameters for assessing successful treatment and improvement in the disease are readily measurable by routine procedures familiar to a physician.

### DETAILED DESCRIPTION

### Compound

An object of the present invention is a compound of formula (I) 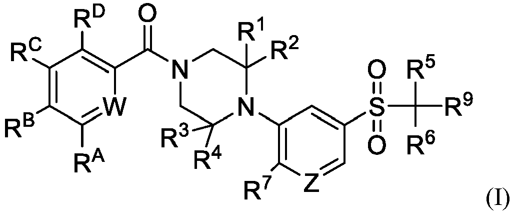 or a pharmaceutically acceptable salt and/or solvate thereof;
wherein
**W** represents CH or N;
**R^{A}**, **R^{B}, R^{C}** and **R^{D}** each independently represents hydrogen, F, Cl, CH₃, CF₃, CHF₂ or CH₂F,
   *provided that* at least one among R^{A}, R^{B}, R^{C} and R^{D} does not represent hydrogen;
**R¹** represents hydrogen or (C₁-C₈) alkyl, wherein the alkyl is optionally substituted by at least one OH, (C₁-C₃) alkoxy or F; and **R²**, **R³** and **R⁴** represents hydrogen;
or **R¹** and **R⁴** form together -CH₂-O-CH₂- or -CH₂-CH₂-, wherein the -CH₂-CH₂- is optionally substituted by at least one F, OH or OCH₃; and **R²** and **R³** each represents hydrogen;
**R⁷** represents hydrogen, OH, halogen, (C₁-C₈) alkyl, cycloalkyl, (C₁-C₈) alkyl-O-, cycloalkyl-O-, cycloalkyl-(C₁-C₈) alkyl-O-, heterocycloalkyl-O-, R¹¹O-(C₁-C₈) alkyl-O-, R¹¹R¹²N-(C₁-C₈) alkyl-O-, (R¹¹O)(R¹²)N-(C₁-C₈) alkyl-O-, R¹¹R¹²N-(C₁-C₈) alkyl-, R¹¹O-(C₁-C₈) alkyl-, NR¹¹R¹², CN, CO₂H, CO₂R¹¹, CONH₂, CON(R¹¹)H, heterocycloalkyl, or heteroaryl; wherein **R¹¹** and **R¹²** each independently represents hydrogen or (C₁-C₈) alkyl;
   wherein the alkyl or cycloalkyl in **R⁷** is optionally substituted by at least one F, Cl, OH, =O, (C₁-C₈) alkyl, (C₁-C₈) alkyl-O-, heterocycloalkyl, aryl or heteroaryl;
   wherein the heterocycloalkyl, aryl or heteroaryl is optionally substituted by at least one F, Cl, (C₁-C₈) alkyl, CF₃, CHF₂, CH₂F, OCF₃, CN, OH, =O, →O, (C₁-C₈) alkoxy, NR¹³R¹⁴, R¹³R¹⁴N-(C₁-C₈) alkyl-, R¹³O₂C-(C₁-C₈) alkyl-, CO₂H, R¹³R¹⁴N-C(O)-, R¹³O-NR¹⁴-, or (C₁-C₈) alkyl-CO₂-; wherein **R¹³** and **R¹⁴** each independently represents hydrogen or (C₁-C₈) alkyl;
**Z** represents C-H, C-**R⁸** or N;
   wherein **R⁸** represents (C₁-C₄) alkyl, F, Cl, CF₃, CHF₂, CH₂F, OCF₃, CN, OH or (C₁-C₄) alkoxy;
or **Z** represents C-**R⁸** and **R⁷** and **R⁸** form together with the carbon atoms to which they are bound a cycloalkyl or heterocycloalkyl,
   wherein the cycloalkyl or heterocycloalkyl is optionally substituted by at least one F, OH, =O, →O, (C₁-C₈) alkyl, CF₃, HO₂C-CH₂-, (C₁-C₄) alkyl-CO₂-CH₂-, R¹⁵R¹⁶N-CH₂-, aryl, or aryl-(C₁-C₈) alkyl-, wherein **R¹⁵** and **R¹⁶** each independently represents hydrogen or (C₁-C₈) alkyl;
**R⁵** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
   wherein the alkyl or cycloalkyl in **R⁵** is optionally substituted by at least one F, Cl, (C₁-C₈) alkyl, CF₃, OCF₃, CN, OH, =O, (C₁-C₈) alkoxy, NR¹⁷R¹⁸, CO₂H, R¹⁷R¹⁸N-C(O)-, R¹⁷O-NR¹⁸-, heterocycloalkyl, aryl or heteroaryl;
   wherein **R¹⁷** and **R¹⁸** each independently represents hydrogen or (C₁-C₈) alkyl;
   wherein the heterocycloalkyl, aryl or heteroaryl (*i.e.,* any heterocycloalkyl, aryl or heteroaryl that is represented by **R⁵** or that is part of any substituent thereof) is optionally substituted by at least one F, Cl, (C₁-C₈) alkyl, CF₃, CHF₂, CH₂F, OCF₃, CN, OH, =O, →O, (C₁-C₈) alkoxy, NR¹⁹R²⁰, CO₂H, R¹⁹R²⁰N-C(O)-, R¹⁹O-NR²⁰-, (C₁-C₈) alkyl-CO₂-, R¹⁹R²⁰N-(C₁-C₈) alkyl-, R¹⁹O₂C-(C₁-C₈) alkyl-, heterocycloalkyl, heteroaryl, aryl, or aryl-(C₁-C₈) alkyl-; wherein **R¹⁹** and **R²⁰** each independently represents hydrogen or (C₁-C₈) alkyl;
**R⁶** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
   wherein the alkyl or cycloalkyl in **R⁶** is optionally substituted by at least one F, Cl, (C₁-C₈) alkyl, CF₃, OCF₃, CN, OH, =O, (C₁-C₈) alkoxy, NR²¹R²², CO₂H, R²¹R²²N-C(O)-, R²¹O-NR²²-, heterocycloalkyl, aryl or heteroaryl;
   wherein **R²¹** and **R²²** each independently represents hydrogen or (C₁-C₈) alkyl;
   wherein the heterocycloalkyl, aryl or heteroaryl (*i.e.,* any heterocycloalkyl, aryl or heteroaryl that is represented by **R⁶** or that is part of any substituent thereof) is optionally substituted by at least one F, Cl, (C₁-C₈) alkyl, CF₃, CHF₂, CH₂F, OCF₃, CN, OH, =O, →O, (C₁-C₈) alkoxy, NR²³R²⁴, CO₂H, R²³R²⁴N-C(O)-, R²³O-NR²⁴-, (C₁-C₈) alkyl-CO₂-, R²³R²⁴N-(C₁-C₈) alkyl-, R²³O₂C-(C₁-C₈) alkyl-, heterocycloalkyl, heteroaryl, aryl, or aryl-(C₁-C₈) alkyl-; wherein **R²³** and **R²⁴** each independently represents hydrogen or (C₁-C₈) alkyl;
or **R⁵** and **R⁶** form together with the carbon atom to which they are bound a cycloalkyl or heterocycloalkyl,
   wherein the cycloalkyl or heterocycloalkyl (*i.e.,* the cycloalkyl or heterocycloalkyl that is formed by **R⁵**, **R⁶** and the carbon atom to which they are bound) is optionally substituted by at least one F, Cl, (C₁-C₈) alkyl, CF₃, OCF₃, CN, OH, =O, →O, (C₁-C₈) alkoxy, NR²⁵R²⁶, CO₂H, (C₁-C₈) alkyl-CO₂-, R²⁵R²⁶N-C(O)-, R²⁵O-NR²⁶-, R²⁵R²⁶N-(C₁-C₈) alkyl-, R²⁵O₂C-(C₁-C₈) alkyl-, cycloalkyl, heterocycloalkyl, aryl, heteroaryl, cycloalkyl-(C₁-C₈) alkyl-, heterocycloalkyl-(C₁-C₈) alkyl-, aryl-(C₁-C₈) alkyl-, heteroaryl-(C₁-C₈) alkyl-, aryl-cycloalkyl-, cycloalkyl-O-, heterocycloalkyl-O-, aryl-O-, heteroaryl-O-, cycloalkyl-(C₁-C₈) alkyl-O-, heterocycloalkyl-(C₁-C₈) alkyl-O-, aryl-(C₁-C₈) alkyl-O-, heteroaryl-(C₁-C₈) alkyl-O-, cycloalkyl-NR²⁵-, heterocycloalkyl-NR²⁵-, aryl-NR²⁵-, heteroaryl-NR²⁵-, cycloalkyl-(C₁-C₈) alkyl-NR²⁵-, heterocycloalkyl-(C₁-C₈) alkyl-NR²⁵-, aryl-(C₁-C₈) alkyl-NR²⁵-, heteroaryl-(C₁-C₈) alkyl-NR²⁵-, benzylidene, heteroarylidene, aryl-(C₁-C₈) alkyl-ylidene- or heteroaryl-(C₁-C₈) alkyl-ylidene-;
   wherein **R²⁵** and **R²⁶** each independently represents hydrogen or (C₁-C₈) alkyl;
   wherein the heterocycloalkyl, aryl, heteroaryl, benzylidene or heteroarylidene (*i.e.,* any heterocycloalkyl, aryl, heteroaryl, benzylidene or heteroarylidene that is part of any substituent of the heterocycloalkyl formed by **R⁵**, **R⁶** and the nitrogen atom to which they are bound) is optionally substituted at least one F, Cl, (C₁-C₈) alkyl, CF₃, CHF₂, CH₂F, OCF₃, CN, OH, =O, →O, (C₁-C₈) alkoxy, NR²⁷R²⁸, CO₂H, R²⁷R²⁸N-C(O)-, R²⁷O-NR²⁸-, (C₁-C₈) alkyl-CO₂-, R²⁷R²⁸N-(C₁-C₈) alkyl-, R²⁷O₂C-(C₁-C₈) alkyl-, heterocycloalkyl, heteroaryl, aryl, or aryl-(C₁-C₈) alkyl-; wherein **R²⁷** and **R²⁸** each independently represents hydrogen or (C₁-C₈) alkyl;
**R⁹** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
   wherein the alkyl or cycloalkyl in **R⁹** is optionally substituted by at least one F, Cl, (C₁-C₈) alkyl, CF₃, OCF₃, CN, OH, =O, (C₁-C₈) alkoxy, NR²⁹R³⁰, CO₂H, R²⁹R³⁰N-C(O)-, R²⁹O-NR³⁰-, heterocycloalkyl, aryl or heteroaryl;
   wherein **R²⁹** and **R³⁰** each independently represents hydrogen or (C₁-C₈) alkyl;
   wherein the heterocycloalkyl, aryl or heteroaryl (*i.e.,* any heterocycloalkyl, aryl or heteroaryl that is represented by **R⁹** or that is part of any substituent thereof) is optionally substituted by at least one F, Cl, CF₃, OCF₃, CN, OH, =O, →O, (C₁-C₈) alkoxy, NR³¹R³², CO₂H, R³¹R³²N-C(O)-, R³¹O-NR³²-, (C₁-C₈) alkyl-CO₂-, R²³R²⁴N-(C₁-C₈)alkyl-, R²³O₂C-(C₁-C₈) alkyl-, heterocycloalkyl, heteroaryl, aryl, or aryl-(C₁-C₈) alkyl-; wherein **R³¹** and **R³²** each independently represents hydrogen or (C₁-C₈) alkyl.

In any one of the following embodiments directed to specific limitations to the structure of the compounds of formula (I), any alkyl, heteroalkyl, cycloalkyl, heterocycloalkyl, aryl, heteroaryl, benzylidene or heteroarylidene may independently be optionally substituted as indicated under formula (I) herein, unless otherwise specified.

According to one preferred embodiment, the present invention relates to a compound of formula (I) or a pharmaceutically acceptable salt and/or solvate thereof; wherein:
**W** represents CH or N;
**R^{A}**, **R^{B}, R^{C}** and **R^{D}** each independently represents hydrogen, F, Cl, CH₃, CF₃, CHF₂ or CH₂F,
   *provided that* at least one among R^{A}, R^{B}, R^{C} and R^{D} does not represent hydrogen;
**R¹** represents hydrogen or (C₁-C₈) alkyl, wherein the alkyl is optionally substituted by at least one OH, (C₁-C₃) alkoxy or F; and **R²**, **R³** and **R⁴** represents hydrogen;
or **R¹** and **R⁴** form together -CH₂-O-CH₂- or -CH₂-CH₂-, wherein the -CH₂-CH₂- is optionally substituted by at least one F, OH or OCH₃; and **R²** and **R³** each represents hydrogen;
**R⁷** represents hydrogen, OH, halogen, (C₁-C₈) alkyl, cycloalkyl, (C₁-C₈) alkyl-O-, cycloalkyl-O-, cycloalkyl-(C₁-C₈) alkyl-O-, heterocycloalkyl-O-, R¹¹O-(C₁-C₈) alkyl-O-, R¹¹R¹²N-(C₁-C₈) alkyl-O- or (R¹¹O)(R¹²)N-(C₁-C₈) alkyl-O-; wherein **R¹¹** and **R¹²** each independently represents hydrogen or (C₁-C₈) alkyl;
   wherein the alkyl or cycloalkyl is optionally substituted by at least one F, Cl, heterocycloalkyl, aryl or heteroaryl;
   wherein the heterocycloalkyl, aryl or heteroaryl is optionally substituted by at least one F, Cl, CF₃, CHF₂, CH₂F, OCF₃, CN, OH, (C₁-C₈) alkoxy, NR¹³R¹⁴, CO₂H, R¹³R¹⁴N-C(O)-, or R¹³O-NR¹⁴-. , wherein **R¹³** and **R¹⁴** each independently represents hydrogen or (C₁-C₈) alkyl;
**Z** represents C-H or N;
or **Z** represents C-**R⁸** and **R⁷** and **R⁸** form together with the carbon atoms to which they are bound a cycloalkyl or heterocycloalkyl,
   wherein the cycloalkyl or heterocycloalkyl is optionally substituted by at least one F, OH, CF₃, HO₂C-CH₂-, (C₁-C₄) alkyl-CO₂-CH₂-, or R¹⁵R¹⁶N-CH₂-, wherein **R¹⁵** and **R¹⁶** each independently represents hydrogen or (C₁-C₈) alkyl;
**R⁵** represents hydrogen, (C₁-C₈) alkyl, CH₃ substituted by one to three (C₁-C₈) alkyl groups, cycloalkyl or cycloalkyl-(C₁-C₈) alkyl;
   wherein the alkyl or cycloalkyl is optionally substituted by at least one F, Cl, CF₃, OCF₃, CN, OH, (C₁-C₈) alkoxy, NR¹⁷R¹⁸, CO₂H, R¹⁷R¹⁸N-C(O)-, R¹⁷O-NR¹⁸-, heterocycloalkyl, aryl or heteroaryl; wherein **R¹⁷** and **R¹⁸** each independently represents hydrogen or (C₁-C₈) alkyl;
   wherein the heterocycloalkyl, aryl or heteroaryl is optionally substituted by at least one F, Cl, CF₃, CHF₂, CH₂F, OCF₃, CN, OH, (C₁-C₈) alkoxy, NR¹⁹R²⁰, CO₂H, R¹⁹R²⁰N-C(O)-, or R¹⁹O-NR²⁰-; wherein **R¹⁹** and **R²⁰** each independently represents hydrogen or (C₁-C₈) alkyl;
**R⁶** represents hydrogen, (C₁-C₈) alkyl, CH₃ substituted by one to three (C₁-C₈) alkyl groups, cycloalkyl or cycloalkyl-(C₁-C₈) alkyl;
   wherein the alkyl or cycloalkyl is optionally substituted by at least one F, Cl, CF₃, OCF₃, CN, OH, (C₁-C₈) alkoxy, NR²¹R²², CO₂H, R²¹R²²N-C(O)-, R²¹O-NR²²-, heterocycloalkyl, aryl or heteroaryl; wherein **R²¹** and **R²²** each independently represents hydrogen or (C₁-C₈) alkyl;
   wherein the heterocycloalkyl, aryl or heteroaryl is optionally substituted by at least one F, Cl, CF₃, CHF₂, CH₂F, OCF₃, CN, OH, (C₁-C₈) alkoxy, NR²³R²⁴, CO₂H, R²³R²⁴N-C(O)-, or R²³O-NR²⁴-; wherein **R²³** and **R²⁴** each independently represents hydrogen or (C₁-C₈) alkyl;
or **R⁵** and **R⁶** form together with the carbon atom to which they are bound a cycloalkyl or heterocycloalkyl,
   wherein the cycloalkyl or heterocycloalkyl is optionally substituted by at least one F, Cl, CF₃, OCF₃, CN, OH, (C₁-C₈) alkoxy, NR²⁵R²⁶, CO₂H, R²⁵R²⁶N-C(O)-, R²⁵O-NR²⁶-, heterocycloalkyl, aryl, heteroaryl, heterocycloalkyl-(C₁-C₈) alkyl-, aryl-(C₁-C₈) alkyl-, heteroaryl-(C₁-C₈) alkyl-, cycloalkyl-(C₁-C₈) alkyl-O-, aryl-(C₁-C₈) alkyl-O-, heteroaryl-(C₁-C₈) alkyl-O-, cycloalkyl-(C₁-C₈) alkyl-NR²⁵-, aryl-(C₁-C₈) alkyl-NR²⁵-, heteroaryl-(C₁-C₈) alkyl-NR²⁵-, benzylidene, heteroarylidene, aryl-(C₁-C₈) alkyl-ylidene- or heteroaryl-(C₁-C₈) alkyl-ylidene-;
   wherein **R²⁵** and **R²⁶** each independently represents hydrogen or (C₁-C₈) alkyl;
   wherein the heterocycloalkyl, aryl, heteroaryl, benzylidene or heteroarylidene is optionally substituted at least one F, Cl, CF₃, CHF₂, CH₂F, OCF₃, CN, OH, (C₁-C₈) alkoxy, NR²⁷R²⁸, CO₂H, R²⁷R²⁸N-C(O)-, or R²⁷O-NR²⁸-; wherein **R²⁷** and **R²⁸** each independently represents hydrogen or (C₁-C₈) alkyl;
**R⁹** represents hydrogen, (C₁-C₈) alkyl, CH₃ substituted by one to three (C₁-C₈) alkyl groups, cycloalkyl or cycloalkyl-(C₁-C₈) alkyl;
   wherein the alkyl or cycloalkyl is optionally substituted by at least one F, Cl, CF₃, OCF₃, CN, OH, (C₁-C₈) alkoxy, NR²⁹R³⁰, CO₂H, R²⁹R³⁰N-C(O)-, R²⁹O-NR³⁰-, heterocycloalkyl, aryl or heteroaryl; wherein **R²⁹** and **R³⁰** each independently represents hydrogen or (C₁-C₈) alkyl;
   wherein the heterocycloalkyl, aryl or heteroaryl is optionally substituted by at least one F, Cl, CF₃, OCF₃, CN, OH, (C₁-C₈) alkoxy, NR³¹R³², CO₂H, R³¹R³²N-C(O)-, or R³¹O-NR³²-; wherein **R³¹** and **R³²** each independently represents hydrogen or (C₁-C₈) alkyl.

According to one embodiment, W represents CH.

According to one embodiment, at least one among **R^{A}**, **R^{B}, R^{C}** and **R^{D}** represents hydrogen. In one embodiment, exactly one among **R^{A}**, **R^{B}, R^{C}** and **R^{D}** represents hydrogen. In one embodiment, exactly two among **R^{A}**, **R^{B}, R^{C}** and **R^{D}** represents hydrogen. In one embodiment, exactly three among **R^{A}**, **R^{B}, R^{C}** and **R^{D}** represents hydrogen.

In one embodiment, at least one among **R^{A}** and **R^{C}** represents hydrogen. In one particular embodiment, **R^{A}** represents hydrogen. In one particular embodiment, **R^{C}** represents hydrogen.

According to one embodiment, at least one among **R^{A}**, **R^{B}** and **R^{D}** represents F or Cl. In one embodiment, at least one among **R^{B}** and **R^{D}** represents F or Cl. In one particular embodiment, **R^{B}** and **R^{D}** each independently represents F or Cl. In one particular embodiment, **R^{B}** represents F. In one particular embodiment, **R^{B}** represents Cl. In one particular embodiment, **R^{D}** represents F. In one particular embodiment, **R^{D}** represents Cl. In one preferred embodiment, **R^{B}** represents F. In one preferred embodiment, **R^{D}** represents Cl. In one further preferred embodiment, **R^{B}** represents F and **R^{D}** represents Cl.

As defined under formula (I) hereinabove, **R¹** may represents (C₁-C₈) alkyl, wherein the alkyl is optionally substituted by at least one OH, (C₁-C₃) alkoxy or F, *i.e.,* the (C₁-C₈) alkyl in **R¹** is optionally substituted by at least one group, and the group may be OH, (C₁-C₃) alkoxy or F. According to one embodiment, **R¹** represents (C₁-C₈) alkyl, wherein the alkyl is optionally substituted by at least one group selected from OH, (C₁-C₃) alkoxy and F.

According to one embodiment, **R¹** represents hydrogen or (C₁-C₈) alkyl, wherein the alkyl is optionally substituted by at least one OH, (C₁-C₃) alkoxy or F; and **R²**, **R³** and **R⁴** represents hydrogen. In one embodiment, **R¹**, **R²**, **R³** and **R⁴** represents hydrogen.

According to one embodiment, **R¹** and **R⁴** form together -CH₂-O-CH₂- or -CH₂-CH₂. In one embodiment, **R¹** and **R⁴** form together -CH₂-CH₂-. In one embodiment, the -CH₂-CH₂- is optionally substituted by at least one F, OH or OCH₃. In one embodiment, the -CH₂-CH₂- is not substituted.

According to one embodiment, **R¹** represents methyl, ethyl, CF₃ or methoxymethyl (CH₃0CH₂-). In one embodiment, **R¹** represents methyl, ethyl or CF₃. In one embodiment, **R¹** represents methoxymethyl (CH₃OCH₂-).

According to one embodiment, **R⁷** represents hydrogen, OH, halogen, (C₁-C₈) alkyl, cycloalkyl, (C₁-C₈) alkyl-O-, cycloalkyl-O-, cycloalkyl-(C₁-C₈) alkyl-O-, heterocycloalkyl-O-, R¹¹O-(C₁-C₈) alkyl-O-, R¹¹R¹²N-(C₁-C₈) alkyl-O- or (R¹¹O)(R¹²)N-(C₁-C₈) alkyl-O-; wherein **R¹¹** and **R¹²** each independently represents hydrogen or (C₁-C₈) alkyl; wherein the alkyl, cycloalkyl, heterocycloalkyl, aryl or heteroaryl is optionally substituted as defined under formula (I) hereinabove.

According to one embodiment, **R⁷** represents hydrogen, OH or halogen. In one embodiment, **R⁷** represents hydrogen. In one preferred embodiment, **R⁷** represents hydroxyl (OH). In one embodiment, **R⁷** represents halogen. In one particular embodiment, **R⁷** represents F or Cl. In one particular embodiment, **R⁷** represents F. In one particular preferred embodiment, **R⁷** represents Cl.

In one preferred embodiment, **R⁷** does not represent hydrogen.

According to one embodiment, **R⁷** represents (C₁-C₈) alkyl-O-(*i.e.,* (C₁-C₈) alkoxy), or cycloalkyl-O-. In one embodiment, the alkyl or cycloalkyl is optionally substituted by at least one F, Cl, OH, (C₁-C₈) alkoxy or aryl. In one particular embodiment, **R⁷** represents OCH₃ (methoxy) or HO-CH₂-CH₂-O-.

According to one embodiment, **R⁷** represents (C₁-C₈) alkyl-O-*(i.e.,* (C₁-C₈) alkoxy). In one embodiment, the alkyl is optionally substituted by at least one F or Cl. In one particular embodiment, the halogen is F. In one embodiment, **R⁷** represents OCH₃ (methoxy), OCH₂CH₃ or OCF₃.

According to one embodiment, **R⁷** represents cycloalkyl-O-. In one embodiment, **R⁷** represents cyclobutyl-O-.

According to one embodiment, **R⁷** represents (C₁-C₈) alkyl-O-(*i.e.,* (C₁-C₈) alkoxy), wherein the alkyl is optionally substituted by at least one OH or (C₁-C₈) alkoxy, *i.e.,* RO-(C₁-C₈) alkyl-O- wherein R represents H or (C₁-C₈) alkyl. In one embodiment, **R⁷** represents HO-CH₂-CH₂-O- or CH₃O-CH₂-CH₂-O-.

According to one embodiment, **R⁷** represents (C₁-C₈) alkyl-O-(*i.e.,* (C₁-C₈) alkoxy), wherein the alkyl is optionally substituted by at least one aryl. In one embodiment, the aryl is not substituted. In one embodiment, **R⁷** represents phenyl-CH₂-O-.

According to one embodiment, **R⁷** represents (C₁-C₈) alkyl-O-(*i.e.,* (C₁-C₈) alkoxy), wherein the alkyl is optionally substituted by at least one heteroaryl. In one embodiment, the heteroaryl is not substituted. In one embodiment, **R⁷** represents heteroaryl-CH₂-O-.

According to one embodiment, **R⁷** represents (C₁-C₈) alkyl-O-(*i.e.,* (C₁-C₈) alkoxy), wherein the alkyl is optionally substituted by at least one heterocycloalkyl. In one embodiment, **R⁷** represents heterocycloalkyl-(C₁-C₈) alkyl-O-, *i.e.,* the alkyl is substituted by exactly one heterocycloalkyl. In one embodiment, the heterocycloalkyl is not substituted.

In one preferred embodiment, **R⁷** represents Cl, OH, OCH₃, HO-CH₂-CH₂-O-, CH₃ (methyl).

According to one embodiment, **Z** represents C-H or N; **or Z** represents C-**R⁸** and **R⁷** and **R⁸** form together with the carbon atoms to which they are bound a cycloalkyl or heterocycloalkyl; wherein the cycloalkyl or heterocycloalkyl is optionally substituted as defined under formula (I) hereinabove. In other words, in this embodiment, **R⁸** represent hydrogen, except where **R⁷** and **R⁸** form together with the carbon atoms to which they are bound a cycloalkyl or heterocycloalkyl.

According to one embodiment, **Z** represents C-H or N. In one embodiment, **Z** represents C-H. In one embodiment, **Z** represents N.

According to one preferred embodiment, **Z** represents C-**R⁸**; wherein **R⁸** represents (C₁-C₄) alkyl, F, Cl, CF₃, CHF₂, CH₂F, OCF₃, CN, OH or (C₁-C₄) alkoxy. In one embodiment, **R⁸** represents methyl, ethyl, F, Cl, CF₃, CN or OH. In one preferred embodiment, **R⁸** represents methyl or Cl.

According to another embodiment, **Z** represents C-**R⁸** and **R⁷** and **R⁸** form together with the carbon atoms to which they **are** bound a cycloalkyl or heterocycloalkyl; wherein the cycloalkyl or heterocycloalkyl is optionally substituted as defined under formula (I) herein.

In one preferred embodiment, **Z** represents C-**R⁸** and **R⁷** and **R⁸** form together with the carbon atoms to which they are bound an heterocycloalkyl; wherein the heterocycloalkyl is optionally substituted as defined under formula (I) herein.

According to one embodiment, **R⁵** and **R⁶** each independently represents (C₁-C₈) alkyl and **R⁹** represents hydrogen. In one preferred embodiment, **R⁵** and **R⁶** each independently represents methyl or ethyl.

According to one preferred embodiment, **R⁵** and **R⁶** each independently represents (C₁-C₈) alkyl or aryl-(C₁-C₈) alkyl-. In one preferred embodiment, **R⁵** and **R⁶** each independently represents methyl, ethyl, *n*-propyl, 2-phenylethyl- or 3-phenylpropyl. In one further preferred embodiment, **R⁹** represents hydrogen.

According to one embodiment, **R⁶** represents (C₁-C₈) alkyl or aryl and **R⁵** and **R⁹** each represents hydrogen. In one embodiment, **R⁶** represents propyl (*e.g*., isopropyl), *tert-*butyl or phenyl.

In one embodiment, **R⁶** represents (C₁-C₈) alkyl and **R⁵** and **R⁹** each represents hydrogen. In one preferred embodiment, **R⁶** represents propyl (*e.g.,* isopropyl) or *tert-*butyl.

According to one preferred embodiment, **R⁶** represents (C₁-C₈) alkyl, cycloalkyl-(C₁-C₈)-alkyl or aryl. In one preferred embodiment, **R⁶** represents *i*-propyl, *n*-propyl, *tert-*butyl*, i*-pentyl, cyclopropyl, cyclobutyl, cyclopentyl, cyclopentyl-CH₂- or phenyl. In one further preferred embodiment, **R⁵** and/or **R⁹** represents hydrogen.

According to one embodiment, **R⁶** represents aryl and **R⁵** and **R⁹** each represents hydrogen. In one embodiment, **R⁶** represents phenyl.

According to one embodiment, **R⁵** represents hydrogen.

According to one embodiment, **R⁵** represents (C₁-C₈) alkyl. In one embodiment, **R⁵** represents methyl or ethyl.

According to one embodiment, **R⁶** represents (C₁-C₈) alkyl. In one embodiment, **R⁶** represents methyl or ethyl.

According to one embodiment, **R⁶** represents aryl. In one embodiment, **R⁶** represents phenyl.

According to one embodiment, **R⁹** represents hydrogen or (C₁-C₈) alkyl. In one embodiment, **R⁹** represents (C₁-C₈) alkyl. In one particular embodiment, **R⁹** represents (C₁-C₄) alkyl. In one preferred embodiment, **R⁹** represents hydrogen.

According to one embodiment, **R⁵** and **R⁶** form together with the carbon atom to which they are bound a cycloalkyl or heterocycloalkyl and **R⁹** represents hydrogen. In one preferred embodiment, **R⁵** and **R⁶** form together with the carbon atom to which they are bound a cyclobutyl, cyclopentyl, cyclohexyl or oxetane.

In one preferred embodiment, **R⁵** and **R⁶** form together with the carbon atom to which they are bound a cyclobutyl, cyclopentyl, cyclohexyl, oxetane, 1-phenyl-4-piperidyl, 1-(2-phenylethyl)-4-piperidyl or 1-[2-(4-fluorophenyl)ethyl]-4-piperidyl.

According to one embodiment, **R⁵** and **R⁶** form together with the carbon atom to which they are bound a cycloalkyl. In one embodiment, **R⁵** and **R⁶** form together with the carbon atom to which they are bound a cyclobutyl, cyclopentyl or cyclohexyl.

According to one embodiment, **R⁵** and **R⁶** form together with the carbon atom to which they are bound a heterocycloalkyl. In one embodiment, **R⁵** and **R⁶** form together with the carbon atom to which they are bound an oxetane.

According to one embodiment, at least one heterocycloalkyl present in the compound of formula (I) is a water-solubilizing group, *i.e.,* the presence of this group in the molecule increases the solubility thereof in water, compared with the same molecule not comprising the heterocycloalkyl.

According to one embodiment, the compound of formula (I) is a compound of formula (I-a) 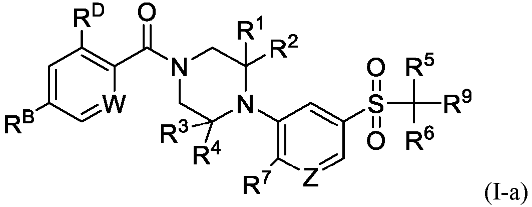 or a pharmaceutically acceptable salt and/or solvate thereof;
wherein **W, R^{B}, R^{D}, Z, R¹-R⁴** and **R⁵-R⁷** are as defined under formula (I) herein.

In one embodiment, W in formula (I-a) represents CH.

In one embodiment, **R^{B}** and **R^{D}** in formula (I-a) each independently represents F or Cl. In one particular embodiment, **R^{B}** represents F, **R^{D}** represents Cl.

According to one embodiment, the compound of formula (I) is selected from the compounds of **Table 1** below, and pharmaceutically acceptable salts and/or solvates thereof.

**Table 1**

| **Cpd** | **Structure** | **Name** |
|---|---|---|
| **001** | 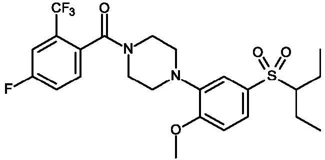 | [4-[5-(1-ethylpropylsulfonyl)-2-methoxyphenyl]piperazin-1-yl]-[4-fluoro-2-(trifluoromethyl)phenyl]m ethanone |
| **002** | 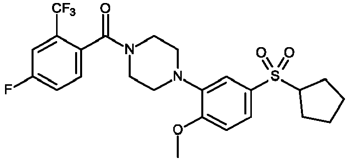 | [4-(5-cyclopentylsulfonyl-2-methoxyphenyl)piperazin-1-yl]-[4-fluoro-2-(trifluoromethyl)phenyl]m ethanone |
| **003** | 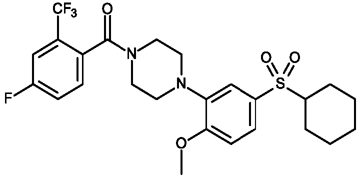 | [4-(5-cyclohexylsulfonyl-2-methoxyphenyl)piperazin-1-yl]-[4-fluoro-2-(trifluoromethyl)phenyl]m ethanone |
| **004** | 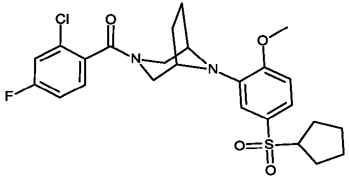 | (2-chloro-4-fluorophenyl)-[(1S,5R)-8-(5-cyclopentylsulfonyl-2-methoxy-phenyl)-3,8-diazabicyclo[3.2.1]octan-3-yl]methanone |
| **005** | 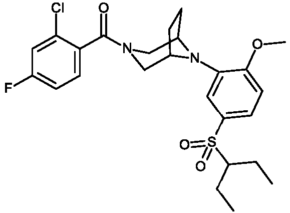 | (2-chloro-4-fluorophenyl)-[(1S,5R)-8-[5-(1-ethylpropylsulfonyl)-2-methoxy-phenyl]-3,8-diazabicyclo[3.2.1]octan-3-yl]methanone |
| **006** | 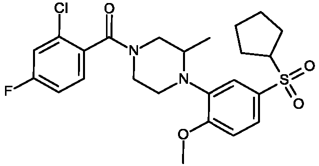 | (2-chloro-4-fluorophenyl)-[rac-(3S)-4-(5-cyclopentylsulfonyl-2-methoxy-phenyl)-3-methyl-piperazin-1-yl]methanone |
| **007** | 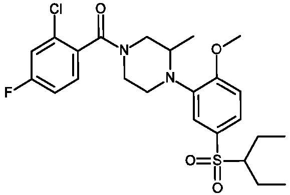 | (2-chloro-4-fluorophenyl)-[rac-(3S)-4-[5-(1-ethylpropylsulfonyl)-2-methoxy-phenyl]-3-methyl-piperazin-1-yl]methanone |
| **008** | 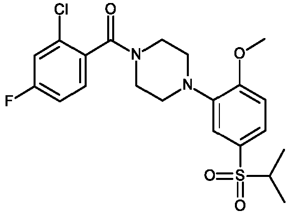 | (2-chloro-4-fluorophenyl)-[4-(5-isopropylsulfonyl-2-methoxyphenyl)piperazin-1-yl]methanone |
| **009** | 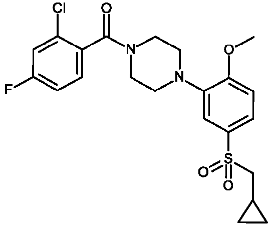 | (2-chloro-4-fluorophenyl)-[4-[5-(cyclopropylmethylsulfonyl )-2-methoxyphenyl]piperazin-1-yl]methanone |
| **010** | 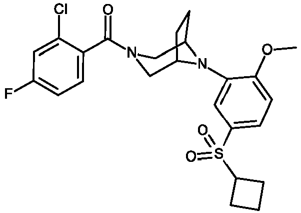 | (2-chloro-4-fluorophenyl)-[(1S,5R)-8-(5-cyclobutylsulfonyl-2-methoxy-phenyl)-3,8-diazabicyclo[3.2.1]octan-3-yl]methanone |
| **011** | 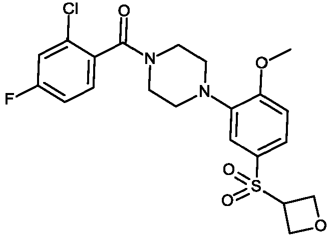 | (2-chloro-4-fluorophenyl)-[4-[2-methoxy-5-(oxetan-3-ylsulfonyl)phenyl]piperazin -1-yl]methanone |
| **012** | 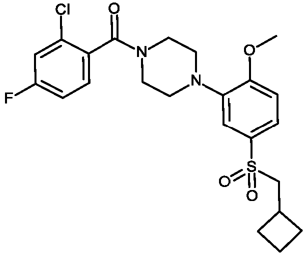 | (2-chloro-4-fluorophenyl)-[4-[5-(cyclobutylmethylsulfonyl )-2-methoxyphenyl]piperazin-1-yl]methanone |
| **013** | 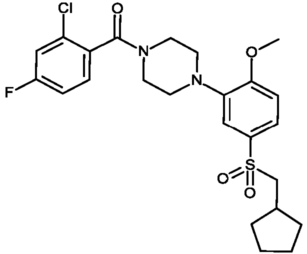 | (2-chloro-4-fluorophenyl)-[4-[5-(cyclopentylmethylsulfonyl )-2-methoxyphenyl]piperazin-1-yl]methanone |
| **014** | 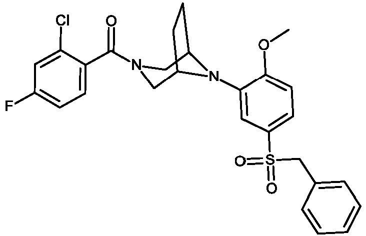 | [(1S,5R)-8-(5-benzylsulfonyl-2-methoxy-phenyl)-3,8-diazabicyclo[3.2.1]octan-3-yl]-(2-chloro-4-fluorophenyl)methanone |
| **015** | 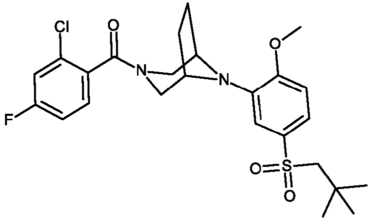 | (2-chloro-4-fluorophenyl)-[(1S,5R)-8-[5-(2,2-dimethylpropylsulfonyl)-2-methoxy-phenyl]-3,8-diazabicyclo[3.2.1]octan-3-yl]methanone |
| **016** | 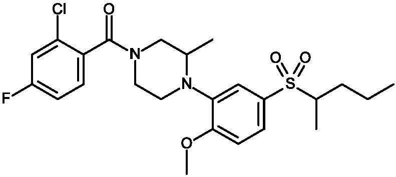 | (2-chloro-4-fluorophenyl)-[rac-(3 S)-4-[2-methoxy-5-[rac-(1S)-1-methylpropyl]sulfonyl-phenyl]-3-methyl-piperazin-1-yl]methanone |
| **017** | 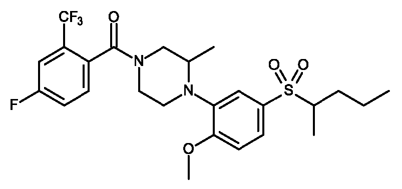 | [4-fluoro-2-(trifluoromethyl)phenyl]-[rac-(3S)-4-[2-methoxy-5-[rac-(1S)-1-methylpropyl]sulfonyl-phenyl]-3-methyl-piperazin-1-yl]methanone |
| **018** | 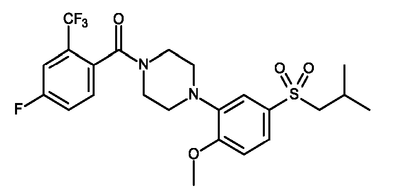 | [4-fluoro-2-(trifluoromethyl)phenyl]-[4-(5-isobutylsulfonyl-2-methoxyphenyl)piperazin-1-yl]methanone |
| **019** | 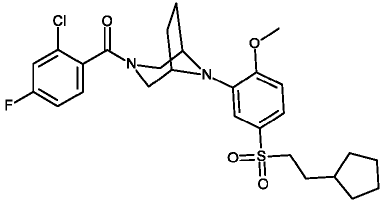 | (2-chloro-4-fluorophenyl)-[(1S,5R)-8-[5-(2-cyclopentylethylsulfonyl)-2-methoxy-phenyl]-3,8-diazabicyclo[3.2.1]octan-3-yl]methanone |
| **020** | 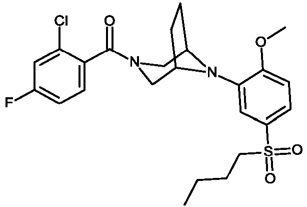 | [(1S,5R)-8-(5-butylsulfonyl-2-methoxyphenyl)-3,8-diazabicyclo[3.2.1]octan-3-yl]-(2-chloro-4-fluorophenyl)methanone |
| **021** | 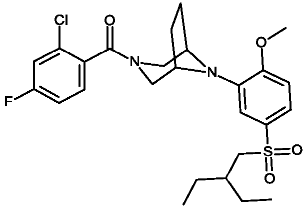 | (2-chloro-4-fluorophenyl)-[(1S,5R)-8-[5-(2-ethylbutylsulfonyl)-2-methoxy-phenyl]-3,8-diazabicyclo[3.2.1]octan-3-yl]methanone |
| **022** | 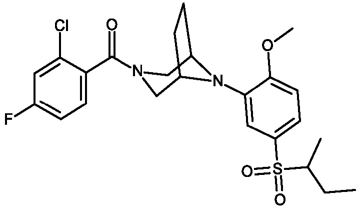 | (2-chloro-4-fluorophenyl)-[(1S,SR)-8-[2-methoxy-5-[(1SR)-1-methylpropyl]sulfonyl-phenyl]-3,8-diazabicyclo[3.2.1]octan-3-yl]methanone |
| **023** | 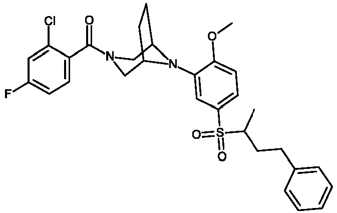 | (2-chloro-4-fluorophenyl)-[(1S,SR)-8-[2-methoxy-5-[(1SR)-1-methyl-3-phenylpropyl]sulfonyl-phenyl]-3,8-diazabicyclo[3.2.1]octan-3-yl]methanone |
| **024** | 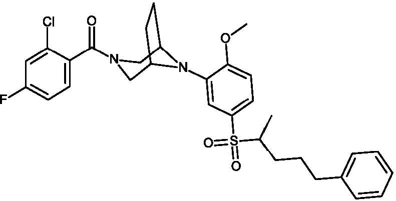 | (2-chloro-4-fluorophenyl)-[(1S,SR)-8-[2-methoxy-5-[(1SR)-1-methyl-4-phenylbutyl]sulfonyl-phenyl]-3,8-diazabicyclo[3.2.1]octan-3-yl]methanone |
| **025** | 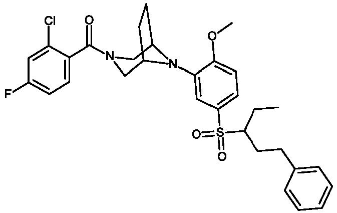 | (2-chloro-4-fluorophenyl)-[(1S,5R)-8-[5-[(1SR)-1-ethyl-3-phenylpropyl]sulfonyl-2-methoxy-phenyl]-3,8-diazabicyclo[3.2.1]octan-3-yl]methanone |
| **026** | 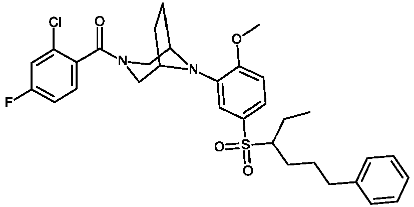 | (2-chloro-4-fluorophenyl)-[(1S,5R)-8-[5-[(1SR)-1-ethyl-4-phenylbutyl]sulfonyl-2-methoxyphenyl]-3,8-diazabicyclo[3.2.1]octan-3-yl]methanone |
| **027** | 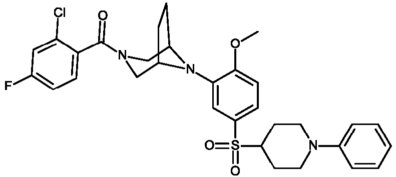 | (2-chloro-4-fluorophenyl)-[(1S,SR)-8-[2-methoxy-5-[(1-phenyl-4-piperidyl)sulfonyl]phenyl] -3,8-diazabicyclo[3.2.1]octan-3-yl]methanone |
| **028** | 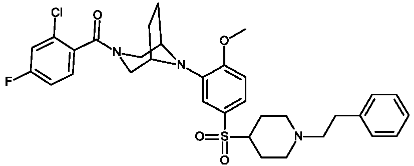 | (2-chloro-4-fluorophenyl)-[(1S,SR)-8-[2-methoxy-5-[[1-(2-phenylethyl)-4-piperidyl]sulfonyl]phenyl] -3,8-diazabicyclo[3.2.1]octan-3-yl]methanone |
| **029** | 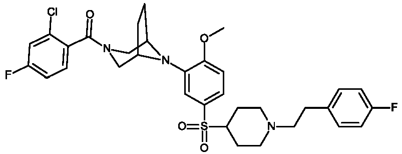 | (2-chloro-4-fluorophenyl)-[(1S,5R)-8-[5-[[1-[2-(4-fluorophenyl)ethyl]-4-piperidyl]sulfonyl]-2-methoxy-phenyl]-3,8-diazabicyclo[3.2.1]octan-3-yl]methanone |
| **030** | 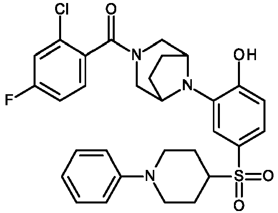 | (2-chloro-4-fluorophenyl)-[(1S,5R)-8-[2-hydroxy-5-[(1-phenyl-4-piperidyl)sulfonyl]phenyl] -3,8-diazabicyclo[3.2.1]octan-3-yl]methanone |
| **031** | 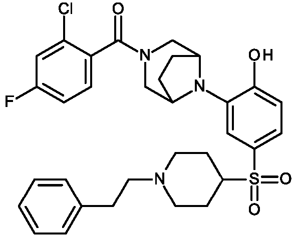 | (2-chloro-4-fluorophenyl)-[(1S,5R)-8-[2-hydroxy-5-[[1-(2-phenylethyl)-4-piperidyl]sulfonyl]phenyl] -3,8-diazabicyclo[3.2.1]octan-3-yl]methanone |
| **032** | 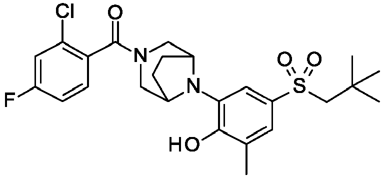 | (2-chloro-4-fluorophenyl)-[(1S,5R)-8-[5-(2,2-dimethylpropylsulfonyl)-2-hydroxy-3-methylphenyl]-3,8-diazabicyclo[3.2.1]octan-3-yl]methanone |
| **033** | 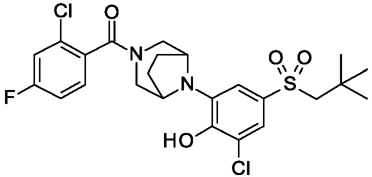 | [(1S,5R)-8-[3-chloro-5-(2,2-dimethylpropylsulfonyl)-2-hydroxy-phenyl]-3,8-diazabicyclo[3.2.1]octan-3-yl]-(2-chloro-4-fluorophenyl)methanone |
| **034** | 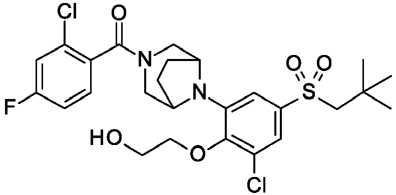 | [(1S,5R)-8-[3-chloro-5-(2,2-dimethylpropylsulfonyl)-2-(2-hydroxyethoxy)phenyl]-3,8-diazabicyclo[3.2.1]octan-3-yl]-(2-chloro-4-fluorophenyl)methanone |
| **035** | 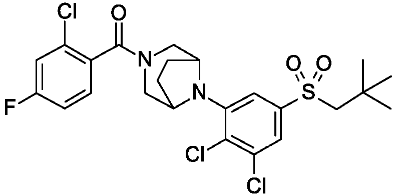 | (2-chloro-4-fluorophenyl)-[(1S,5R)-8-[2,3-dichloro-5-(2,2-dimethylpropylsulfonyl)ph enyl]-3,8-diazabicyclo[3.2.1]octan-3-yl]methanone |
| **036** | 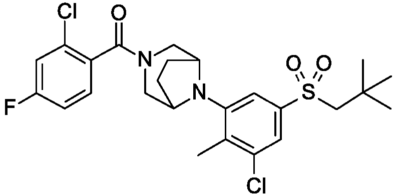 | [(1S,5R)-8-[3-chloro-5-(2,2-dimethylpropylsulfonyl)-2-methyl-phenyl]-3,8-diazabicyclo[3.2.1]octan-3-yl]-(2-chloro-4-fluorophenyl)methanone |

All references herein to a compound of the invention (e.g., "compound of formula (I)") include references to salts, solvates, multi component complexes and liquid crystals thereof. All references herein to a compound of the invention include references to polymorphs and crystal habits thereof. All references herein to a compound of the invention include references to isotopically-labelled compounds thereof, including deuterated compounds thereof. All references herein to a compound of the invention include references to stereoisomers thereof.

In particular, the compounds of the invention may be in the form of pharmaceutically acceptable salts. According to one embodiment, the compound of the invention is a pharmaceutically acceptable salt.

Pharmaceutically acceptable salts include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinafoate salts. Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts. Hemisalts of acids and bases may also be formed, for example, hemisulphate and hemicalcium salts. When a compound contains an acidic group as well as a basic group the compound may also form internal salts, and such compounds are within the scope of the invention. When a compound contains a hydrogen-donating heteroatom (e.g., NH), the invention also encompasses salts and/or isomers formed by transfer of said hydrogen atom to a basic group or atom within the molecule.

Pharmaceutically acceptable salts of compounds of the invention may be prepared by one or more of these methods: (i) by reacting the compound with the desired acid; (ii) by reacting the compound with the desired base; (iii) by removing an acid- or base-labile protecting group from a suitable precursor of the compound or by ring-opening a suitable cyclic precursor, *e.g.,* a lactone or lactam, using the desired acid; and/or (iv) by converting one salt of the compound to another by reaction with an appropriate acid or by means of a suitable ion exchange column. All these reactions are typically carried out in solution. The salt may precipitate from solution and be collected by filtration or may be recovered by evaporation of the solvent. The degree of ionization in the salt may vary from completely ionized to almost non-ionized.

In particular, the compounds of the invention may be in the form of pharmaceutically acceptable solvates. According to one embodiment, the compound of the invention is a pharmaceutically acceptable solvate. According to one embodiment, the compound of the invention is a pharmaceutically acceptable salt and solvate.

In particular, the compounds of the invention may include at least one asymmetric center(s) and thus may exist as different stereoisomeric forms. Accordingly, all references herein to a compound of the invention include all possible stereoisomers and include not only the racemic compounds, but the individual enantiomers and their non-racemic mixtures as well. Non-racemic mixtures may comprise any amounts of each distinct stereoisomer, for example one stereoisomer may be preponderant (*e.g.*, a 90/10 or 80/20 mixture), or the enantiomeric ratio may be close to a racemic mixture (*e.g.*, a 40/60 mixture). When a compound is desired as a single enantiomer, such single enantiomer may be obtained by stereospecific synthesis, by resolution of the final product or any convenient intermediate, or by chiral chromatographic methods as each are known in the art. Resolution of the final product, an intermediate, or a starting material may be carried out by any suitable method known in the art.

### Pharmaceutical composition

Another object of the present invention is a composition comprising a compound according to the invention, as described herein. According to one embodiment, the composition further comprises at least one pharmaceutically acceptable carrier, so that the composition is a "pharmaceutical composition" as defined herein.

In a first embodiment, the pharmaceutical composition comprises the compound according to the invention as sole therapeutic agent. In a second embodiment, the pharmaceutical composition further comprises at least another therapeutic agent such as, for example, a therapeutic agent suitable for treating a neurological disorder.

Another object of the present invention is a medicament comprising a compound according to the invention, as described herein.

### Kit

Another object of the present invention is a kit of parts (in short "kit") comprising a compound or composition according to the invention, as described herein. According to one embodiment, the kit comprises a manufacture such as, for example, a package or a container. According to one embodiment, the kit comprises instructions for use. The kit may be promoted, distributed, or sold as a unit for performing the uses of the present invention.

According to one embodiment, the kit comprises: a pharmaceutical composition comprising the compound according to the invention, and another separate pharmaceutical composition comprising at least another therapeutic agent such as, for example, a therapeutic agent suitable for treating a neurological disorder.

### Medical use of the compound

Another object of the present invention is a compound or a composition according to the invention, as described herein, for use as a medicament.

Another object of the present invention is a compound or a composition according to the invention, as described herein, for use in the treatment of a neurological disorder.

According to one embodiment, the use comprises a step of administering to a subject a therapeutically effective amount of a compound, a composition or a medicament according to the invention, as described herein.

According to one embodiment, the neurological disorder to be treated by the use of the invention is:
- a disease or a disorder associated with defective neurogenesis such as, for example, Hirschsprung disease, schizophrenia, Ataxia telangiectasia, age-related decline of nervous system performance, or a neurodevelopmental disorder;
- a neurodegenerative disease or disorder, such as, for example, Alzheimer's disease, Parkinson's disease, Huntington's disease, Amyotrophic Lateral Sclerosis, Fronto-temporal dementia, retinal neurodegenerative diseases, neuro-ophthalmic diseases, neurotrophic keratitis, Charcot-Marie-Tooth disease, Spinal Muscular Atrophy, epilepsy (*e*.*g.*, an epilepsy disorder, or a seizure disorder, or a chronic neurological disorder presenting with epilepsy), dementia, age-related decline of nervous system performance, a prion disease, Creutzfeldt-Jacob disease, Multiple System Atrophy (Shy Drager Syndrome), Multiple sclerosis, or Guillain-Barre syndrome;
- a disease or a disorder associated with nerve injury or neurotoxicity, such as, for example, head injury, brain damage, traumatic brain injury, peripheral nerve injury, traumatic peripheral nerve injury, peripheral neuropathy, nerve transplantation complications, spinal cord injury, traumatic spinal cord injury, severance of nerves or nerve damage, severance of cerebrospinal nerve cord, a damage to brain or nerve cells, syringomyelia, optic neuropathy, trauma, stroke, ischemia, stroke, ischemic stroke, neurotoxicity caused by alcohol or substance abuse (*e.g*., ecstasy, methamphetamine etc.), or aphasia;

- a neurodevelopmental disorder, such as, for example, Rett syndrome, X-linked mental retardation, fragile X syndrome, Down's syndrome, Autism Spectrum Disorder, Hirschsprung's disease, Tourette syndrome, childhood learning disorder, an attention deficit disorder, Attention Deficit Hyperactivity Disorder (ADHD), Angelman syndrome, micropreemie, schizophrenia, Language disorder, preterm birth, perinatal arterial ischemic stroke, spina bifida, mental retardation, nonsyndromic X-linked mental retardation, Ondine syndrome, or WAGR syndrome;
- a neuropsychiatric disorder such as, for example, depression, Major Depressive Disorder, schizophrenia, schizophrenic form disorder, schizoaffective disorder, delusional disorder, anxiety, anxiety disorders, panic disorder, phobias, an obsessive-compulsive disorder, a post-traumatic stress disorder, a bipolar disorder, anorexia nervosa, bulimia nervosa, anhedonia, apathy, dementia, substance-induced dementia, a motor and/or tic disorder characterized by motor and/or vocal tics (*e.g*., Tourette's disorder), a substance use behavior, addiction, mood disorders, suicidality, cancer-related psychiatric symptoms, Alzheimer's disease, Huntington's disease, Fronto-temporal dementia, or a reward deficiency syndrome (RDS);
- a movement disorder, such as, for example, Parkinson's disease, Huntington's disease, Amyotrophic Lateral Sclerosis, a motor and tic disorder characterized by motor and/or vocal tics (*e.g.*, Tourette's disorder), an ataxia, ataxias muscular rigidity (spasticity), Charcot-Marie-Tooth disease, Spinal Muscular Atrophy, Werdnig-Hoffmann disease, or chronic proximal spinal muscular atrophy;
- a pain disorder, such as, for example, neuralgia, trigeminal neuralgia, chronic pain, inflammatory pain, pain associated with arthritis, fibromyalgia, back pain, cancer-associated pain, pain associated with digestive disease, pain associated with Crohn's disease, pain associated with autoimmune disease, pain associated with endocrine disease, pain associated with diabetic neuropathy, phantom limb pain, spontaneous pain, chronic post-surgical pain, chronic, temporomandibular pain, causalgia, post-herpetic neuralgia, AIDS-related pain, complex regional pain syndromes type I and II, trigeminal neuralgia, chronic back pain, pain associated with spinal cord injury, pain associated with drug intake and recurrent acute pain, neuropathic pain, or inappropriate neuronal activity resulting in neurodysthesia in a disease such as diabetes, an MS and a motor neuron disease;
- an ophthalmic disease or an ocular disorder such as, for example, a retinal disorder, retinal neurodegenerative diseases, Retinitis Pigmentosa, Non-Arthritic Anterior Ischemic Optic Neuropathy (NAION), macular degeneration, age-related macular degeneration, glaucoma, diabetic retinopathy, optic neuropathy and retinal degeneration, neuro-ophthalmic diseases, age-related cataract, primary open-angle glaucoma (POAG), retinal ganglion cell damage, ocular hypertension, ischemic optic neuropathy, macular telangiectasia, cystoid macular edema, Macular Telangiectasia Type 2, neurotrophic keratitis, or strabismus;
- a disorder of the enteric system or a gastro-intestinal disorder, such as, for example, a disorder of intestinal motility, constipation, Hirschsprung's disease, Inflammatory Bowel Disease, Intestinal Neuronal Dysplasia, ulcerative colitis, Achalasia, Esophageal spasm, duodenal ulcer, Zollinger-Ellison Syndrome, hypersecretion of gastric acid, malabsorptive disorder or intestinal inflammation;
- a progressive muscular dystrophy, such as, for example, Duchenne, Becker, Emery-Dreifuss, Landowy-Dejerine, scapulohumeral, limb-girdle, Von Graefe-Fuchs, oculopharyngeal, myotonic and congenital, a congenital or acquired myopathy, Charcot-Marie-Tooth disease, Werdnig-Hoffmann disease, or chronic proximal spinal muscular atrophy;
- a disease or a disorder associated with defective long-term or short-term memory, such as, for example, memory loss, benign forgetfulness, or Alzheimer's disease;
- an autoimmune disorder such as, for example, multiple sclerosis, autoimmune encephalomyelitis, autoimmune encephalitis, autoimmune hemolytic anemia, chronic lymphocytic leukemia, Churg-Strauss syndrome, anti-N-methyl-D-aspartate receptor (NMDAR) encephalitis, Thyroid-associated orbitopathy, autoimmune thyroiditis, Guillain-Barre syndrome, or autoimmune thrombocytopenic purpura;
- a neuro-ontological disease or disorder such as, for example, cochlear sensory cell damage, defective auditory perception, hearing loss, or tinnitus;
- a sleep disorder, such as, for example, narcolepsy, restless leg syndrome, Obstructive sleep apnea, chronic insomnia disorder, paradoxical sleep deprivation or REM sleep deprivation;
- a cerebrovascular disease or a neurovascular disease, such as, for example, early brain injury (EBI) after subarachnoid hemorrhage (SAH), cerebral ischemia, stroke, hypoxic-ischemic brain injury, perinatal arterial ischemic stroke, or neovascular Age-related macular degeneration (nvAMD);
- a substance abuse disorder, such as, for example, substance dependence, substance abuse and the sequalae of substance abuse dependence, substance-induced psychological disorder, substance withdrawal and substance-induced dementia or amnestic disorder;
- a neuronal reaction to viral infection, Trypanosoma infection, a neurological deficit associated with AIDS, obesity, temporomandibular joint dysfunction, aphasia, Bell's palsy, encephalitis, a kidney disease or renal dysfunction, phaeochromocytoma, a metabolic syndrome, cancer, eczema, thrombocytopenia; hypoplasia; disseminated intravascular coagulation (DIC); myelodysplasia; immune thrombocytopenic purpura (ITP), HIV induced ITP, a neuro-oncological disease or disorder, neuro-immunological disease or disorder, multiple endocrine neoplasia type 2, von Hippel-Lindau disease (VHL), type I neurofibromatosis, Scleroderma, an epidermal and stromal wound healing disorder and/or a scarring disorder; or
- a disease or disorder associated with aging and/or senescence.

According to one embodiment, the neurological disorder is epilepsy, such as, for example, Dravet syndrome, benign Rolandic epilepsy, frontal lobe epilepsy, infantile spasms, juvenile myoclonic epilepsy (JME), juvenile absence epilepsy, childhood absence epilepsy (e.g. pyknolepsy), febrile seizures, progressive myoclonus epilepsy of Lafora, Lennox-Gastaut syndrome, Landau-Kleffner syndrome, Generalized Epilepsy with Febrile Seizures (GEFS+), Severe Myoclonic, Epilepsy of Infancy (SMEI), Benign Neonatal Familial Convulsions (BFNC), West Syndrome, Ohtahara Syndrome, early myoclonic encephalopathies, migrating partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorders, epilepsy in subcortical band heterotopia, epilepsy in Walker-Warburg syndrome, epilepsy in Alzheimer's disease, post-traumatic epilepsy, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Unverricht-Lundborg disease, or photosensitive epilepsy.

According to one embodiment, the composition or the medicament according to the invention, as described herein, is to be administered to a subject, and may be formulated using methods well-known in the art. Non-limiting examples of forms adapted for administration include solutions (such as, for example, sterile aqueous solutions), gels, dispersions, emulsions, suspensions and solid forms suitable for using to prepare solutions or suspensions upon the addition of a liquid prior to use (such as, for example, powder or liposomal forms).

The composition or the medicament according to the invention, as described herein, may be administered using administration route well-known in the art such as, for example, parenterally, orally, by inhalation, spray, rectally, nasally, or via an implanted reservoir.

It will be however understood that the total daily usage of the compound, the composition or the medicament will be decided by the attending physician within the scope of sound medical judgment. The specific therapeutically effective dose level for any particular patient will depend upon a variety of factors including the disease being treated and the severity of the disease; activity of the compound employed; the age, body weight, general health, sex and diet of the subject; the time of administration, route of administration, and rate of excretion of the specific therapeutic agent employed; the duration of the treatment; drugs used in combination or coincidental with the specific therapeutic agent employed; and like factors well known in the medical arts. For example, it is well within the skill of the art to start doses of the compound at levels lower than those required to achieve the desired therapeutic effect and to gradually increase the dosage until the desired effect is achieved. The total dose required for each treatment may be administered by multiple doses or in a single dose.

In one embodiment, the dosage of the compound is about 0.01 to 500 mg per kg patient body weight per day, which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be about 0.05 to 0.5, about 0.5 to 5 or about 5 to 50 mg/kg per day. For oral administration, the compositions are preferably provided in the form of tablets containing from about 1.0 to 1000 milligrams of the active ingredient, particularly about 1.0, about 5.0, about 10.0, about 15.0, about 20.0, about 25.0, about 50.0, about 75.0, about 100.0, about 150.0, about 200.0, about 250.0, about 300.0, about 400.0, about 500.0, about 600.0, about 750.0, about 800.0, about 900.0, and about 1000.0 milligrams of the active ingredient for the symptomatic adjustment of the dosage to the patient to be treated. The compounds may be administered on a regimen of 1 to 4 times per day, preferably once or twice per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

According to a first embodiment, the composition or the medicament according to the invention, as described herein, is to be administered as sole therapeutic agent. According to a second embodiment, the composition or the medicament according to the invention, as described herein, is to be administered before at least another therapeutic agent, concomitantly with at least another therapeutic agent, and/or after at least another therapeutic agent. In one embodiment, the other therapeutic agent is suitable for treating a neurological disorder.

Another object of the present invention is a compound or a composition according to the invention, as described herein, for use in promoting neuronal cell's survival and/or neuronal cell's functions. According to one embodiment, the use comprises a step of contacting a neuronal cell with a therapeutically effective amount of a compound, a composition or a medicament according to the invention, as described herein. The use may be *in vitro, ex vivo* or *in vivo.*

Another object of the present invention is a compound or a composition according to the invention, as described herein, for use in rescuing neuronal cell's functions after the neuronal cell has been subjected to insults, events, or conditions detrimental to neuronal cell's functions. Such insults, events, or conditions include, without limitation, neuronal stress, for instance, caused by hypoxia or ischemia; traumatic injuries; and exposure to toxic molecules, for instance, to abnormal misfolded proteins, protein aggregates, excitotoxins, reactive oxygen species, endoplasmic reticulum stressors, mitochondrial stressors, Golgi apparatus antagonists and the like. According to one embodiment, the use comprises a step of contacting a neuronal cell with a therapeutically effective amount of a compound, a composition or a medicament according to the invention, as described herein. The use may be *in vitro, ex vivo* or *in vivo.*

Another object of the present invention is a compound or a composition according to the invention, as described herein, for binding or modulating GFRα1. According to one preferred embodiment, the compound or a composition is for activating GFRα1. In one embodiment, GFRα1 is human GFRα1, preferably with SEQ ID NO: 1.

Another object of the present invention is a compound or a composition according to the invention, as described herein, for activating the GFRα1/RET signaling pathway.

Another object of the present invention is a compound or a composition according to the invention, as described herein, for detecting GFRα1 in a sample. In one embodiment, GFRα1 is human GFRα1, preferably with SEQ ID NO: 1. In one embodiment, the compound according to the invention may be fused to a detectable label, such as, *e.g.,* a fluorophore or any other moiety that can re-emit light upon light excitation, a radiolabel, a contrast agent and the like.

### Manufacturing process

### Synthesis of the compound

The compound according to the invention, as described herein, may be manufactured by means of synthetic methods well-known in the art.

Another object of the present invention is a process for manufacturing a compound of the invention, as described herein. According to one embodiment, the process is a Buchwald-Hartwig amination.

According to one embodiment, the process comprises a step of reaction of:
a compound of formula (II) 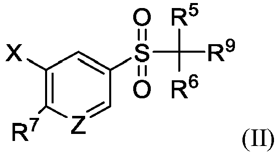 wherein **Z, R⁵**, **R⁶, R⁷** and **R⁹** are as defined under formula (I) herein and X represents halide or -CF₃SO₃,
with a compound of formula (III) 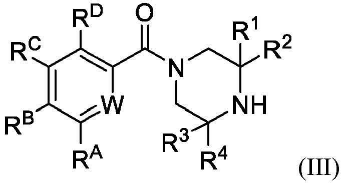 wherein **W, R^{A}-R^{D}** and **R¹-R⁴** are as defined under formula (I) herein,
in presence of a base and a metal catalyst; thereby obtaining the compound of the invention.

In one embodiment, the base is cesium carbonate (Cs₂CO₃), sodium carbonate (Na₂CO₃) or potassium carbonate (K₂CO₃). In one particular embodiment, the base is cesium carbonate (Cs₂CO₃). In one embodiment, the base is sodium *tert*-butanoate (*t*-BuONa), potassium *tert*-butanoate (*t*-BuOK) or potassium phosphate. In one particular embodiment, the base is sodium *tert*-butanoate (*t*-BuONa).

In one embodiment, the catalyst is a palladium catalyst. In one particular embodiment, the catalyst is a Pd(OAc)₂ and rac-BINAP (2,2'-bis(diphenylphosphino)-1,1'-binaphtyle) system. In one particular embodiment, the catalyst is XPhos-Pd-G3.

In one embodiment, **X** represents halide. In one particular embodiment, **X** represents Br.

In one embodiment, the reaction is carried out in a solvent. In one particular embodiment, the solvent is toluene. In one embodiment, the reaction is carried out at reflux.

According to another embodiment, the process comprises:
(a'-1) a step of reaction of
   a compound of formula (II) 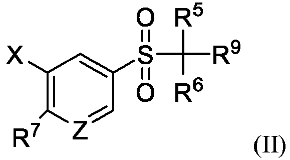 wherein **Z**, **R⁵**, **R⁶, R⁷** and **R⁹** are as defined under formula (I) herein and X represents halide or -CF₃SO₃,
   with a mono-protected piperazine (*i.e*., a piperazine wherein only one of the NH groups is protected by means of a protecting group **R^{P}**) of the following formula (MPP), 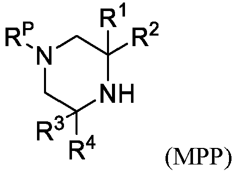
   wherein **R¹-R⁴** are as defined under formula (I) herein;
   in presence of a base and a metal catalyst;
   thereby obtaining a compound of formula (IV) 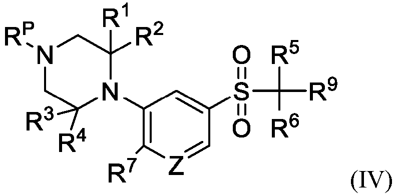 wherein **Z, R¹-R⁴, R⁵**, **R⁶, R⁷** and **R⁹** are as defined under formula (I) herein and **R^{P}** is the protecting group; then
(a'-2) a step of deprotection of the compound of formula (IV);
   thereby obtaining a compound of formula (V) 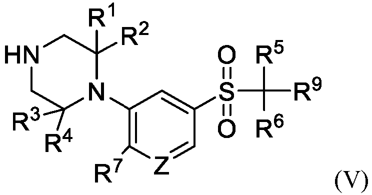 wherein **Z, R¹-R⁴, R⁵**, **R⁶, R⁷** and **R⁹** are as defined under formula (I) herein; and
(a'-3) a step of reaction of the compound of formula (V) with a compound of formula (VI) 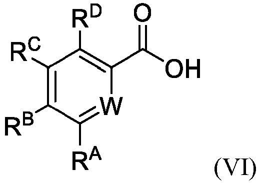 wherein **W and R^{A}-R^{D}** are as defined under formula (I) herein,
   in presence of a peptide coupling agent and a base;
   thereby obtaining the compound of the invention.

In one embodiment, the base and/or the catalyst at step (a'-1) are as described hereinabove under step (a-1). In one embodiment, **X** represents halide. In one particular embodiment, **X** represents Br.

The protecting group may be any protecting group known in the art such as, for example, *tert*-butyloxycarbonyl (Boc). The protective group may be removed at step (a'-2) by any method known in the art appropriate to the nature of the protective group, such as, for example, addition of a strong Bronsted acid (*e.g*., hydrochloric acid [HCl]).

The peptide coupling agent at step (a'-3) may be any peptide coupling agent known in the art such as, for example, 2-(*1H*-benzotriazole-1-yl)-1,1,3,3-tetramethylaminium tetrafluoroborate (TBTU).

The base at step (a'-3) may be any base known in the art such as, for example, an amine base. According to one embodiment, the amine is triethylamine (Et₃N) or diisopropylethylamine (iPr₂NEt).

In one embodiment, the reaction at step (a'-3) is carried out in a solvent. In one particular embodiment, the solvent is dimethylformamide (DMF) and/or tetrahydrofuran (THF). In one particular embodiment, the solvent is dichloroethane (DCE) and/or acetonitrile (MeCN). In one embodiment, the reaction is carried out at room temperature (RT).

In one embodiment, the process further comprises a work-up step (b). In one embodiment, the work-up step (b) comprises a step of extraction by a solvent. In one particular embodiment, the solvent is ethyl acetate (EtOAc). In one particular embodiment, the solvent is water or a 1N HCl solution. In one particular embodiment, the solvent is dichloromethane (DCM). In one embodiment, the work-up step (b) comprises a step of filtration. In one particular embodiment, the filtration is over Celite^{®} In one embodiment, the work-up step (b) comprises a step of concentration under reduced pressure.

In one embodiment, the process further comprises a purification step (c). In one embodiment, the purification step (c) comprises a purification by chromatography. In one particular embodiment, the chromatography is flash chromatography (FC) (*e.g.,* cHex/EtOAc gradient), preparative thin-layer chromatography (PTLC) or semi-preparative high-performance liquid chromatography (HPLC).

### Synthetic intermediates

Another object of the present invention is a compound of formula (II) 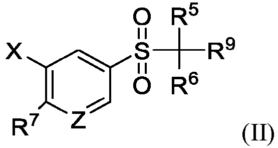 wherein **Z**, **R⁵**, **R⁶, R⁷** and **R⁹** are as defined under formula (I) herein and X represents halide or -CF₃SO₃.

Another object of the present invention is a compound of formula (III) 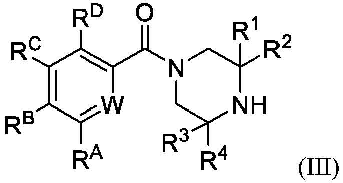 wherein W, **R^{A}-R^{D}** and **R¹-R⁴** are as defined under formula (I) herein.

Another object of the present invention is a compound of formula (IV) 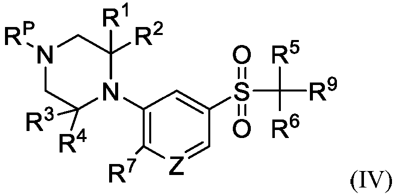 wherein Z, **R¹-R⁴, R⁵**, **R⁶, R⁷** and **R⁹** are as defined under formula (I) herein and **R^{P}** is a protecting group *(e.g.,* Boc).

Another object of the present invention is a compound of formula (V) 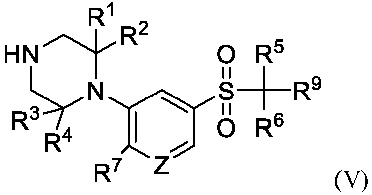 wherein Z, **R¹-R⁴, R⁵**, **R⁶, R⁷** and **R⁹** are as defined under formula (I) herein.

### EXAMPLES

The present invention is further illustrated by the following examples.

### Example 1: Synthesis of the compounds

### General material and methods

### Abbreviations

### List of abbreviations:

Ac: acetyl
Ar: argon
BINAP: (2,2'-bis(diphenylphosphino)-1,1'-binaphthyl)
cHex: cyclohexane
DCM: dichloromethane
DMF: dimethylformamide
Et: ethyl
FC: flash chromatography
m-CPBA: *meta*-chloroperbenzoic acid
MTBE: methyl *tert*-butyl ether
PTLC: preparative thin-layer chromatography
RT: room temperature
TBTU: 2-(*1H*-benzotriazole-1-yl)-1,1,3,3-tetramethylaminium tetrafluoroborate
TFA: trifluoroacetic acid
THF: tetrahydrofuran
XPhos: dicyclohexyl[2',4',6'-tris(propan-2-yl)[1,1'-biphenyl]-2-yl]phosphane

### Analytical methods

¹H NMR spectra (400 MHz) and ¹⁹F NMR spectra (376 MHz) were recorded with a Bruker ULTRASHIELD 400 spectrometer. Processing and analyses of the spectra were performed with MestReNova. Data appear in the following order: chemical shifts in ppm which were referenced to the internal solvent signal, multiplicity, coupling constant J in Hertz and number of protons.

Reversed-phase HPLC/MS analyses were carried out with a Waters Alliance 2795 HPLC equipped with an autosampler, an inline membrane degasser, a column oven 10 (T° = 45 °C), a UV detector, and a ZQ quadrupole mass detector working in ionization electrospray mode. Analyzed compounds (0.1 to 0.3 mg) were solubilized in a minimum amount of DMSO completed with acetonitrile (total volume: 1 mL). Standard analytical parameters: flow rate: 1 mL/min, V_{inj.}: 5µL. Acidic conditions: Waters XSelect CSH C18 column (3.5µm, 2.1x 15 50 mm). Gradient: (H₂O + 0.04% v/v HCO₂H (10mM))/ACN from 95/5 to 0/100 in 2.5 min. Alkaline conditions: Waters Xbridge C18 column (3.5µm, 2.1x 50 mm). Gradient: (H₂O + 0.06% v/v NH₃ (aq.) (10mM))/ACN from 95/5 to 0/100 in 2.5 min.

### General synthetic methods

### General protocol 1 (GP-1): Thiol alkylation

To a solution of 3-bromo-4-methoxy benzenethiol in DMF, were added the alkylhalide or pseudo-halide and K₂CO₃. The mixture was stirred the required time at the required temperature. The reaction mixture was partitioned between water and MTBE. The layers were separated and the aqueous phase was extracted with MTBE. The combined organic extracts were washed (brine), dried (Na₂SO₄), filtered and concentrated under reduced pressure. The residue was purified by FC (cHex/EtOAc gradient) to afford the required thioether.

### General protocol 2 (GP-2): Thioether oxidation

To a solution of thioether from **GP-1** in DCM at 0°C, was added m-CPBA. The mixture was stirred at RT for the required time. Aq. Sat. Na₂S₂O₃, aq. sat. NaHCO₃ and EtOAc were added. The mixture was vigorously stirred for 10 min. The layers were separated and the aqueous phase was extracted with EtOAc. The combined organic extracts were washed (brine), dried (Na₂SO₄), filtered and concentrated under reduced pressure. The residue was purified by FC (cHex/EtOAc gradient) to afford the required sulfone.

### General protocol 3 (GP-3): Buchwald coupling using Pd(OAc)₂/rac-BINAP

A microwave reaction vial was charged with the arylbromide from **GP-2,** the required piperazine, Cs₂CO₃, Pd(OAc)₂ and rac-BINAP. The vial was flushed with argon and degassed toluene was added. The vial was sealed and the reaction was stirred at reflux in a preheated heating-block for the required time. After cooling down to RT, EtOAc was added and the suspension as filtered over Celite^{®} (EtOAc rinses). The filtrate was concentrated under reduced pressure. The residue was purified by FC (cHex/EtOAc gradient) or PTLC to afford the required product.

### General protocol 4 (GP-4): Buchwald coupling using XPhos-Pd-G3

A microwave reaction vial was charged with the arylbromide from **GP-2** the required piperazine, t-BuONa and XPhos-Pd-G3. The vial was flushed with argon and degassed toluene was added. The vial was sealed and the reaction was stirred at reflux in a preheated heating-block for the required time. After cooling down to RT, EtOAc was added and the suspension was filtered over Celite^{®} (EtOAc rinses). The filtrate was concentrated under reduced pressure. The residue was purified by FC (cHex/EtOAc gradient) or PTLC to afford the required product.

### Synthesis of intermediate compounds

### Synthesis of thioethers using GP-1

### 2-bromo-4-(1-ethylpropylsulfanyl)-1-methoxy-benzene (I-001)

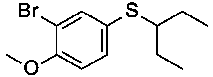

**Formula** Weight: 289,23

Molecular Formula: C₁₂H₁₇BrOS

Using **GP-1, I-001** was obtained as a colorless oil in 99% yield using 3-bromo-4-methoxy benzenethiol (156mg, 712µmol, 1 equiv.), 3-bromopentane (215mg, 1.42mmol, 2 equiv.) and K₂CO₃ (197mg, 1.42mmol, 2 equiv.) in DMF (2.1mL) at RT for 16h. ¹H NMR (400 MHz, Chloroform-*d*) δ 7.65 (d, *J* = 2.2 Hz, 1H), 7.37 (dd, *J=* 8.5, 2.2 Hz, 1H), 6.84 (d, *J* = 8.5 Hz, 1H), 3.91 (s, 3H), 2.84 (p, *J =* 6.3 Hz, 1H), 1.82-1.39 (m, 4H), 1.03 (t, *J=* 7.4 Hz, 6H). MS (ESI⁺): [M+H]⁺ 289.0/291.0.

### 2-bromo-4-cyclopentylsulfanyl-1-methoxy-benzene (I-002)

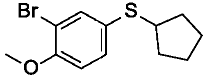

Formula Weight: 287,22

Molecular Formula: C₁₂H₁₅BrOS

Using **GP-1, I-002** was obtained as a light yellow oil in 80% yield using 3-bromo-4-methoxy benzenethiol (560mg, 2.56mmol, 1 equiv.), iodocyclopentane (442µL, 3.83mmol, 1.5 equiv.) and K₂CO₃ (530mg, 3.83mmol, 1.5 equiv.) in DMF (13mL) at RT for 4h. ¹H NMR (400 MHz, Chloroform-*d*) δ 7.54 (d, *J=* 2.2 Hz, 1H), 7.25 (dd, *J* = 8.5, 2.2 Hz, 1H), 6.74 (d, *J =* 8.5 Hz, 1H), 3.80 (s, 3H), 3.43-3.32 (m, 1H), 1.95-1.83 (m, 2H), 1.75-1.62 (m, 2H), 1.58-1.43 (m, 4H). MS (ESI⁺): [M+H]⁺ 287.0/289.0.

### 2-bromo-4-cyclohexylsulfanyl-1-methoxy-benzene (I-003)

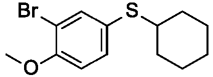

Formula Weight: 301,24

Molecular Formula: C₁₃H₁₇BrOS

Using **GP-1, I-003** was obtained as a light yellow oil in 50% yield using 3-bromo-4-methoxy benzenethiol (124mg, 566µmol, 1 equiv.), iodocyclohexane (110µL, 849µmol, 1.5 equiv.) and K₂CO₃ (117mg, 849µmol, 1.5 equiv.) in DMF (2.3mL) at RT for 60h. ¹H NMR (400 MHz, Chloroform-*d*) δ 7.67 (d, *J =* 2.2 Hz, 1H), 7.37 (t, *J* = 2.0 Hz, 1H), 6.84 (d, *J =* 2.8 Hz, 1H), 3.91 (s, 3H), 3.03-2.82 (m, 1H), 2.05-1.87 (m, 2H), 1.85-1.72 (m, 2H), 1.69-1.58 (m, 1H), 1.41-1.15 (m, 5H). MS (ESI⁺): [M+H]⁺ 301.0/303.0.

### 2-bromo-4-(cyclopropylmethylsulfanyl)-1-methoxy-benzene (I-004)

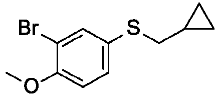

**Formula** Weight: 273,19

Molecular Formula: C₁₁H₁₃BROS

Using **GP-1, I-004** was obtained as a light yellow oil in 87% yield using 3-bromo-4-methoxy benzenethiol (120mg, 548µmol, 1 equiv.), (bromomethyl)cyclopropane (80µL, 0.82mmol, 1.5 equiv.) and K₂CO₃ (114mg, 822µmol, 1.5 equiv.) in DMF (2.7mL) at RT for 4h. ¹H NMR (400 MHz, DMSO-*d6*) δ 7.40 (d, *J= 2.3* Hz, 1H), 7.20 (dd, *J =* 8.6, 2.2 Hz, 1H), 6.88 (d, *J* = 8.6 Hz, 1H), 3.65 (s, 3H), 2.66 (d, *J =* 7.0 Hz, 2H), 0.80-0.69 (m, 1H), 0.36-0.25 (m, 2H), 0.05-0.06 (m, 2H). MS (ESI⁺): [M+H]⁺ 273.0/275.0.

### 2-bromo-4-(cyclopropylmethylsulfanyl)-1-methoxy-benzene (I-005)

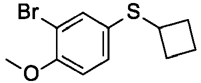

**Formula** Weight: 273,19

Molecular Formula: C₁₁H₁₃BrOS

Using **GP-1, I-005** was obtained as a light yellow oil in 71% yield using 3-bromo-4-methoxy benzenethiol (170mg, 776µmol, 1 equiv.), bromocyclobutane (98µL, 1.2mmol, 1.5 equiv.) and K₂CO₃ (161mg, 1.16mmol, 1.5 equiv.) in DMF (3.9mL) at 80°C for 16h. Purified by FC (cHex/EtOAc = 90/10 to 60/40). ¹H NMR (400 MHz, Chloroform-*d*) δ 7.53 (d, *J =* 2.2 Hz, 1H), 7.24 (d, *J =* 2.2 Hz, 1H), 6.82 (d, *J =* 8.5 Hz, 1H), 3.88 (s, 3H), 3.75 (p, *J =* 7.8 Hz, 1H), 2.42-2.31 (m, 2H), 2.05-1.87 (m, 4H). MS (ESI⁺): [M+H]⁺ 273.0/275.0.

### 3-(3-bromo-4-methoxy-phenyl)sulfanyloxetane (I-006)

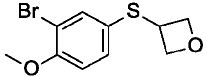

Formula Weight: 275,16

Molecular Formula: C₁₀H₁₁BrO₂S

Using **GP-1, I-006** was obtained as a light yellow oil in 69% yield using 3-bromo-4-methoxy benzenethiol (150mg, 685µmol, 1 equiv.), 3-bromooxetane (78µL, 1.03mmol, 1.5 equiv.) and K₂CO₃ (141mg, 1.03mmol, 1.5 equiv.) in DMF (3.4mL) at RT for 2h. Purified by FC (cHex/EtOAc = 90/10 to 60/40). ¹H NMR (400 MHz, Chloroform-*d*) δ 7.54 (d, *J=* 2.2 Hz, 1H), 7.28 (dd, *J =* 8.6, 2.4 Hz, 1H), 6.84 (d, *J* = 8.5 Hz, 1H), 4.95 (t, *J =* 7.0 Hz, 2H), 4.61 (t, *J =* 6.5 Hz, 2H), 4.40-4.27 (m, 1H), 3.89 (s, 3H). MS (ESI⁺): [M+H]⁺ 275.0/277.0.

### 2-bromo-4-(cyclobutylmethylsulfanyl)-1-methoxy-benzene (I-007)

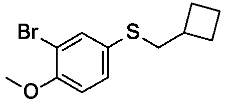

**Formula** Weight: 287,22

Molecular Formula: C₁₂H₁₅BrOS

Using **GP-1, I-007** was obtained as a colorless oil in 64% yield using 3-bromo-4-methoxy benzenethiol (120mg, 548µmol, 1 equiv.), (bromomethyl)cyclobutane (92µL, 0.82mmol, 1.5 equiv.) and K₂CO₃ (114mg, 0.82mmol, 1.5 equiv.) in DMF (2.7mL) at RT for 2h. Purified by FC (cHex/EtOAc = 90/10 to 60/40). ¹H NMR (400 MHz, Chloroform-*d*) δ 7.57 (d, *J* = 2.2 Hz, 1H), 7.29 (dd, *J* = 8.5, 2.3 Hz, 1H), 6.81 (d, *J* = 8.5 Hz, 1H), 3.88 (s, 3H), 2.89 (d, *J =* 7.6 Hz, 2H), 2.46 (hept, *J* = 7.6 Hz, 1H), 2.15-2.02 (m, 2H), 1.90-1.75 (m, 2H), 1.75-1.63 (m, 2H). MS (ESI⁺): [M+H]⁺ 287.0/289.0.

### 2-bromo-4-(cyclopentylmethylsulfanyl)-1-methoxy-benzene (I-008)

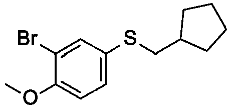

Formula Weight: 301,24

Molecular Formula: C₁₃H₁₇BrOS

Using **GP-1, I-008** was obtained as a colorless oil in 30% yield using 3-bromo-4-methoxy benzenethiol (120mg, 548µmol, 1 equiv.), (bromomethyl)cyclobutane (103µL, 822µmol, 1.5 equiv.) and K₂CO₃ (114mg, 822µmol, 1.5 equiv.) in DMF (2.7mL) at RT for 2h. Purified by FC (cHex/EtOAc = 90/10 to 60/40). ¹H NMR (400 MHz, Chloroform-*d*) δ 7.58 (d, *J* = 2.3 Hz, 1H), 7.30 (dd, *J =* 8.5, 2.3 Hz, 1H), 6.82 (d, *J =* 8.6 Hz, 1H), 3.88 (s, 3H), 2.84 (d, *J =* 7.3 Hz, 2H), 2.05 (hept, *J =* 7.6 Hz, 1H), 1.89-1.76 (m, 2H), 1.63-1.50 (m, 4H), 1.33-1.18 (m, 2H). MS (ESI⁺): [M+H]⁺ 301.0/303.0.

### 4-benzylsulfanyl-2-bromo-1-methoxy-benzene (I-009)

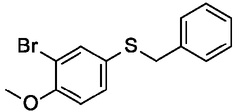

Formula Weight: 309,22

Molecular Formula: C₁₄H₁₃BrOS

Using **GP-1, I-009** was obtained as a colorless oil in 100% yield using 3-bromo-4-methoxy benzenethiol (170mg, 776µmol, 1 equiv.), benzyl bromide (142µL, 1.16mmol, 1.5 equiv.) and K₂CO₃ (161mg, 1.16mmol, 1.5 equiv.) in DMF (3.9mL) at RT for 1h. Purified by FC (cHex/EtOAc = 90/10 to 60/40). ¹H NMR (400 MHz, Chloroform-*d*) δ 7.55 (d, *J=* 2.2 Hz, 1H), 7.45-7.24 (m, 5H), 7.22 (d, *J* = 2.1 Hz, 1H), 6.79 (d, *J =* 8.5 Hz, 1H), 4.03 (s, 2H), 3.89 (s, 3H). MS (ESI⁺): [M+H]⁺ 309.0/311.0.

### 2-bromo-4-(2,2-dimethylpropylsulfanyl)-1-methoxy-benzene (I-010)

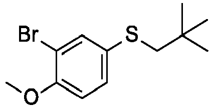

Formula Weight: 289,23

Molecular Formula: C₁₂H₁₇BrOS

Using **GP-1, I-010** was obtained as a colorless oil in 22% yield using 3-bromo-4-methoxy benzenethiol (170mg, 776µmol, 1 equiv.), 1-bromo-2,2-dimethylpropane (147µL, 1.16mmol, 1.5 equiv.) and K₂CO₃ (161mg, 1.16mmol, 1.5 equiv.) in DMF (3.9mL) at RT for 1h. Purified by FC (cHex/EtOAc = 90/10 to 60/40). ¹H NMR (400 MHz, Chloroform-*d*) δ 7.58 (d, *J=* 2.3 Hz, 1H), 7.31 (dd, *J =* 8.6, 2.3 Hz, 1H), 6.80 (d, *J =* 8.5 Hz, 1H), 3.87 (s, 3H), 2.82 (s, 2H), 1.01 (s, 9H). MS (ESI⁺): [M+H]⁺ 289.0/291.0.

### 2-bromo-4-isopropylsulfanyl-1-methoxy-benzene (I-011)

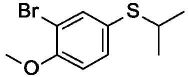

Formula Weight: 261.18

Molecular Formula: C₁₀H₁₃BrOS

Using **GP-1, I-011** was obtained as a colorless oil in 42% yield using 3-bromo-4-methoxy benzenethiol (120mg, 548µmol, 1 equiv.), 1-Bromo-2,2-dimethylpropane (77µL, 0.82mmol, 1.5 equiv.) and K₂CO₃ (114mg, 822µmol, 1.5 equiv.) in DMF (2.7mL) at RT for 4h. Purified by FC (cHex/EtOAc = 90/10 to 60/40). ¹H NMR (400 MHz, Chloroform-*d*) δ 7.57 (d, *J=* 2.2 Hz, 1H), 7.28 (dd, *J=* 8.5, 2.2 Hz, 1H), 6.75 (d, *J=* 8.5 Hz, 1H), 3.81 (s, 3H), 3.13 (hept, *J* = 6.7 Hz, 1H), 1.17 (d, *J =* 6.6 Hz, 6H). MS (ESI⁺): [M+H]⁺ 261.0/263.0.

### 2-bromo-4-isobutylsulfanyl-1-methoxy-benzene (I-023)

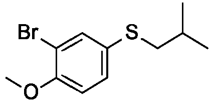

Formula Weight: 275.21

Molecular Formula: C₁₁H₁₅BrOS

Using **GP-1, I-023** was obtained as a colorless oil in 65% yield using 3-bromo-4-methoxy-benzenethiol (219mg, 999µmol, 1 equiv.), 1-bromo-2-methylpropane (120µL, 1.10mmol, 1.1 equiv.) and K₂CO₃ (145mg, 1.05mmol, 1.05 equiv.) in DMF (9.1mL) at RT for 5h. Purified by FC (cHex/EtOAc = 100/0 to 80/20). ¹H NMR (400 MHz, Chloroform-*d*) δ 7.58 (d, *J= 2.3* Hz, 1H), 7.30 (dd, *J* = 8.5, 2.3 Hz, 1H), 6.82 (d, *J* = 8.5 Hz, 1H), 3.88 (s, 3H), 2.72 (d, *J =* 6.9 Hz, 2H), 1.80 (hept, *J =* 13.4, 6.7 Hz, 1H), 1.01 (d, *J* = 6.7 Hz, 6H). MS (ESI⁺): [M+H]⁺ 275.0/277.0.

### 2-bromo-1-methoxy-4-(1-methylbutylsulfanyl)benzene (I-025)

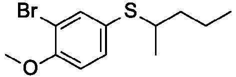

Formula Weight: 289.23

Molecular Formula: C₁₂H₁₇BrOS

Using **GP-1, I-025** was obtained as a colorless oil in 88% yield using 3-bromo-4-methoxy-benzenethiol (1.37g, 6.27mmol, 1 equiv.), 2-bromopentane (1.16mL, 9.41mmol, 1.5 equiv.) and K₂CO₃ (1.30g, 9.41mmol, 1.5 equiv.) in DMF (25mL) at RT for 5h. Desired product **I-025** was contaminated by 10% of disulfide and was used for the further reaction without further purification. ¹H NMR (400 MHz, Chloroform-*d*) δ 7.64 (d, *J=* 2.2 Hz, 1H), 7.35 (ddd, *J=* 8.5, 4.6, 2.2 Hz, 1H), 6.83 (d, *J=* 8.4 Hz, 1H), 3.89 (d, *J=* 1.3 Hz, 4H), 3.13-2.96 (m, 1H), 1.51-1.38 (m, 3H), 1.22 (d, *J =* 6.7 Hz, 3H), 0.95-0.86 (m, 3H). MS (ESI⁺): [M+H]⁺ 288.9/290.9.

### 2-bromo-4-(2-cyclopentylethylsulfanyl)-1-methoxy-benzene (I-032)

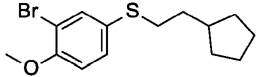

Formula Weight: 315,27

Molecular Formula: C₁₄H₁₉BrOS

Using **GP-1, I-032** was obtained as a colorless oil in 26% yield using 3-bromo-4-methoxy-benzenethiol (270mg, 1.23mmol, 1 equiv.), 2-bromoethylcyclopentane (0.21mL, 1.5mmol, 1.2 equiv.) and K₂CO₃ (255mg, 1.85mmol, 1.5 equiv.) in DMF (6.2mL) at RT for 2h. Purified by FC (cHex/EtOAc = 95/5 to 60/40). ¹H NMR (400 MHz, Chloroform-*d*) δ 7.57 (d, *J= 2.2* Hz, 1H), 7.29 (dd, *J=* 8.5, 2.3 Hz, 1H), 6.82 (d, *J* = 8.6 Hz, 1H), 3.88 (s, 3H), 2.87 - 2.80 (m, 2H), 1.94 - 1.82 (m, 1H), 1.80 - 1.72 (m, 2H), 1.61 - 1.47 (m, 6H), 1.12 - 1.01 (m, 2H). MS (ESI⁺): [M+H]⁺ 315.0/317.0.

### 2-bromo-4-butylsulfanyl-1-methoxy-benzene (I-033)

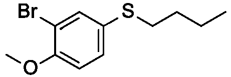

Formula Weight: 275,21

Molecular Formula: C₁₁H₁₅BrOS

Using **GP-1, I-033** was obtained as a colorless oil in 53% yield using 3-bromo-4-methoxy-benzenethiol (150mg, 0.685mmol, 1 equiv.), 1-bromobutane (0.11mL, 1.03mmol, 1.5 equiv.) and K₂CO₃ (142mg, 1.03mmol, 1.5 equiv.) in DMF (3.4mL) at RT for 2h. Purified by FC (cHex/EtOAc = 90/10 to 60/40). ¹H NMR (400 MHz, Chloroform-*d*) δ 7.58 (d, *J=* 2.2 Hz, 1H), 7.30 (dd, *J* = 8.5, 2.3 Hz, 1H), 6.82 (d, *J =* 8.5 Hz, 1H), 3.88 (s, 3H), 2.87 - 2.78 (m, 2H), 1.61 - 1.55 (m, 2H), 1.49 - 1.36 (m, 2H), 0.91 (t, *J =* 7.3 Hz, 3H). MS (ESI⁺): [M+H]⁺ 275.0/277.0.

### 2-bromo-4-(2-ethylbutylsulfanyl)-1-methoxy-benzene (I-034)

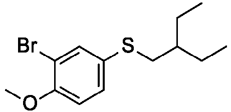

Formula Weight: 303,26

Molecular Formula: C₁₃H₁₉BrOS

Using **GP-1, I-034** was obtained as a colorless oil in 90% yield using 3-bromo-4-methoxy-benzenethiol (170mg, 0.776mmol, 1 equiv.), 3-(bromomethyl)pentane (0.16mL, 1.2mmol, 1.5 equiv.) and K₂CO₃ (161mg, 1.16mmol, 1.5 equiv.) in DMF (3.9mL) at RT for 16h. Purified by FC (cHex/EtOAc = 90/10 to 60/40). ¹H NMR (400 MHz, Chloroform-*d*) δ 7.57 (d, *J= 2.3* Hz, 1H), 7.29 (dd, *J* = 8.5, 2.3 Hz, 1H), 6.81 (d, *J* = 8.5 Hz, 1H), 3.87 (s, 3H), 2.84 - 2.79 (m, 2H), 1.48 - 1.38 (m, 5H), 0.88 - 0.83 (m, 6H). MS (ESI⁺): [M+H]⁺ 303.0/305.0.

### 2-bromo-1-methoxy-4-sec-butylsulfanyl-benzene (I-035)

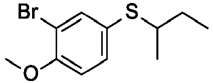

Formula Weight: 275,21

Molecular Formula: C₁₁ H₁₅BrOS

Using **GP-1, I-035** was obtained as a colorless oil in 53% yield using 3-bromo-4-methoxy-benzenethiol (150mg, 0.684mmol, 1 equiv.), 2-bromobutane (0.11mL, 1.0mmol, 1.5 equiv.) and K₂CO₃ (142mg, 1.03mmol, 1.5 equiv.) in DMF (3.4mL) at RT for 2h. Purified by FC (cHex/EtOAc = 90/10 to 60/40). ¹H NMR (400 MHz, Chloroform-d) δ 7.64 (d, *J=* 2.2 Hz, 1H), 7.35 (dd, *J=* 8.5, 2.2 Hz, 1H), 6.83 (d, *J* = 8.5 Hz, 1H), 3.89 (s, 3H), 3.05 - 2.94 (m, 1H), 1.67 - 1.56 (m, 1H), 1.53 - 1.40 (m, 1H), 1.22 (d, J = 6.8 Hz, 3H), 1.00 (t, J = 7.4 Hz, 3H). MS (ESI⁺): [M+H]⁺ 275.0/277.0.

### 2-bromo-1-methoxy-4-(1-methyl-3-phenyl-propyl)sulfanyl-benzene (I-036)

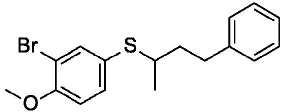

Formula Weight: 351,3

Molecular Formula: C₁₇H₁₉BrOS

Using **GP-1, I-036** was obtained as a colorless oil in 40% yield using 3-bromo-4-methoxy-benzenethiol (200mg, 0.913mmol, 1 equiv.), (1-methyl-3-phenyl-propyl) methanesulfonate (250mg, 1.10mmol, 1.2 equiv.) and K₂CO₃ (189mg, 1.37mmol, 1.5 equiv.) in DMF (4.6mL) at RT for 2h. Purified by FC (cHex/EtOAc = 100/0 to 70/30). ¹H NMR (400 MHz, Chloroform-d) δ 7.63 (d, *J=* 2.2 Hz, 1H), 7.38 - 7.26 (m, 3H), 7.22 - 7.12 (m, 3H), 6.83 - 6.75 (m, 1H), 3.89 (s, 3H), 3.04 (h, *J=* 6.8 Hz, 1H), 2.81 - 2.70 (m, 2H), 2.01 - 1.68 (m, 2H), 1.29 - 1.25 (d, *J=* 6.8 Hz, 3H).

### 2-bromo-1-methoxy-4-(1-methyl-4-phenyl-butyl)sulfanyl-benzene (I-037)

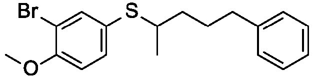

Formula Weight: 365,33

Molecular Formula: C₁₈H₂₁BrOS

Using **GP-1, I-037** was obtained as a colorless oil in 40% yield using 3-bromo-4-methoxy-benzenethiol (150mg, 0.684mmol, 1 equiv.), (1-methyl-3-phenyl-butyl) methanesulfonate (199mg, 0.821mmol, 1.2 equiv.) and K₂CO₃ (142mg, 1.03mmol, 1.5 equiv.) in DMF (4.6mL) at RT for 2h. Purified by FC (cHex/EtOAc = 100/0 to 70/30). ¹H NMR (400 MHz, Chloroform-d) δ 7.62 (d, *J=* 2.2 Hz, 1H), 7.35 - 7.27 (m, 3H), 7.23 - 7.03 (m, 3H), 6.80 (d, *J =* 8.5 Hz, 1H), 3.89 (s, 3H), 3.05 (h, *J =* 6.7 Hz, 1H), 2.66 - 2.56 (m, 2H), 1.88 - 1.69 (m, 2H), 1.67 - 1.46 (m, 2H), 1.22 (d, *J=* 6.7 Hz, 3H).

### 2-bromo-4-(1-ethyl-3-phenyl-propyl)sulfanyl-1-methoxy-benzene (I-038)

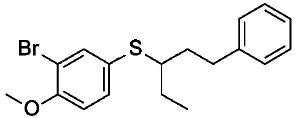

Formula Weight: 365,33

Molecular Formula : C₁₈H₂₁BrOS

Using **GP-1, I-038** was obtained as a colorless oil in 40% yield using 3-bromo-4-methoxy-benzenethiol (200mg, 0.913mmol, 1 equiv.), (1-methyl-3-phenyl-propyl) methanesulfonate (265mg, 1.10mmol, 1.2 equiv.) and K₂CO₃ (189mg, 1.37mmol, 1.5 equiv.) in DMF (4.6mL) at RT for 2h. Purified by FC (cHex/EtOAc = 100/0 to 70/30). ¹H NMR (400 MHz, Chloroform-d) δ 7.63 (d, *J=* 2.2 Hz, 1H), 7.37 - 7.26 (m, 3H), 7.23 - 7.13 (m, 3H), 6.81 (d, *J =* 8.4 Hz, 1H), 3.89 (s, 3H), 2.94 - 2.68 (m, 3H), 1.94 - 1.72 (m, 2H), 1.69 - 1.51 (m, 2H), 1.01 (t, *J=* 7.4 Hz, 3H).

### 2-bromo-4-(1-ethyl-4-phenyl-butyl)sulfanyl-1-methoxy-benzene (I-039)

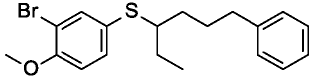

Formula Weight: 379,35

Molecular Formula: C₁₉H₂₃BrOS

Using **GP-1, I-039** was obtained as a colorless oil in 40% yield using 3-bromo-4-methoxy-benzenethiol (87mg, 0.40mmol, 1.2 equiv.), (1-ethyl-4-phenyl-butyl) methanesulfonate (85mg, 0.33mmol, 1.0 equiv.) and K₂CO₃ (69mg, 0.50mmol, 1.5 equiv.) in DMF (1.7mL) at RT for 2h. Purified by FC (cHex/EtOAc = 100/0 to 70/30). ¹H NMR (400 MHz, Chloroform-d) δ 7.53 (d, *J= 2.2* Hz, 1H), 7.26 - 7.16 (m, 3H), 7.16 7.04 (m, 3H), 6.71 (d, *J* = 8.5 Hz, 1H), 3.81 (s, 3H), 2.79 (p, *J =* 6.4 Hz, 1H), 2.53 (t, *J* = 7.6 Hz, 2H), 1.84 - 1.62 (m, 2H), 1.57 - 1.38 (m, 4H), 0.90 (t, *J=* 7.3 Hz, 3H).

### Synthesis of sulfones using GP-2:

### 2-bromo-4-(1-ethylpropylsulfonyl)-1-methoxy-benzene (I-012)

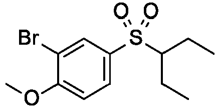

Formula Weight: 321,23

Molecular Formula: C₁₂H₁₇BrO₃S

Using **GP-2, I-012** was obtained as a colorless oil in 96% yield using **I-001** (200mg, 285µmol, 1 equiv.) and m-CPBA (70% wet, 371mg, 1.51mmol, 2.2 equiv.) in DCM (3.4mL) at RT for 2h. Purified by FC (cHex/EtOAc = 95/5 to 50/50). ¹H NMR (400 MHz, Chloroform-*d*) δ 8.07 (d, *J =* 2.2 Hz, 1H), 7.83 (dd, *J =* 8.6, 2.2 Hz, 1H), 7.03 (d, *J* = 8.7 Hz, 1H), 4.01 (s, 3H), 2.80 (tt, *J* = 7.2, 4.8 Hz, 1H), 1.97-1.81 (m, 2H), 1.80-1.63 (m, 2H), 1.03 (t, *J=* 7.5 Hz, 6H). MS (ESI⁺): [M+H]⁺ 321.0/323.0.

### 2-bromo-4-cyclopentylsulfonyl-1-methoxy-benzene (I-013)

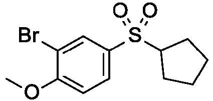

**Formula** Weight: 319,21

Molecular Formula: C₁₂H₁₅BrO₃S

Using **GP-2, I-013** was obtained as a colorless oil in 94% yield using **I-002** (590mg, 332µmol, 1 equiv.) and m-CPBA (70% wet, 1.01g, 4.52mmol, 2.2 equiv.) in DCM (3.4mL) at RT for 2h. Purified by FC (cHex/EtOAc = 95/5 to 50/50). ¹H NMR (400 MHz, Chloroform-*d*) δ 8.09 (d, *J=* 2.3 Hz, 1H), 7.84 (dd, *J* = 8.6, 2.3 Hz, 1H), 7.02 (d, *J* = 8.7 Hz, 1H), 4.00 (s, 3H), 3.48 (tt, *J* = 8.8, 7.2 Hz, 1H), 2.15-1.99 (m, 2H), 1.97-1.72 (m, 4H), 1.69-1.56 (m, 2H). MS (ESI⁺): [M+H]⁺ 319.0/321.0.

### 2-bromo-4-cyclohexylsulfonyl-1-methoxy-benzene (I-014)

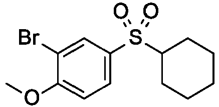

**Formula** Weight: 333,24

Molecular Formula: C₁₃H₁₇BrO₃S

Using **GP-2, I-014** was obtained as a colorless oil in 94% yield using **I-003** (100mg, 332µmol, 1 equiv.) and m-CPBA (70% wet, 275g, 1.23mmol, 3.7 equiv.) in DCM (2.2mL) at RT for 2h. Purified by FC (cHex/EtOAc = 95/5 to 50/50). ¹H NMR (400 MHz, Chloroform-*d*) δ 8.03 (d, *J =* 2.2 Hz, 1H), 7.78 (dd, *J =* 8.6, 2.2 Hz, 1H), 7.00 (d, *J =* 8.6 Hz, 1H), 3.98 (s, 3H), 2.87 (tt, *J=* 12.1, 3.4 Hz, 1H), 2.13-2.01 (m, 2H), 1.95-1.81 (m, 2H), 1.39 (qd, *J=* 12.3, 3.4 Hz, 2H), 1.28-1.09 (m, 4H). MS (ESI⁺): [M+H]⁺ 333.0/335.0.

### 2-bromo-4-(cyclopropylmethylsulfonyl)-1-methoxy-benzene (I-015)

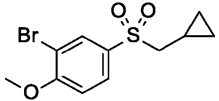

**Formula** Weight: 305,19

Molecular Formula: C₁₁H₁₃BrO₃S

Using **GP-2, I-015** was obtained as a colorless oil in 76% yield using **I-004** (130mg, 476µmol, 1 equiv.) and m-CPBA (70% wet, 258mg, 1.05mmol, 2.2 equiv.) in DCM (2.4mL) at RT for 2h. Purified by FC (cHex/EtOAc = 95/5 to 50/50). ¹H NMR (400 MHz, Chloroform-*d*) δ 8.11 (d, *J* = 2.3 Hz, 1H), 7.86 (dd, *J* = 8.6, 2.2 Hz, 1H), 7.01 (d, *J* = 8.7 Hz, 1H), 3.99 (s, 3H), 3.01 (d, *J =* 7.2 Hz, 2H), 1.09-0.94 (m, 1H), 0.66-0.53 (m, 2H), 0.22-0.09 (m, 2H). MS (ESI⁺): [M+H]⁺ 305.0/306.9.

### 2-bromo-4-cyclobutylsulfonyl-1-methoxy-benzene (I-016)

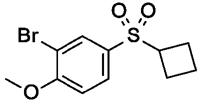

Formula Weight: 305,19

Molecular Formula: C₁₁H₁₃BrO₃S

Using **GP-2, I-016** was obtained as a colorless oil in 66% yield using **I-005** (150mg, 549µmol, 1 equiv.) and m-CPBA (70% wet, 298mg, 1.21mmol, 2.2 equiv.) in DCM (2.7mL) at RT for 2h. Purified by FC (cHex/EtOAc = 95/5 to 50/50). ¹H NMR (400 MHz, Chloroform-*d*) δ 8.03 (d, *J=* 2.2 Hz, 1H), 7.79 (dd, *J =* 8.6, 2.3 Hz, 1H), 6.99 (d, *J* = 8.7 Hz, 1H), 3.97 (s, 3H), 3.84-3.73 (m, 1H), 2.61-2.48 (m, 2H), 2.25-2.13 (m, 2H), 2.05-1.91 (m, 2H). MS (ESI⁺): [M+H]⁺ 304.9/307.0.

### 3-(3-bromo-4-methoxy-phenyl)sulfonyloxetane (I-017)

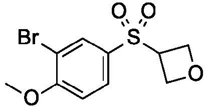

Formula Weight: 307,16

Molecular Formula: C₁₀H₁₁BrO₄S

Using **GP-2, I-017** was obtained as a colorless oil in 69% yield using **I-006** (130mg, 472µmol, 1 equiv.) and m-CPBA (70% wet, 256mg, 1.04mmol, 2.2 equiv.) in DCM (2.4mL) at RT for 16h. Purified by FC (cHex/EtOAc = 95/5 to 50/50). ¹H NMR (400 MHz, Chloroform-*d*) δ 8.07 (d, *J= 2.2* Hz, 1H), 7.85 (dd, *J=* 8.7, 2.3 Hz, 1H), 7.03 (d, *J* = 8.7 Hz, 1H), 4.96-4.89 (m, 2H), 4.80 (t, *J* = 7.7 Hz, 2H), 4.44 (tt, *J=* 8.1, 6.2 Hz, 1H), 3.99 (s, 3H). MS (ESI⁺): [M+H]⁺ 307.0/309.0.

### 2-bromo-4-(cyclobutylmethylsulfonyl)-1-methoxy-benzene (I-018)

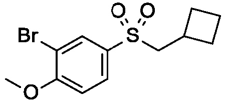

Formula Weight: 319,21

Molecular Formula: C₁₂H₁₅BrO₃S

Using **GP-2, I-018** was obtained as a colorless oil in 54% yield using **I-007** (100mg, 348µmol, 1 equiv.) and m-CPBA (70% wet, 119mg, 766µmol, 2.2 equiv.) in DCM (1.7mL) at RT for 16h. Purified by FC (cHex/EtOAc = 95/5 to 50/50). ¹H NMR (400 MHz, Chloroform-*d*) δ 8.03 (d, *J= 2.3* Hz, 1H), 7.79 (dd, *J* = 8.7, 2.3 Hz, 1H), 6.99 (d, *J* = 8.6 Hz, 1H), 3.97 (s, 3H), 3.18 (d, *J=* 7.3 Hz, 2H), 2.68 (tt, *J =* 15.6, 7.7 Hz, 1H), 2.12-2.01 (m, 2H), 1.98-1.68 (m, 4H). MS (ESI⁺): [M+H]⁺ 319.0/320.9.

### 2-bromo-4-(cyclopentylmethylsulfonyl)-1-methoxy-benzene (I-019)

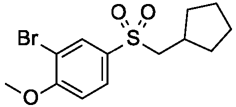

**Formula** Weight: 333,24

Molecular Formula: C₁₃H₁₇BrO₃S Using **GP-2, I-019** was obtained as a colorless oil in 100% yield using **I-008** (50mg, 166µmol, 1 equiv.) and m-CPBA (70% wet, 90mg, 0.37mmol, 2.2 equiv.) in DCM (0.8mL) at RT for 16h. Purified by FC (cHex/EtOAc = 95/5 to 50/50). ¹H NMR (400 MHz, Chloroform-*d*) δ 8.04 (d, *J= 2.3* Hz, 1H), 7.80 (dd, *J* = 8.6, 2.3 Hz, 1H), 6.99 (d, *J* = 8.7 Hz, 1H), 3.96 (s, 3H), 3.10 (d, *J=* 6.8 Hz, 2H), 2.28-2.15 (m, 1H), 1.92-1.81 (m, 2H), 1.66-1.47 (m, 4H), 1.26-1.14 (m, 2H). MS (ESI⁺): [M+H]⁺ 332.9/335.0.

### 4-benzylsulfonyl-2-bromo-1-methoxy-benzene (1-020)

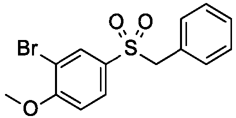

**Formula** Weight: 341,22

Molecular Formula: C₁₄H₁₃BrO₃S

Using **GP-2, I-020** was obtained as a colorless oil in 100% yield using **I-009** (240mg, 776µmol, 1 equiv.) and m-CPBA (70% wet, 421mg, 1.70mmol, 2.2 equiv.) in DCM (3.9mL) at RT for 2h. Purified by FC (cHex/EtOAc = 95/5 to 50/50). ¹H NMR (400 MHz, Chloroform-*d*) δ 7.76 (d, *J* = 2.3 Hz, 1H), 7.49 (dd, *J =* 8.6, 2.3 Hz, 1H), 7.38-7.27 (m, 3H), 7.13-7.07 (m, 2H), 6.86 (d, *J =* 8.7 Hz, 1H), 4.29 (s, 2H), 3.95 (s, 3H). MS (ESI⁺): [M+H]⁺ 341.0/342.9.

### 2-bromo-4-(2,2-dimethylpropylsulfonyl)-1-methoxy-benzene (I-021)

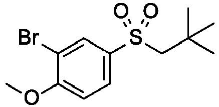

**Formula** Weight: 321,23

Molecular Formula: C₁₂H₁₇BrO₃S

Using **GP-2, I-021** was obtained as a colorless oil in 72% yield using **I-010** (50mg, 173µmol, 1 equiv.) and m-CPBA (70% wet, 94mg, 0.38mmol, 2.2 equiv.) in DCM (0.9mL) at RT for 16h. Purified by FC (cHex/EtOAc = 95/5 to 50/50). ¹H NMR (400 MHz, Chloroform-*d*) δ 8.08 (d, *J= 2.3* Hz, 1H), 7.84 (dd, *J=* 8.7, 2.3 Hz, 1H), 6.99 (d, *J* = 8.6 Hz, 1H), 3.98 (s, 3H), 3.01 (s, 2H), 1.19 (s, 9H). MS (ESI⁺): [M+H]⁺ 321.0/322.9.

### 2-bromo-4-isopropylsulfonyl-1-methoxy-benzene (I-022)

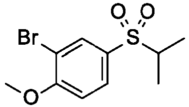

**Formula** Weight: 293.18

Molecular Formula: C₁₀H₁₃BrO₃S

Using **GP-2, I-022** was obtained as a colorless oil in 67% yield using **I-011** (60mg, 230µmol, 1 equiv.) and m-CPBA (70% wet, 125mg, 505µmol, 2.2 equiv.) in DCM (1.5mL) at RT for 2h. Purified by FC (cHex/EtOAc = 95/5 to 50/50). ¹H NMR (400 MHz, Chloroform-*d*) δ 8.04 (d, *J* = 2.3 Hz, 1H), 7.80 (dd, *J* = 8.6, 2.3 Hz, 1H), 7.01 (d, *J* = 8.7 Hz, 1H), 3.98 (s, 3H), 3.22-3.11 (m, 1H), 1.29 (d, *J =* 6.8 Hz, 6H). MS (ESI⁺): [M+H]⁺ 293.0/295.0.

### 2-bromo-1-methoxy-4-(1-methylbutylsulfonyl)benzene (I-026)

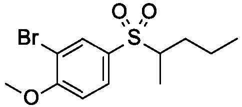

**Formula** Weight: 321.23

Molecular Formula: C₁₂H₁₇BrO₃S

Using **GP-2, I-026** was obtained as a colorless oil in 80% yield using **I-025** (1.64g, 5.68mmol, 1 equiv.) and m-CPBA (70% wet, 3.08g, 12.5mmol, 2.2 equiv.) in DCM (28mL) at RT for 2h. Purified by FC (cHex/EtOAc = 95/5 to 60/40). ¹H NMR (400 MHz, Chloroform-*d*) δ 8.04 (d, *J* = 2.2 Hz, 1H), 7.80 (dd, *J* = 8.6, 2.2 Hz, 1H), 7.01 (d, *J* = 8.7 Hz, 1H), 3.99 (s, 3H), 3.01 (dqd, *J=* 10.4, 6.9, 3.6 Hz, 1H), 1.92 (dddd, *J=* 12.7, 9.5, 5.7, 3.2 Hz, 1H), 1.53-1.31 (m, 2H), 1.29-1.22 (m, 4H), 0.91 (t, *J =* 7.2 Hz, 3H). MS (ESI⁺): [M+H]⁺ 320.9/323.0.

### 2-bromo-4-(2-cyclopentylethylsulfonyl)-1-methoxy-benzene (I-040)

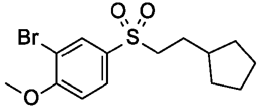

**Formula** Weight: 347,27

Molecular Formula: C₁₄H₁₉BrO₃S

Using **GP-2, I-040** was obtained as a colorless oil in 66% yield using **I-032** (100mg, 0.317mmol, 1 equiv.) and m-CPBA (70% wet, 172mg, 0.697mmol, 2.2 equiv.) in DCM (1.6mL) at RT for 1h. Purified by FC (cHex/EtOAc = 95/5 to 50/50). ¹H NMR (400 MHz, Chloroform-*d*) δ 8.07 (d, *J* = 2.2 Hz, 1H), 7.83 (dd, *J* = 8.6, 2.3 Hz, 1H), 7.01 (d, *J* = 8.7 Hz, 1H), 3.99 (s, 3H), 3.12 - 3.04 (m, 2H), 1.84 - 1.69 (m, 5H), 1.62 - 1.40 (m, 4H), 1.11 - 1.00 (m, 2H). MS (ESI⁺): [M+H]⁺ 347.0/349.0.

### 2-bromo-4-butylsulfonyl-1-methoxy-benzene (I-041)

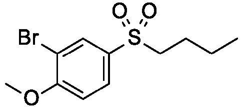

**Formula** Weight: 307,2

Molecular Formula: C₁₁H₁₅BrO₃S

Using **GP-2, I-041** was obtained as a colorless oil in 36% yield using **I-033** (100mg, 0.363mmol, 1 equiv.) and m-CPBA (70% wet, 197mg, 0.800mmol, 2.2 equiv.) in DCM (1.8mL) at RT for 16h. Purified by FC (cHex/EtOAc = 95/5 to 50/50). ¹H NMR (400 MHz, Chloroform-*d*) 1H NMR (400 MHz, Chloroform-*d*) δ 8.07 (d, *J=* 2.3 Hz, 1H), 7.83 (dd, *J* = 8.7, 2.3 Hz, 1H), 7.01 (d, *J* = 8.7 Hz, 1H), 3.99 (s, 3H), 3.11 - 3.03 (m, 2H), 1.74 - 1.64 (m, 2H), 1.50 - 1.30 (m, 2H), 0.90 (t, *J =* 7.4 Hz, 3H). MS (ESI⁺): [M+H]⁺ 307.0/308.9.

### 2-bromo-4-(2-ethylbutylsulfonyl)-1-methoxy-benzene (I-042)

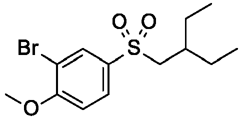

**Formula** Weight: 335,26

Molecular Formula: C₁₃H₁₉BrO₃S

Using **GP-2, I-042** was obtained as a colorless oil in 73% yield using **I-034** (235mg, 0.775mmol, 1 equiv.) and m-CPBA (70% wet, 420mg, 1.70mmol, 2.2 equiv.) in DCM (3.9mL) at RT for 16h. Purified by FC (cHex/EtOAc = 95/5 to 50/50). ¹H NMR (400 MHz, Chloroform-*d*) 1H NMR (400 MHz, Chloroform-*d*) δ 8.07 (d, *J=* 2.3 Hz, 1H), 7.83 (dd, *J* = 8.6, 2.3 Hz, 1H), 7.00 (d, *J =* 8.7 Hz, 1H), 3.98 (s, 3H), 3.00 (d, *J =* 6.0 Hz, 2H), 1.89 (hept, *J=* 6.1 Hz, 1H), 1.48 - 1.41 (m, 4H), 0.83 (t, *J=* 7.4 Hz, 6H). MS (ESI⁺): [M+H]⁺ 335.0/337.0.

### 2-bromo-1-methoxy-4-sec-butylsulfonyl-benzene (I-043)

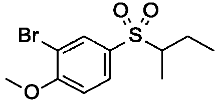

Formula Weight: 307,2

Molecular Formula: C₁₁H₁₅BrO₃S

Using **GP-2, I-043** was obtained as a colorless oil in 72% yield using **I-035** (50mg, 0.182mmol, 1 equiv.) and m-CPBA (70% wet, 98mg, 0.40mmol, 2.2 equiv.) in DCM (0.9mL) at RT for 16h. Purified by FC (cHex/EtOAc = 95/5 to 50/50). ¹H NMR (400 MHz, Chloroform-*d*) 1H NMR (400 MHz, Chloroform-*d*) δ 8.04 (d, *J=* 2.2 Hz, 1H), 7.80 (dd, *J=* 8.6, 2.2 Hz, 1H), 7.01 (d, *J* = 8.6 Hz, 1H), 3.99 (s, 3H), 2.98 - 2.87 (m, 1H), 2.07 - 1.94 (m, 1H), 1.49 - 1.36 (m, 1H), 1.27 (d, *J=* 6.9 Hz, 3H), 0.99 (t, *J =* 7.5 Hz, 3H). MS (ESI⁺): [M+H]⁺ 306.9/308.9.

### 2-bromo-1-methoxy-4-(1-methyl-3-phenyl-propyl)sulfonyl-benzene (I-044)

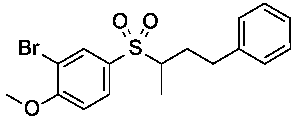

**Formula** Weight: 383,3

Molecular Formula: C₁₇H₁₉BrO₃S

Using **GP-2, I-044** was obtained as a colorless oil in 58% yield using **I-036** (156mg, 0.444mmol, 1 equiv.) and m-CPBA (70% wet, 274mg, 1.11mmol, 2.2 equiv.) in DCM (2.2mL) at RT for 2h. Purified by FC (cHex/EtOAc = 95/5 to 50/50). ¹H NMR (400 MHz, Chloroform-*d*) δ 8.00 (d, *J* = 2.2 Hz, 1H), 7.76 (dd, *J* = 8.6, 2.3 Hz, 1H), 7.31 - 7.24 (m, 2H), 7.24 - 7.17 (m, 1H), 7.14 - 7.08 (m, 2H), 6.98 (d, *J* = 8.7 Hz, 1H), 3.98 (s, 3H), 3.00 (dqd, *J=* 9.5, 6.9, 3.7 Hz, 1H), 2.82 (ddd, *J =* 14.3, 9.3, 5.3 Hz, 1H), 2.60 (ddd, *J =* 13.9, 8.9, 7.5 Hz, 1H), 2.29 (dddd, *J=* 13.2, 9.3, 7.5, 3.7 Hz, 1H), 1.80 - 1.64 (m, 1H), 1.32 (d, *J* = 6.9 Hz, 3H).

### 2-bromo-1-methoxy-4-(1-methyl-4-phenyl-butyl)sulfonyl-benzene (I-045)

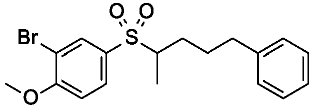

**Formula** Weight: 397,33

Molecular Formula: C₁₈H₂₁BrO₃S

Using **GP-2, I-045** was obtained as a colorless oil in 50% yield using **I-037** (185mg, 0.506mmol, 1 equiv.) and m-CPBA (70% wet, 308mg, 1.25mmol, 2.5 equiv.) in DCM (2.5mL) at RT for 2h. Purified by FC (cHex/EtOAc = 95/5 to 50/50). ¹H NMR (400 MHz, Chloroform-*d*) δ 8.00 (d, *J* = 2.2 Hz, 1H), 7.76 (dd, *J* = 8.6, 2.3 Hz, 1H), 7.31 - 7.24 (m, 2H), 7.24 - 7.17 (m, 1H), 7.14 - 7.08 (m, 2H), 6.98 (d, *J* = 8.7 Hz, 1H), 3.99 (s, 3H), 3.10 - 2.90 (m, 1H), 2.70 - 2.50 (m, 2H), 2.02 - 1.93 (m, 1H), 1.90 - 1.52 (m, 2H), 1.51 - 1.39 (m, 1H), 1.35 - 1.21 (m, 3H).

### 2-bromo-4-(1-ethyl-3-phenyl-propyl)sulfonyl-1-methoxy-benzene (I-046)

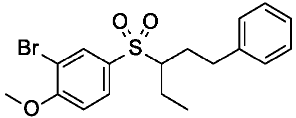

Formula Weight: 397,33

Molecular Formula: C₁₈H₂₁BrO₃S

Using **GP-2, I-046** was obtained as a colorless oil in 75% yield using **I-038** (228mg, 0.624mmol, 1 equiv.) and m-CPBA (70% wet, 384mg, 1.56mmol, 2.2 equiv.) in DCM (3.1mL) at RT for 2h. Purified by FC (cHex/EtOAc = 95/5 to 50/50). ¹H NMR (400 MHz, Chloroform-*d*) δ 8.01 (d, *J=* 2.3 Hz, 1H), 7.77 (dd, *J=* 8.7, 2.2 Hz, 1H), 7.31 - 7.23 (m, 2H), 7.22 7.15 (m, 1H), 7.14 - 7.07 (m, 2H), 6.98 (d, *J* = 8.7 Hz, 1H), 3.99 (s, 3H), 2.89 - 2.74 (m, 2H), 2.67 (ddd, *J* = 13.9, 9.5, 6.6 Hz, 1H), 2.14 (dddd, *J =* 14.6, 9.5, 6.6, 5.1 Hz, 1H), 1.97 - 1.82 (m, 2H), 1.69 (dp, *J =* 14.7, 7.4 Hz, 1H), 1.00 (t, *J=* 7.5 Hz, 3H).

### 2-bromo-4-(1-ethyl-4-phenyl-butyl)sulfonyl-1-methoxy-benzene (I-047)

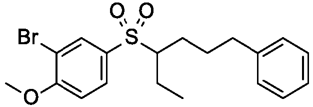

Formula Weight: 411,35

Molecular Formula: C₁₉H₂₃BrO₃S

Using **GP-2, I-047** was obtained as a colorless oil in 38% yield using **I-039** (61mg, 0.16mmol, 1 equiv.) and m-CPBA (70% wet, 98mg, 0.40mmol, 2.5 equiv.) in DCM (0.8mL) at RT for 2h. Purified by FC (cHex/EtOAc = 95/5 to 50/50). ¹H NMR (400 MHz, Chloroform-*d*) δ 8.01 (d, *J=* 2.3 Hz, 1H), 7.72 (dd, *J=* 8.6, 2.2 Hz, 1H), 7.30 - 7.25 (m, 2H), 7.21 - 7.04 (m, 3H), 6.95 (d, *J=* 8.7 Hz, 1H), 3.98 (s, 3H), 2.81 (dt, *J* = 6.8, 4.5 Hz, 1H), 2.59 (t, *J =* 6.9 Hz, 2H), 1.99 - 1.57 (m, 6H), 0.97 (t, *J =* 7.5 Hz, 3H).

### Synthesis of common intermediate piperazines I-028 to I-031:

### tert-butyl 4-[4-fluoro-2-(trifluoromethyl)benzoyl]piperazine-1-carboxylate (Boc-I-028)

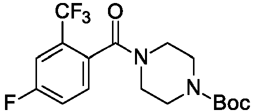

Formula Weight: 376,35

Molecular Formula: C₁₇H₂₀F₄N₂O₃

To a solution of 4-fluoro-2-(trifluoromethyl)benzoic acid (12.0g, 57.7mmol, 1 equiv.) in THF (180mL) at RT, was added TBTU (18.5g, 57.7mmol, 1 equiv.). The mixture was stirred 15min at RT, and a solution of tert-butyl piperazine-1-carboxylate (12.9g, 69.2mmol, 1.2 equiv.) and Et₃N (12.1mL, 86.5mmol, 1.5 equiv.) in THF (60mL) was added dropwise. The mixture was stirred at RT for 60h. The reaction mixture was concentrated under reduced pressure. The reaction mixture was partitioned between 1N HCl and EtOAc. The layers were separated and the aqueous phase was extracted with EtOAc(2*). The combined organic extracts were washed (as. 1N HCl, aq. sat. NaHCO₃, brine), dried (Na₂SO₄), filtered and concentrated under reduced pressure to afford 23.1g (100%) of tert-butyl 4-[4-fluoro-2-(trifluoromethyl)benzoyl]piperazine-1-carboxylate **(Boc-I-028)** as a brown oil that solidifies upon standing. ¹H NMR (400 MHz, Chloroform-*d*) δ 7.43 (dd, *J* = 8.6, 2.1 Hz, 1H), 7.34 - 7.28 (m, 2H), 3.90 - 3.65 (m, 2H), 3.62 - 3.44 (m, 2H), 3.35 (dd, *J* = 6.4, 4.0 Hz, 2H), 3.15 (dd, *J* = 6.5, 4.0 Hz, 2H), 1.46 (s, 9H). MS (ESI⁺): [M+H]⁺ 377.1.

### [4-fluoro-2-(trifluoromethyl)phenyl]-piperazin-1-yl-methanone (I-028)

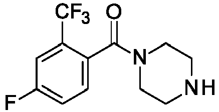

Formula Weight: 276,23

Molecular Formula: C₁₂H₁₂F₄N₂O

To a solution of crude tert-butyl 4-(4-fluoro-2-(trifluoromethyl)benzoyl)piperazine-1-carboxylate **Boc-I-028** (23.1g, 57.9mmol, 1 equiv.) in dioxane (25 mL) at RT, was added HCl (4M solution in dioxane, 75.0mL, 300 mmol, 5 equiv.). The mixture was stirred at RT for 16h. The reaction mixture was concentrated under reduced pressure and the residue was partitioned between water and DCM. K₂CO₃(s) was added portionwise until pH >11. The layers were separated and the aqueous phase was extracted with DCM (2*). The combined organic extracts were dried (Na₂SO₄), filtered and concentrated under reduced pressure. The residue was purified by FC (120g column, dry load, DCM/MeOH = 98/2 to 80/20) to afford 13.6g (83%) of [4-fluoro-2-(trifluoromethyl)phenyl]-piperazin-1-yl-methanone **I-028** as an orange solid. ¹H NMR (400 MHz, Chloroform-*d*) δ 7.41 (dd, *J* = 8.8, 2.4 Hz, 1H), 7.32 (qd, *J =* 8.4, 5.6 Hz, 2H), 3.87-3.69 (m, 2H), 3.15 (t, *J =* 5.1 Hz, 2H), 2.94 (t, *J =* 5.2 Hz, 2H), 2.76 (tq, *J =* 12.1, 6.1, 5.0 Hz, 2H). MS (ESI⁺): [M+H]⁺ 277.4.

### tert-butyl 4-(2-chloro-4-fluoro-benzoyl)piperazine-1-carboxylate (Boc-I-029)

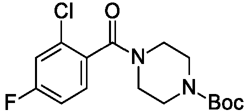

Formula Weight: 342,79

Molecular Formula: C₁₆H₂₀ClFN₂O₃

To a solution of 2-chloro-4-fluorobenzoic acid (5.00g, 28.6mmol, 1 equiv.) in THF (89mL) at RT, was added TBTU (9.20g, 28.6mmol, 1 equiv.). The mixture was stirred 15min at RT, and a solution of tert-butyl piperazine-1-carboxylate (6.40g, 34.4mmol, 1.2 equiv.) and Et₃N (6.0mL, 43mmol, 1.5 equiv.) in THF (30mL) was added dropwise. The mixture was stirred at RT for 60h. The reaction mixture was concentrated under reduced pressure. The reaction mixture was partitioned between 1N HCl and EtOAc. The layers were separated and the aqueous phase was extracted with EtOAc(2*). The combined organic extracts were washed (aq. 1N HCl, aq. sat. NaHCO₃, brine), dried (Na₂SO₄), filtered and concentrated under reduced pressure to afford 10.2g (100%) of tert-butyl 4-(2-chloro-4-fluoro-benzoyl)piperazine-1-carboxylate **(Boc-I-029)** as an orange oil. ¹H NMR (400 MHz, Chloroform-*d*) δ 7.32-7.29 (m, 1H), 7.18 (dd, *J =* 8.5, 2.5 Hz, 1H), 7.07 (td, *J=* 8.3, 2.5 Hz, 1H), 3.92-3.79 (m, 1H), 3.73 (dt, *J=* 13.2, 5.7 Hz, 1H), 3.63-3.50 (m, 2H), 3.48-3.34 (m, 2H), 3.34-3.10 (m, 2H), 1.48 (s, 9H). MS (ESI⁺): [M+H]⁺ 343.1/345.1.

### (2-chloro-4-fluoro-phenyl)-piperazin-1-yl-methanone (I-029)

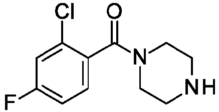

Formula Weight: 242,68

Molecular Formula: C₁₁H₁₂ClFN₂O

To a solution of crude tert-butyl 4-(2-chloro-4-fluoro-benzoyl)piperazine-1-carboxylate **Boc-I-029** (10.2g, 28.6mmol, 1 equiv.) in dioxane (37 mL) at RT, was added HCl (4M solution in dioxane, 37mL, 148 mmol, 5 equiv.). The mixture was stirred at RT for 16h. The reaction mixture was concentrated under reduced pressure and the residue was partitioned between water and DCM. K₂CO₃(s) was added portionwise until pH >11. The layers were separated and the aqueous phase was extracted with DCM (2*). The combined organic extracts were dried (Na₂SO₄), filtered and concentrated under reduced pressure. The residue was purified by FC (80g column, dry load, DCM/MeOH = 98/2 to 80/20) to afford 5.24g (72%) of (2-chloro-4-fluoro-phenyl)-piperazin-1-yl-methanone **I-029** as an orange solid. ¹H NMR (400 MHz, Chloroform-*d*) δ 7.41 (dd, *J=* 8.8, 2.4 Hz, 1H), 7.32 (qd, *J =* 8.4, 5.6 Hz, 2H), 3.87-3.69 (m, 2H), 3.15 (t, *J =* 5.1 Hz, 2H), 2.94 (t, *J* = 5.2 Hz, 2H), 2.80 - 2.70 (m, 2H). MS (ESI⁺): [M+H]⁺ 243.1/245.1.

### tert-butyl 3-(2-chloro-4-fluoro-benzoyl)-3,8-diazabicyclo[3.2.1]octane-8-carboxylate (Boc-I-030)

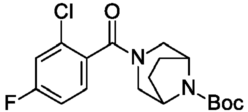

Formula Weight: 368,83

Molecular Formula: C₁₈H₂₂ClFN₂O₃

To a solution of 2-chloro-4-fluorobenzoic acid (9.87g, 56.5mmol, 1.2 equiv.) in DMF (118mL) at RT, was added TBTU (18.1g, 56.5mmol, 1.2 equiv.). The mixture was stirred 15min at RT, and a solution of tert-butyl 3,8-diazabicyclo[3.2.1]octane-8-carboxylate (10.0g, 47.1mmol, 1 equiv.) and Et₃N (9.8mL, 71mmol, 1.5 equiv.) in THF (118mL) was added dropwise. The mixture was stirred at RT for 16h. The reaction mixture was concentrated under reduced pressure. The reaction mixture was partitioned between aq. sat. NH₄Cl and EtOAc. The layers were separated and the aqueous phase was extracted with EtOAc(2*). The combined organic extracts were washed (aq. sat. NH₄Cl, aq. sat. NaHCO₃, brine), dried (Na₂SO₄), filtered and concentrated under reduced pressure to afford 21.3g (76% pure, 93%) of tert-butyl 3-(2-chloro-4-fluoro-benzoyl)-3,8-diazabicyclo[3.2.1]octane-8-carboxylate **(Boc-I-030)** as an orange oil. ¹H NMR (400 MHz, Chloroform-*d*) δ 7.33 (dd, *J* = 8.5, 5.9 Hz, 0.5H), 7.23-7.09 (m, 1.5H), 7.09-6.97 (m, 1H), 4.54-4.40 (m, 1H), 4.33 (s, 1H), 4.23-4.01 (m, 1H), 3.56-3.17 (m, 1H), 3.17-2.93 (m, 2H), 2.11-1.70 (m, 4H), 1.47 (s, 9H). MS (ESI⁺): [M+H]⁺ 369.1/371.1.

### (2-chloro-4-fluoro-phenyl)-(3,8-diazabicyclo[3.2.1]octan-3-yl)methanone (I-030)

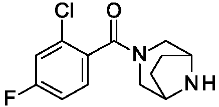

Formula Weight: 268,71

Molecular Formula: C₁₃H₁₄ClFN₂O

To a solution of crude tert-butyl 3-(2-chloro-4-fluoro-benzoyl)-3,8-diazabicyclo[3.2.1]octane-8-carboxylate **Boc-I-030** (21.3g, 76% pure, 43.9mmol, 1 equiv.) in dioxane (55 mL) at RT, was added HCl (4M solution in dioxane, 55mL, 220 mmol, 5 equiv.). The mixture was stirred at RT for 60h. The reaction mixture was concentrated under reduced pressure and the residue was partitioned between water and DCM. K₂CO₃(s) was added portionwise until pH >11. The layers were separated and the aqueous phase was extracted with DCM (2*). The combined organic extracts were dried (Na₂SO₄), filtered and concentrated under reduced pressure. The residue was purified by FC (220g column, dry load, DCM/MeOH (7N NH₃) = 99/1 to 95/5) to afford 10.3g (88%) of (2-chloro-4-fluoro-phenyl)-(3,8-diazabicyclo[3.2.1]octan-3-yl)methanone **I-030** as a white solid. ¹H NMR (400 MHz, Chloroform-*d*) δ 7.32 (dd, *J* = 8.5, 5.9 Hz, 0.5H), 7.20-7.15 (m, 0.5H), 7.15-7.10 (m, 1H), 7.09-6.98 (m, 1H), 4.47-4.37 (m, 1H), 3.69-3.56 (m, 1H), 3.44-3.34 (m, 1.5H), 3.22 (d, *J =* 11.9 Hz, 0.5H), 3.10-2.98 (m, 2H), 1.95-1.48 (m, 4H). MS (ESI⁺): [M+H]⁺ 269.1/271.0.

### tert-butyl 4-(2-chloro-4-fluoro-benzoyl)-2-methyl-piperazine-1-carboxylate (Boc-I-031)

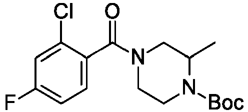

Formula Weight: 356,82

Molecular Formula: C₁₇H₂₂ClFN₂O₃

To a solution of 2-chloro-4-fluorobenzoic acid (10.5g, 59.9mmol, 1.2 equiv.) in THF (120mL) at RT, was added TBTU (19.2g, 59.9mmol, 1 equiv.). The mixture was stirred 15min at RT, and a solution of tert-butyl 2-methylpiperazine-1-carboxylate (10.0g, 49.9mmol, 1 equiv.) and Et₃N (6.5mL, 37mmol, 0.75 equiv.) in THF (120mL) was added dropwise. The mixture was stirred at RT for 60h. The reaction mixture was concentrated under reduced pressure. The reaction mixture was partitioned between aq. sat. NH₄Cl and EtOAc. The layers were separated and the aqueous phase was extracted with EtOAc(2*). The combined organic extracts were washed (aq. sat. NH₄Cl, aq. sat. NaHCO₃, brine), dried (Na₂SO₄), filtered and concentrated under reduced pressure. The residue was purified by FC (220g column, dry load, cHex/EtOAc = 90/10 to 50/50) to afford 16.8g (94%) of tert-butyl 4-(2-chloro-4-fluoro-benzoyl)-2-methyl-piperazine-1-carboxylate **(Boc-1-031)** as a white solid. ¹H NMR (400 MHz, Chloroform-*d*, multiple sets of rotamers) δ 7.36-7.21 (m, 1H), 7.20-7.09 (m, 1H), 7.08-6.97 (m, 1H), 4.67-4.13 (m, 2H), 3.99-3.74 (m, 1H), 3.41-3.23 (m, 1H), 3.23-2.83 (m, 3H), 1.44 (s, 9H), 1.29-0.97 (m, 3H). MS (ESI⁺): [M+H]⁺ 357.0/359.0.

### (2-chloro-4-fluoro-phenyl)-(3-methylpiperazin-1-yl)methanone (I-031)

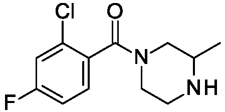

**Formula** Weight: 256,7

Molecular Formula: C₁₂H₁₄ClFN₂O

To a solution of crude tert-butyl 4-(2-chloro-4-fluoro-benzoyl)-2-methyl-piperazine-1-carboxylate **Boc-I-031** (16.8g, 47.1mmol, 1 equiv.) in DCM (235mL) at 0°C, was added TFA (72mL, 941mmol, 20 equiv.). The mixture was stirred at RT for 2h. The reaction mixture was concentrated under reduced pressure and the residue was partitioned between water and DCM at 0°C. K₂CO₃(s) was added portionwise until pH >11. The layers were separated and the aqueous phase was extracted with DCM (2*). The combined organic extracts were dried (Na₂SO₄), filtered and concentrated under reduced pressure. The residue was purified by FC (80g column, dry load, DCM/MeOH (7N NH₃) = 99/1 to 90/10) to afford 9.8g (81%) of (2-chloro-4-fluoro-phenyl)-(3-methylpiperazin-1-yl)methanone **I-031** as a white solid. ¹H NMR (400 MHz, Chloroform-*d*, 2 sets of rotamers) δ 7.32 (ddd, *J =* 8.6, 5.9, 1.3 Hz, 0.5H), 7.25 (ddd, *J* = 8.5, 5.9, 1.4 Hz, 0.5H), 7.21-7.13 (m, 1H), 7.09-7.01 (m, 1H), 4.69-4.56 (m, 1H), 3.31-2.75 (m, 5H), 2.51 (m, 1H), 1.15 (dd, *J* = 6.3, 2.4 Hz, 1.5H), 0.98 (dd, *J* = 6.3, 4.5 Hz, 1.5H). MS (ESI+): [M+H]+ 257.1/259.1.

### Synthesis of intermediates required for final products 016 and 017

### 1-(5-isobutylsulfonyl-2-methoxy-phenyl)-2-methyl-piperazine (I-027)

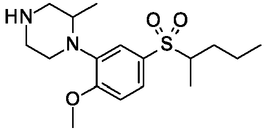

Formula Weight: 340.48

Molecular Formula: C₁₇H₂₈N₂O₃S

A microwave reaction vial was charged with the sulfone **I-026** (1.53g, 4.77mmol, 1 equiv.), commercial *tert*-butyl 3-methylpiperazine-1-carboxylate (1.15g, 5.73mmol, 1.2 equiv.), Cs₂CO₃ (4.66g, 14.3mmol, 3 equiv.), Pd(OAc)₂ (107mg, 477µmol, 0.1 equiv.) and rac-BINAP (297mg, 477µmol, 0.1 equiv.). The vial was flushed with argon and degassed toluene (24mL) was added. The vial was sealed and the reaction was stirred at reflux in a preheated heating-block for 16h. After cooling down to RT, EtOAc was added and the suspension was filtered over Celite^{®} (EtOAc rinses). The filtrate was concentrated under reduced pressure. The residue was directly treated with HCl (4M solution in dioxane, 1.68mL, 6.72mmol, 10 equiv.). The mixture was stirred at RT for 4h. The reaction mixture was concentrated under reduced pressure and the residue was partitioned between water and DCM. NaOH 2M was added dropwise until pH > 11. The layers were separated and the aqueous phase was extracted with DCM (2*). The combined organic extracts were dried (Na₂SO₄), filtered and concentrated under reduced pressure. The residue was purified by FC (24g column, dry load, DCM/MeOH (7N NH₃) = 99/1 to 95/5) to afford 160mg (9% over two steps) of **I-027** as a yellow oil. ¹H NMR (400 MHz, Chloroform-*d*) δ 7.55 (dd, *J* = 8.6, 2.3 Hz, 1H), 7.42 (d, *J=* 2.3 Hz, 1H), 6.97 (d, *J=* 8.6 Hz, 1H), 3.93 (s, 3H), 3.65-3.51 (m, 1H), 3.25 (ddd, *J=* 11.2, 5.0, 2.1 Hz, 1H), 3.21-3.14 (m, 1H), 3.14-2.94 (m, 3H), 2.84-2.71 (m, 2H), 1.95-1.87 (m, 1H), 1.54-1.27 (m, 3H), 1.25 (d, *J =* 6.9 Hz, 4H), 0.93 (dd, *J=* 6.4, 1.3 Hz, 3H), 0.89 (t, *J* = 7.2 Hz, 3H). MS (ESI⁺): [M+H]⁺ 341.1.

### Synthesis of intermediates required for final product 018

*[4-fluoro-2-(trifluoromethyl)phenyl]-[4-(5-isobutylsulfanyl-2-methoxyphenyl)piperazin-1-yl]methanone **(I-024)*** 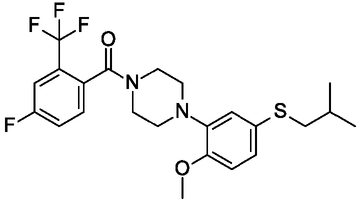 Formula Weight: 470.52

Molecular Formula: C₂₃H₂₆F₄N₂O₂S

A microwave reaction vial was charged with the thioether **I-023** (180mg, 654µmol, 1 equiv.), piperazine **I-028** (271mg, 981µmol, 1.5 equiv.), Cs₂CO₃ (426mg, 1.31mmol, 2 equiv.), Pd(OAc)₂ (15mg, 67µmol, 0.1 equiv.) and rac-BINAP (50mg, 81µmol, 0.12 equiv.). The vial was flushed with Ar and degassed toluene (3.4mL) was added. The vial was sealed and the reaction was stirred at reflux in a preheated heating-block for 4h30. After cooling down to RT, EtOAc was added and the suspension was filtered over Celite (EtOAc rinses). The filtrate was concentrated under reduced pressure. The residue was purified by FC (cHex/EtOAc = 100/0 to 30/70) affording **I-024** in 83% yield. ¹H NMR (400 MHz, Chloroform-*d*) δ 7.43 (dd, *J=* 8.9, 2.5 Hz, 1H), 7.38 (dd, *J=* 8.6, 5.3 Hz, 1H), 7.32 (td, *J =* 8.3, 2.6 Hz, 1H), 7.06 (d, *J =* 8.4 Hz, 1H), 6.97-6.89 (m, 1H), 6.80 (d, *J* = 8.4 Hz, 1H), 4.04-3.91 (m, 2H), 3.85 (s, 3H), 3.43-3.30 (m, 2H), 3.20-3.06 (m, 2H), 3.05-2.88 (m, 2H), 2.73 (d, *J=* 6.8 Hz, 2H), 1.81 (hept, *J* = 6.7 Hz, 1H), 1.01 (d, *J=* 6.7 Hz, 6H). ¹⁹F NMR (376 MHz, Chloroform-*d*) δ -60.24, -109.44. MS (ESI⁺): [M+H]⁺ 471.1.

### Synthesis of intermediates required for final product 027, 028 & 029

### tert-butyl 4-(3-bromo-4-methoxy-phenyl)sulfonylpiperidine-1-carboxylate (I-048)

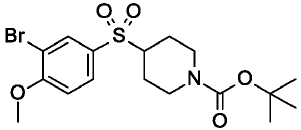

Formula Weight: 434,35

Molecular Formula: C₁₇H₂₄BrNO₅S

To a solution of 3-bromo-4-methoxy benzenethiol (1.38g, 6.31mmol, 1.05 equiv.) in MeCN (30mL) at RT, were added t-Butyl 4-(methanesulfonyloxy)piperidine-1-carboxylate (1.68g, 6.01mmol, 1 equiv.) and K₂CO₃ (1.25g, 9.02mmol, 1.5 equiv.). The mixture was stirred at 80°C for 16h. After cooling down to RT, the suspension was filtered over fritted glass and the filtrate was concentrated under reduced pressure. The residue was solubilized in DCM (26mL) at 0°C and m-CPBA (70% wet, 9.36g, 41.7 mmol, 7 equiv.) was added. The mixture was stirred at RT for 16h. Aq. sat. Na₂S₂O₃, aq. sat. NaHCO₃ and EtOAc were added. The mixture was vigorously stirred for 10 min. The layers were separated and the aqueous phase was extracted with EtOAc. The combined organic extracts were washed (brine), dried (Na₂SO₄), filtered and concentrated under reduced pressure. The residue was purified by FC (cHex/EtOAc = 95/5 to 50/50) affording **I-048** in 36% yield. ¹H NMR (400 MHz, Chloroform-*d*) δ 8.03 (d, *J =* 2.2 Hz, 1H), 7.78 (dd, *J =* 8.7, 2.3 Hz, 1H), 7.02 (d, *J =* 8.7 Hz, 1H), 4.23 (s, 2H), 3.99 (s, 3H), 3.01 (tt, *J* = 12.1, 3.6 Hz, 1H), 2.66 (s, 2H), 1.98 (d, *J* = 12.9 Hz, 2H), 1.59 (dd, *J* = 12.4, 4.9 Hz, 2H), 1.44 (s, 9H). MS (ESI⁺): [M+H]⁺ 434.1/436.1.

### tert-butyl 4-[3-[3-(2-chloro-4-fluoro-benzoyl)-3,8-diazabicyclo[3.2.1]octan-8-yl]-4-methoxy-phenyl]sulfonylpiperidine-1-carboxylate (I-049)

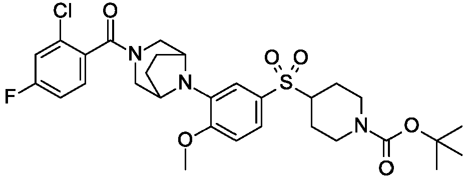

Formula Weight: 622,15

Molecular Formula: C₃₀H₃₇ClFN₃O₆S

A microwave reaction vial was charged with the sulfone **I-048** (500mg, 1.15mmol, 1 equiv.), piperazine **I-030** (371mg, 1.38mmol, 1.2 equiv.), Cs₂CO₃ (1.12g, 3.45mmol, 3 equiv.), Pd(OAc)₂ (25.8mg, 115µmol, 0.1 equiv.) and rac-BINAP (108mg, 172µmol, 0.15 equiv.). The vial was flushed with Ar and degassed toluene (5.8mL) was added. The vial was sealed and the reaction was stirred at reflux in a preheated heating-block for 16h. After cooling down to RT, EtOAc was added and the suspension was filtered over Celite (EtOAc rinses). The filtrate was concentrated under reduced pressure. The residue was purified by FC (cHex/EtOAc = 90/10 to 0/100) affording **I-049** in 76% yield. ¹H NMR (400 MHz, Chloroform-*d*, *2 sets of rotamers)* δ 7.45 - 7.32 (m, 1.5H), 7.24 - 7.12 (m, 2.5H), 7.12 - 7.00 (m, 1H), 6.97 (d, *J=* 8.5 Hz, 1H), 4.56 - 4.45 (m, 1H), 4.35 - 4.25 (m, 1H), 4.25 - 4.15 (m, 2H), 4.06 (s, 1H), 3.94 & 3.93 (s, 3H), 3.67 & 3.50 (d, *J=* 12.4 Hz, 1H), 3.33 - 3.23 (m, 1H), 3.20 - 3.07 (m, 1H), 3.02 - 2.90 (m, 1H), 2.64 (s, 2H), 2.00 - 1.85 (m, 4.5H), 1.75 - 1.65 (m, 0.5H), 1.60 - 1.50 (m, 3H), 1.43 (s, 9H). ¹⁹F NMR (376 MHz, Chloroform-d, *2 sets of rotamers)* δ -109.21, -109.33. MS (ESI+): [M+H]⁺ 622.3/624.3.

### (2-chloro-4-fluoro-phenyl)-[8-[2-methoxy-5-(4-piperidylsulfonyl)phenyl]-3,8-diazabicyclo[3.2.1]octan-3-yl]methanone;hydrochloride (I-050)

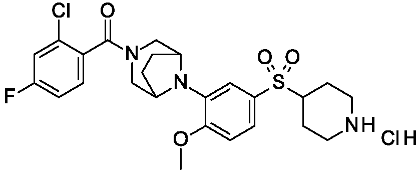

Formula Weight: 558,49

Molecular Formula: C₂₅H₂₉ClFN₃O₄S.ClH

To a solution of **I-049** (544mg, 0.874mmol, 1.00 equiv.) diluted in DCM (4.3mL) at RT, was added HCl 4N in dioxane (4.37mL, 17.5mmol, 20.0 equiv.). The resulting solution was stirred at RT for 2h. The reaction was concentrated under reduced pressure to afford **I-050** in 100% yield. ¹H NMR (400 MHz, DMSO-d6, *2 sets of rotamers)* δ 9.19 & 9.16 (s, 1H), 8.71 & 8.67 (s, 1H), 7.65 - 7.40 (m, 2H), 7.39 - 7.25 (m, 2H), 7.21 (d, *J =* 8.6 Hz, 1H), 7.11 (d, *J= 2.2* Hz, 1H), 4.43 - 4.23 (m, 2H), 3.89 (s, 3H), 3.55 - 3.25 (m, 4H), 3.18 - 2.92 (m, 2H), 2.84 (q, *J =* 12.0 Hz, 2H), 2.05 - 1.55 (m, 8H). ¹⁹F NMR (376 MHz, DMSO-d6, *2 sets of rotamers)* δ -110.01, -110.22. MS (ESI+): [M-Cl]⁺ 522.1/523.9.

### Synthesis of intermediates required for final product 030 & 031

### 2-bromo-4-(4-piperidylsulfonyl)phenol (I-051)

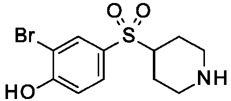

Formula Weight: 320,2

Molecular Formula: C₁₁H₁₄BrNO₃S

To a solution of **I-048** (406mg, 0.935mmol, 1.00 equiv.) in DCM (4.7mL) at RT, was added BBr₃ (1.0M solution in DCM, 1.87mL, 1.87mmol, 2 equiv. The resulting solution was stirred at RT for 30h. Water was added dropwise and the pH of the aqueous phase was adjusted to 11 using 2N NaOH. Both phases were concentrated under reduced pressure. The residue was dissolved in DCM/MeOH (50/50) and the resulting suspension was filtered over Celite. The filtrate was concentrated under reduced pressure. The residue was purified by FC (DCM/MeOH = 99/1 to 80/20) affording **I-051** in 97% yield. ¹H NMR (400 MHz, DMSO-*d6*) δ 7.85 (d, *J* = 2.3 Hz, 1H), 7.65 (dd, *J* = 8.6, 2.3 Hz, 1H), 7.17 (d, *J =* 8.6 Hz, 1H), 3.61 - 3.47 (m, 1H), 3.30 - 3.25 (m, 4H), 2.08 - 1.94 (m, 2H), 1.78 - 1.51 (m, 2H).MS (ESI+): [M+H]⁺ 320.0/322.0.

### 1-[4-(3-bromo-4-hydroxy-phenyl)sulfonyl-1-piperidyl]-2,2,2-trifluoro-ethanone (I-052)

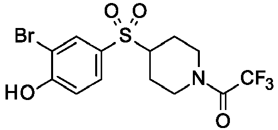

Formula Weight: 416,21

Molecular Formula: C₁₃H₁₃BrF₃NO₄S

To a solution of **I-051** (298 mg, 0.93mmol, 1.00 equiv.) and Et₃N (0.26mL, 1.9mmol, 2 equiv.) in DCM (4.7mL) at RT, was added trifluoroacetic anhydride (0.19mL, 1.4mmol, 1.5 equiv.). The mixture was stirred at RT for 2h. HCl 1N was added. The aqueous phase was extracted with EtOAc. The combined organic layers were washed with HCl 1N, aq. sat. NaHCO₃ and brine, dried (Na₂SO₄), filtered and concentrated under reduced pressure. The residue was purified by FC (cHex/EtOAc = 95/5 to 0/100) affording **I-052** in 39% yield. ¹H NMR (400 MHz, Chloroform-*d*) δ 8.00 (d, *J =* 2.2 Hz, 1H), 7.73 (dd, *J =* 8.6, 2.2 Hz, 1H), 7.19 (d, *J =* 8.6 Hz, 1H), 6.19 (s, 1H), 4.63 (d, *J =* 13.8 Hz, 1H), 4.14 (d, *J* = 7.1 Hz, 1H), 3.14 (dddd, *J =* 12.3, 8.5, 6.1, 3.3 Hz, 2H), 2.90 - 2.73 (m, 1H), 2.14 (t, *J* = 15.7 Hz, 2H), 1.90 - 1.69 (m, 2H). ¹⁹F NMR (376 MHz, Chloroform-*d*) δ -69.0. MS (ESI+): [M+H]⁺ 416.0/418.0.

### 1-[4-[3-bromo-4-(methoxymethoxy)phenyl]sulfonyl-1-piperidyl]-2,2,2-trifluoro-ethanone (I-053)

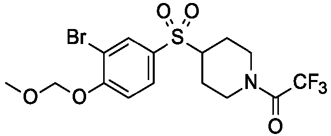

Formula Weight: 460,26

Molecular Formula: C₁₅H₁₇BrF₃NO₅S

To a solution of **I-052** (165 mg, 0.353mmol, 1.00 equiv.) and iPr₂EtN (0.18mL, 1.1mmol, 3 equiv.) in DCM (1.8mL) at RT, was added chloromethyl methyl ether (0.05mL, 0.7mmol, 2 equiv.). The mixture was stirred at RT for 2h. HCl 1N was added. The aqueous phase was extracted with DCM. The combined organic layers were washed with HCl 1N and brine, dried (Na₂SO₄), filtered and concentrated under reduced pressure affording **I-053** in 95% yield as a colorless oil. ¹H NMR (400 MHz, Chloroform-*d*) δ 8.05 (d, *J=* 2.3 Hz, 1H), 7.75 (dd, *J=* 8.7, 2.3 Hz, 1H), 7.31 (d, *J =* 8.7 Hz, 1H), 5.35 (s, 2H), 4.63 (d, *J* = 13.9 Hz, 1H), 4.14 (d, *J =* 14.2 Hz, 1H), 3.54 (s, 3H), 3.26 - 3.04 (m, 2H), 2.80 (t, *J =* 12.1 Hz, 1H), 2.22 - 2.03 (m, 2H), 1.87 - 1.66 (m, 2H).¹⁹F NMR (376 MHz, Chloroform-*d*) δ -69.0. MS (ESI+): [M+H]⁺ 460.0/461.9.

### 1-[4-[3-[3-(2-chloro-4-fluoro-benzoyl)-3,8-diazabicyclo[3.2.1]octan-8-yl]-4-(methoxymethoxy)phenyl]sulfonyl-1-piperidyl]-2,2,2-trifluoro-ethanone (I-054)

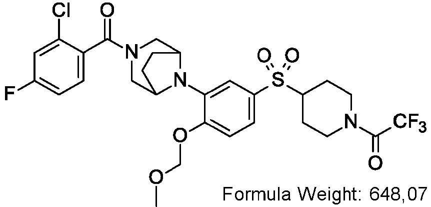

Molecular Formula: C₂₈H₃₀ClF₄N₃O₆S

A microwave reaction vial was charged with the sulfone **I-053** (155mg, 0.337mmol, 1 equiv.), piperazine **I-030** (109mg, 0.404mmol, 1.2 equiv.), Cs₂CO₃ (329mg, 1.01mmol, 3 equiv.), Pd(OAc)₂ (11.3mg, 50.5µmol, 0.15 equiv.) and rac-BINAP (62.9mg, 101µmol, 0.30 equiv.). The vial was flushed with Ar and degassed toluene (5.7mL) was added. The vial was sealed and the reaction was stirred at reflux in a preheated heating-block for 4h. After cooling down to RT, EtOAc was added and the suspension was filtered over Celite (EtOAc rinses). The filtrate was concentrated under reduced pressure. The residue was purified by FC (cHex/EtOAc = 90/10 to 0/100) affording **I-054** in 52% yield. ¹H NMR (400 MHz, Chloroform-*d*, *2 sets of rotamers)* δ 7.43 - 7.33 (m, 1.5H), 7.25 - 7.13 (m, 3.5H), 7.12 - 6.99 (m, 1H), 5.32 - 5.25 (m, 2H), 4.67 - 4.46 (m, 2H), 4.33 (s, 1H), 4.15 - 4.05 (m, 1H), 3.72 - 3.42 (m, 4H), 3.35 - 3.25 (m, 1H), 3.20 - 3.05 (m, 3H), 2.78 (t, *J* = 12.7 Hz, 1H), 2.04 (s, 7H), 1.79 - 1.58 (m, 2H). ¹⁹F NMR (376 MHz, Chloroform-d, 2 *sets of rotamers)* δ -69.0, -109.06, -109.20. MS (ESI+): [M+H]⁺ 648.2/650.1.

### (2-chloro-4-fluoro-phenyl)-[8-[2-(methoxymethoxy)-5-(4-piperidylsulfonyl)phenyl]-3,8-diazabicyclo[3.2.1]octan-3-yl]methanone (I-055)

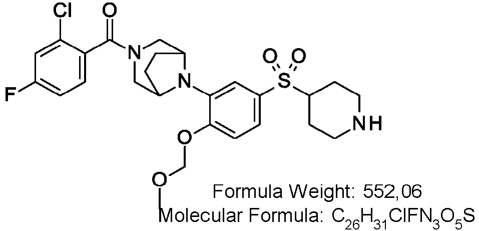

To a solution of sulfone **I-054** (130mg, 0.177mmol, 1 equiv.) in THF/MeOH/H₂O (3/1/1, 2mL) at RT, was added LiOH (21mg, 0.88mmol, 5 equiv.). The mixture was stirred at RT for 1h. Water and DCM were added. The layers were separated and the aqueous phase was extracted with DCM. The combined organic layers were dried (Na₂SO₄), filtered and concentrated under reduced pressure affording **I-055** in 76% yield. ¹H NMR (400 MHz, Chloroform-*d*, *2 sets of rotamers)* δ 7.40 - 7.32 (m, 1.5H) 7.23 - 7.12 (m, 3.5H), 7.12 - 7.00 (m, 1H), 5.29 - 5.23 (m, 2H), 4.58 - 4.45 (m, 1H), 4.32 (s, 1H), 4.08 (s, 1H), 3.70 - 3.45 (m, 4H), 3.28 (d, *J =* 12.9 Hz, 1H), 3.22 - 3.09 (m, 3H), 2.96 (tt, *J =* 12.2, 3.6 Hz, 1H), 2.55 (td, *J =* 12.4, 2.6 Hz, 2H), 2.11 - 1.88 (m, 5.5H), 1.70 - 1.60 (s, 0.5H), 1.58 - 1.50 (m, 3H). ¹⁹F NMR (376 MHz, Chloroform-d, *2 sets of rotamers)* δ -109.17, -109.31. MS (ESI+): [M+H]⁺ 552.2/554.1.

### (2-chloro-4-fluoro-phenyl)-[8-[2-(methoxymethoxy)-5-[(1-phenyl-4-piperidyl)sulfonyl]phenyl]-3,8-diazabicyclo[3.2.1]octan-3-yl]methanone (I-056)

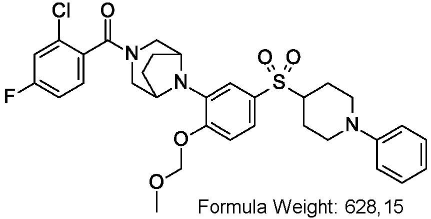

Molecular Formula: C₃₂H₃₅ClFN₃O₅S

A microwave reaction vial was charged with the amine **I-055** (50mg, 0.068mmol, 1 equiv.), bromobenzene (13mg, 0.082mmol, 1.2 equiv.), Cs₂CO₃ (55.3mg, 0.170mmol, 2.5 equiv.), Pd(OAc)₂ (3.1mg, 14µmol, 0.2 equiv.) and rac-BINAP (16.9mg, 27.2µmol, 0.40 equiv.). The vial was flushed with Ar and degassed toluene (0.7mL) was added. The vial was sealed and the reaction was stirred at reflux in a preheated heating-block for 16h. After cooling down to RT, EtOAc was added and the suspension was filtered over Celite (EtOAc rinses). The filtrate was concentrated under reduced pressure. The residue was purified by FC (cHex/EtOAc = 90/10 to 0/100) affording **I-056** in 62% yield. ¹H NMR (400 MHz, Chloroform-*d*, *2 sets of rotamers)* δ 7.50 - 7.30 (m, 5H), 7.25 - 7.18 (m, 4H), 7.10 - 7.00 (m, 1H), 6.95 - 6.85 (m, 1H), 5.38 - 5.18 (m, 2H), 4.60 - 4.48 (m, 1H), 4.33 (s, 1H), 4.09 (s, 1H), 3.80 - 3.70 (m, 1H), 3.70 - 3.48 (m, 4H), 3.52 (d, *J =* 3.8 Hz, 3H), 3.29 (d, *J=* 13.0 Hz, 1H), 3.15 (t, *J =* 10.5 Hz, 1H), 3.05 - 2.90 (m, 1H), 2.72 - 2.60 (m, 2H), 2.20 - 1.90 (m, 3.5H), 1.90 - 1.70 (m, 2H), 1.70 - 1.60 (m, 0.5H).¹⁹F NMR (376 MHz, Chloroform-*d*, *2 sets of rotamers)* δ -109.13, -109.27. MS (ESI+): [M+H]⁺ 628.2/630.2.

### (2-chloro-4-fluoro-phenyl)-[8-[2-(methoxymethoxy)-5-[[1-(2-phenylethyl)-4-piperidyl]sulfonyl]phenyl]-3,8-diazabicyclo[3.2.1]octan-3-yl]methanone (I-057)

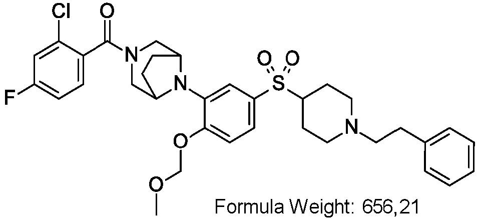

Molecular Formula: C₃₄H₃₉ClFN₃O₅S

To a solution of amine **I-055** (50mg, 0.068mmol, 1 equiv.) in DCM (0.7mL), were added acetic acid (7.8µL, 0.13mmol, 2 equiv.), phenylacetaldehyde (15.8µL, 0.136mmol, 2 equiv.) and NaBH(Oac)₃ (14mg, 0.068mmol, 1 equiv). The mixture was stirred at RT for 16h. Sat. aq. NaHCO₃ and DCM were added. The layers were separated and the aqueous phase was extracted with DCM. The combined organic extracts were dried (Na₂SO₄), filtered and concentrated under reduced pressure. The residue was purified by FC (DCM/MeOH = 99/1 to 90/10) affording I-057 in 80% yield. ¹H NMR (400 MHz, Chloroform-*d*, *2 sets of rotamers)* δ 7.41 - 7.30 (m, 3H), 7.23 - 7.14 (m, 6H), 7.12 - 6.96 (m, 2H), 5.34 - 5.19 (m, 2H), 4.56 - 4.45 (m, 1H), 4.32 (s, 1H), 4.08 (s, 1H), 3.70 - 3.45 (m, 4H), 3.30 & 3.27 (s, 1H), 3.20 - 2.97 (m, 3H), 2.97 - 2.42 (m, 7H), 2.12 - 1.85 (m, 5.5H), 1.75 - 1.60 (m, 2.5H). ¹⁹F NMR (376 MHz, Chloroform-*d*, *2 sets of rotamers)* δ *-* 109.17, -109.31. MS (ESI+): [M+H]⁺ 656.2/658.2.

### Synthesis of intermediates required for final product 032

### 4-(2,2-dimethylpropylsulfanylj-1-methoxy-2-methyl-benzene (I-058)

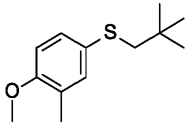

Formula Weight: 224,36

Molecular Formula: C₁₃H₂₀OS

To a solution of 4-methoxy-3-methyl-benzenethiol (350mg, 2.27mmol, 1 equiv.) in dry DMF (2.3mL) at RT, was added sodium hydride (60% in grease, 109mg, 2.73mmol, 1.2 equiv). The mixture was stirred at RT for 30min and 1-bromo-2,2-dimethylpropane (0.34mL, 2.73mmol, 1.2 equiv). The mixture was stirred at 80°C for 1h. After cooling down to RT, water and MTBE were added. The layers were separated and the aqueous phase was extracted with MTBE. The combined organic extracts were washed (brine), dried (Na₂SO₄), filtered and concentrated under reduced pressure. The residue was purified by FC (cHex/EtOAc = 99/1 to 90/10) affording **I-058** in 89% yield. ¹H NMR (400 MHz, Chloroform-*d*) δ 7.17 - 7.11 (m, 2H), 6.66 (d, *J =* 8.2 Hz, 1H), 3.73 (s, 3H), 2.75 (s, 2H), 2.11 (s, 3H), 0.94 (s, 9H). MS (ESI+): [M+H]⁺ 225.1.

### 4-(2,2-dimethylpropylsulfonylj-1-methoxy-2-methyl-benzene (I-059)

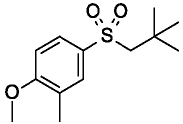

Formula Weight: 256,36

Molecular Formula: C₁₃H₂₀O₃S

To a solution of **I-058** (453mg, 2.02mmol, 1 equiv.) in DCM (10mL) at 0°C, was added m-CPBA (70% wet, 1.10g, 4.44mmol, 2.2 equiv.). The mixture was stirred at RT for 1h. Aq. sat. Na₂S₂O₃, aq. sat. NaHCO₃ and EtOAc were added. The mixture was vigorously stirred for 10 min. The layers were separated and the aqueous phase was extracted with EtOAc. The combined organic extracts were washed (brine), dried (Na₂SO₄), filtered and concentrated under reduced pressure. The residue was purified by FC (cHex/EtOAc = 95/5 to 50/50) affording **I-059** in 86% yield. ¹H NMR (400 MHz, Chloroform-*d*) δ 7.73 (dd, *J* = 8.6, 2.5 Hz, 1H), 7.65 (dd, *J=* 2.4, 0.9 Hz, 1H), 6.90 (d, *J =* 8.6 Hz, 1H), 3.90 (s, 3H), 3.01 (s, 2H), 2.26 (s, 3H), 1.17 (s, 9H). MS (ESI+): [M+H]⁺ 257.1.

### 4-(2,2-dimethylpropylsulfonyl)-2-methyl-phenol (I-060)

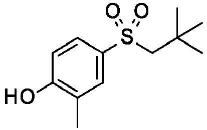

Formula Weight: 242,33

Molecular Formula: C₁₂H₁₈O₃S

To a solution of **I-059** (550mg, 1.74mmol, 1 equiv.) in DCM (5mL) at 0°C, was added BBr₁₃ (1.0M in DCM, 5.2mL, 5.2mmol, 3 equiv.). The mixture was stirred at RT for 16h. Water was added dropwise and the layers were separated. The aqueous phase was extracted with DCM. The combined organic extracts were dried (Na₂SO₄), filtered and concentrated under reduced pressure. The residue was purified by FC (cHex/EtOAc = 98/2 to 75/25) affording **I-060** in 71% yield. ¹H NMR (400 MHz, Chloroform-*d*) δ 1H NMR (400 MHz, Chloroform-*d*) δ 7.67 (dd, *J =* 2.4, 0.9 Hz, 1H), 7.63 (dd, *J =* 8.4, 2.4 Hz, 1H), 6.87 (d, *J =* 8.4 Hz, 1H), 5.44 (s, 1H), 3.01 (s, 2H), 2.30 (s, 3H), 1.17 (s, 9H). MS (ESI+): [M+H]⁺ 243.1.

### 2-bromo-4-(2,2-dimethylpropylsulfonyl)-6-methyl-phenol (I-061)

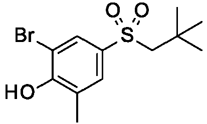

Formula Weight: 321,23

Molecular Formula: C₁₂H₁₇BrO₃S

To a solution of **I-060** (300mg, 1.24mmol, 1 equiv.) in DMF (6.2mL) at RT, was added N-bromosuccinimide (242mg, 1.36mmol, 1.1 equiv.). The mixture was stirred at RT for 1h. Water and EtOAc were added and the layers were separated. The aqueous phase was extracted with EtOAc. The combined organic extracts were washed (water, brine), dried (Na₂SO₄), filtered and concentrated under reduced pressure. The residue was purified by FC (cHex/EtOAc = 97/3 to 70/30) affording **I-061** in 80% yield. ¹H NMR (400 MHz, Chloroform-*d*) δ 1H NMR (400 MHz, Chloroform-*d*) δ 7.90 - 7.87 (m, 1H), 7.64 - 7.59 (m, 1H), 6.04 (s, 1H), 3.00 (s, 2H), 2.36 (s, 3H), 1.19 (s, 9H). MS (ESI+): [M+H]⁺ 321.0/323.0.

### 1-bromo-5-(2,2-dimethylpropylsulfonyl)-2-(methoxymethoxy)-3-methyl-benzene (I-062)

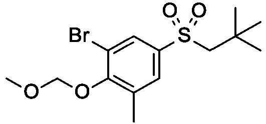

Formula Weight: 365,28

Molecular Formula: C₁₄H₂₁BrO₄S

To a solution of **I-061** (320 mg, 0.996mmol, 1.00 equiv.) and Et₃N (0.28mL, 2.0mmol, 2 equiv.) in DCM (4mL) at RT, was added chloromethyl methyl ether (0.11mL, 1.5mmol, 2 equiv.). The mixture was stirred at RT for 2h. Sat. aq. NH₄Cl and EtOAc were added. The layers were separated and the aqueous phase was extracted with EtOAc. The combined organic layers were washed (water, brine), dried (Na₂SO₄), filtered and concentrated under reduced pressure. The residue was purified by FC (cHex/EtOAc = 97/3 to 70/30) affording **I-062** in 70% yield. ¹H NMR (400 MHz, Chloroform-*d*) δ 7.95 (d, *J=* 2.3 Hz, 1H), 7.68 (d, *J=* 2.3 Hz, 1H), 5.14 (s, 2H), 3.64 (s, 3H), 3.00 (s, 2H), 2.42 (s, 3H), 1.21 (s, 9H). MS (ESI+): [M+H]⁻ 365.0/367.0.

### (2-chloro-4-fluoro-phenyl)-[8-[5-(2,2-dimethylpropylsulfonyl)-2-(methoxymethoxy)-3-methyl-phenyl]-3,8-diazabicyclo[3.2.1]octan-3-yl]methanone (I-063)

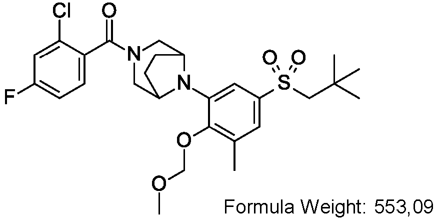

Molecular Formula: C₂₇H₃₄ClFN₂O₅S

A microwave reaction vial was charged with the sulfone **I-062** (255mg, 0.698mmol, 1 equiv.), piperazine **I-030** (225mg, 0.834mmol, 1.2 equiv.), t-BuONa (201mg, 2.09mmol, 3 equiv.) and XPhos-Pd-G3 (59.1mg, 69.8µmol, 0.1equiv.). The vial was flushed with Ar and degassed toluene (5.7mL) was added. The vial was sealed and the reaction was stirred at reflux in a preheated heating-block for 2h. After cooling down to RT, EtOAc was added and the suspension was filtered over Celite (EtOAc rinses). The filtrate was concentrated under reduced pressure. The residue was purified by FC (cHex/EtOAc = 96/4 to 60/40) affording **I-063** in 26% yield. ¹H NMR (400 MHz, Chloroform-*d*, *2 sets of rotamers*) δ 7.32 (s, 1H), 7.23 - 7.01 (m, 4H), 5.17 - 5.10 (m, 2H), 4.56 - 4.48 (m, 1H), 4.30 (s, 1H), 4.08 (s, 1H), 3.62 - 3.38 (m, 4H), 3.22 (d, *J =* 12.9 Hz, 1H), 3.14 (t, *J =* 12.1 Hz, 1H), 2.98 (s, 2H), 2.36 (s, 3H), 2.03 - 1.85 (m, 3.5H), 1.77 - 1.67 (m, 0.5H), 1.18 (s, 9H). ¹⁹F NMR (376 MHz, Chloroform-*d*, *2 sets of rotamers)* δ -109.11, -109.26. MS (ESI+): [M+H]⁺ 553.1/555.1.

### Synthesis of intermediates required for final product 033

### 2-chloro-4-(2,2-dimethylpropylsulfanyl)-1-methoxy-benzene (I-064)

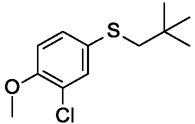

Formula Weight: 244,78

Molecular Formula: C₁₂H₁₇ClOS

To a solution of 3-chloro-4-methoxy-benzenethiol (500mg, 2.86mmol, 1 equiv.) in dry DMF (2.9mL) at RT, was added sodium hydride (60% in grease, 137mg, 3.43mmol, 1.2 equiv). The mixture was stirred at RT for 30min and 1-bromo-2,2-dimethylpropane (0.43mL, 3.44mmol, 1.2 equiv). The mixture was stirred at 80°C for 1h. After cooling down to RT, water and MTBE were added. The layers were separated and the aqueous phase was extracted with MTBE. The combined organic extracts were washed (brine), dried (Na₂SO₄), filtered and concentrated under reduced pressure. The residue was purified by FC (cHex/EtOAc = 99/1 to 90/10) affording **I-064** in 92% yield. ¹H NMR (400 MHz, Chloroform-*d*) δ 7.44 (d, *J= 2.3* Hz, 1H), 7.29 (dd, *J* = 8.5, 2.3 Hz, 1H), 6.86 (d, *J* = 8.6 Hz, 1H), 3.90 (s, 3H), 2.85 (s, 2H), 1.04 (s, 9H). MS (ESI+): [M+H]⁺ 245.1/247.1.

### 2-chloro-4-(2,2-dimethylpropylsulfonyl)-1-methoxy-benzene (I-065)

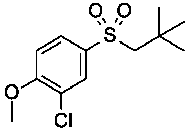

Formula Weight: 276,78

Molecular Formula: C₁₂H₁₇ClO₃S

To a solution of **I-064** (640mg, 2.59mmol, 1 equiv.) in DCM (13mL) at 0°C, was added m-CPBA (70% wet, 1.40g, 5.69mmol, 2.2 equiv.). The mixture was stirred at RT for 1h. Aq. sat. Na₂S₂O₃, aq. sat. NaHCO₃ and EtOAc were added. The mixture was vigorously stirred for 10 min. The layers were separated and the aqueous phase was extracted with EtOAc. The combined organic extracts were washed (brine), dried (Na₂SO₄), filtered and concentrated under reduced pressure. The residue was purified by FC (cHex/EtOAc = 95/5 to 50/50) affording **I-065** in 98% yield. ¹H NMR (400 MHz, Chloroform-*d*) δ 7.94 (d, *J=* 2.3 Hz, 1H), 7.82 (dd, *J* = 8.7, 2.3 Hz, 1H), 7.05 (d, *J* = 8.7 Hz, 1H), 4.01 (s, 3H), 3.04 (s, 2H), 1.21 (s, 9H). MS (ESI+): [M+H]⁺ 277.0/279.0.

### 2-chloro-4-(2,2-dimethylpropylsulfonyl)phenol (I-066)

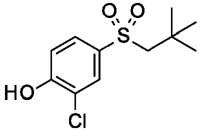

Formula Weight: 262,75

Molecular Formula: C₁₁H₁₅ClO₃S

To a solution of **I-065** (739mg, 2.54mmol, 1 equiv.) in DCM (5mL) at 0°C, was added BBr₃ (1.0M in DCM, 5.1mL, 5.1mmol, 2 equiv.). The mixture was stirred at RT for 16h. Water was added dropwise and the layers were separated. The aqueous phase was extracted with DCM. The combined organic extracts were dried (Na₂SO₄), filtered and concentrated under reduced pressure. The residue was purified by FC (cHex/EtOAc = 95/5 to 50/50) affording **I-066** in 92% yield. ¹H NMR (400 MHz, Chloroform-*d*) δ 7.94 (d, *J=* 2.2 Hz, 1H), 7.76 (dd, *J =* 8.6, 2.2 Hz, 1H), 7.18 (d, *J =* 8.6 Hz, 1H), 6.17 (s, 1H), 3.04 (s, 2H), 1.21 (s, 9H). MS (ESI+): [M+H]⁺ 263.0/265.0.

### 2-bromo-6-chloro-4-(2,2-dimethylpropylsulfonyl)phenol (I-067)

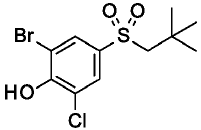

Formula Weight: 341,65

Molecular Formula: C₁₁H₁₄BrClO₃S

To a solution of **I-066** (624mg, 2.30mmol, 1 equiv.) in DMF (11.5mL) at RT, was added N-bromosuccinimide (451mg, 2.53mmol, 1.1 equiv.). The mixture was stirred at RT for 1h. Water and EtOAc were added and the layers were separated. The aqueous phase was extracted with EtOAc. The combined organic extracts were washed (water, brine), dried (Na₂SO₄), filtered and concentrated under reduced pressure. The residue was purified by FC (cHex/EtOAc = 95/5 to 50/50) affording **I-067** in 94% yield. ¹H NMR (400 MHz, Chloroform-*d*) δ 1H NMR (400 MHz, Chloroform-*d*) δ 8.00 (d, *J* = 2.1 Hz, 1H), 7.90 (d, *J=* 2.1 Hz, 1H), 6.40 (s, 1H), 3.04 (s, 2H), 1.23 (s, 9H).

### 1-bromo-3-chloro-5-(2,2-dimethylpropylsulfonyl)-2-(methoxymethoxy)benzene (I-068)

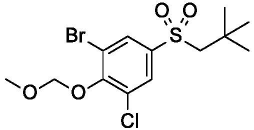

**Formula** Weight: 385,7

Molecular Formula: C₁₃H₁₈BrClO₄S

To a solution of **I-067** (740 mg, 2.17mmol, 1.00 equiv.) and Et₃N (0.60mL, 4.3mmol, 2 equiv.) in DCM (8.7mL) at RT, was added chloromethyl methyl ether (0.25mL, 3.2mmol, 2 equiv.). The mixture was stirred at RT for 2h. Sat. aq. NH₄Cl and EtOAc were added. The layers were separated and the aqueous phase was extracted with EtOAc. The combined organic layers were washed (water, brine), dried (Na₂SO₄), filtered and concentrated under reduced pressure. The residue was purified by FC (cHex/EtOAc = 97/3 to 70/30) affording **I-068** in 62% yield. ¹H NMR (400 MHz, Chloroform-*d*) δ 8.04 (d, *J=* 2.2 Hz, 1H), 7.92 (d, *J=* 2.2 Hz, 1H), 5.29 (s, 2H), 3.73 (s, 3H), 3.04 (s, 2H), 1.25 (s, 9H). MS (ESI+): [M+H]⁺ 384.9/386.9.

### [8-[3-chloro-5-(2,2-dimethylpropylsulfonyl)-2-(methoxymethoxy)phenyl]-3,8-diazabicyclo[3.2.1]octan-3-yl]-(2-chloro-4-fluoro-phenyl)methanone (I-069)

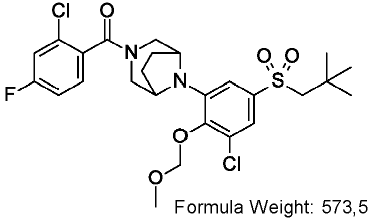

Molecular Formula: C₂₆H₃₁Cl₂FN₂O₅S

A microwave reaction vial was charged with the sulfone **I-068** (260mg, 0.674mmol, 1 equiv.), piperazine **I-030** (235mg, 0.876mmol, 1.3 equiv.), t-BuONa (194mg, 2.02mmol, 3 equiv.) and XPhos-Pd-G3 (57.1mg, 67.4µmol, 0.1equiv.). The vial was flushed with Ar and degassed toluene (5.7mL) was added. The vial was sealed and the reaction was stirred at reflux in a preheated heating-block for 2h. After cooling down to RT, EtOAc was added and the suspension was filtered over Celite (EtOAc rinses). The filtrate was concentrated under reduced pressure. The residue was purified by FC (cHex/EtOAc = 96/4 to 60/40) affording **I-069** in 53% yield. ¹H NMR (400 MHz, Chloroform-d, *2 sets of rotamers*) δ 7.55 - 7.50 (m, 1H), 7.39 (dd, J = 8.5, 5.8 Hz, 0.5H), 7.27 - 7.15 (m, 2.5H), 7.15 - 7.01 (m, 1H), 5.27 - 5.18 (m, 2H), 4.60 - 4.49 (m, 1H), 4.40 (s, 1H), 4.20 & 4.19 (s, 1H), 3.70 - 3.42 (m, 4H), 3.34 - 3.12 (m, 2H), 3.02 (s, 2H), 2.05 - 1.87 (m, 3.5H), 1.72 - 1.65 (m, 0.5H), 1.22 (s, 9H). ¹⁹F NMR (376 MHz, Chloroform-d, *2 sets of rotamers)* δ -108.93, - 109.08. MS (ESI+): [M+H]⁺ 573.1/575.1.

### Synthesis of intermediates required for final product 035

### 1-bromo-2,3-dichloro-5-iodo-benzene (I-070)

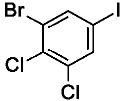

Formula Weight: 351,79

Molecular Formula: C₆H₂BrCl₂I

3-bromo-4,5-dichloroaniline (617mg, 2.56mmol, 1 equiv.) was added to a solution of H₂SO₄ (0.41mL, 7.7mmol, 3 equiv.) in water (9.5mL). The mixture was stirred at 80°C for 10min before being cooled down to 5°C. A solution of NaNO₂ (177mg, 2.56mmol, 1 equiv.) in water (1.1mL) was added dropwise. The mixture was stirred at RT for 1h and a solution of KI (425mg, 2.56mmol, 1 equiv.) in water (1.1mL) was added dropwise. The resulting mixture was stirred at RT for 30min and at 40°C for 30min. EtOAc was added and the layers were separated. The aqueous phase was extracted with EtOAc. The combined organic extracts were dried (Na₂SO₄), filtered and concentrated under reduced pressure. The residue was purified by FC (cHex/EtOAc = 99/1 to 80/20) affording **I-070** in 62% yield. ¹H NMR (400 MHz, Chloroform-*d*) δ 7.88 (d, *J =* 2.0 Hz, 1H), 7.76 (d, *J*= 1.9 Hz, 1H).

### 1-bromo-2,3-dichloro-5-(2,2-dimethylpropylsulfanyl)benzene (I-071)

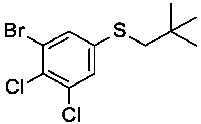

Formula Weight: 328,1

Molecular Formula: C₁₁H₁₃BrCl₂S

A MW vial was charged with **I-070** (500mg, 1.42mmol, 1 equiv.) and XantPhos-Pd-G3 (135mg, 0.142mmol, 0.1 equiv.). The vial was flushed with Ar and degassed toluene (7.1mL) was added. iPr₂NEt (495µL, 3.84mmol, 2 equiv.) and 2,2-dimethylpropane-1-thiol (172µL, 1.42mmol, 1 equiv.) were added. The vial was sealed and the reaction was stirred at 100°C in a preheated heating-block for 1h. After cooling down to RT, the reaction mixture was concentrated under reduced pressure. The residue was purified by FC (cHex/EtOAc = 100/0 to 80/20) affording **I-071** in 66% yield. ¹H NMR (400 MHz, Chloroform-*d*) δ 7.47 (d, *J =* 2.2 Hz, 1H), 7.35 (d, *J =* 2.2 Hz, 1H), 2.87 (s, 2H), 1.06 (s, 9H).

### 1-bromo-2,3-dichloro-5-(2,2-dimethylpropylsulfonyl)benzene (I-072)

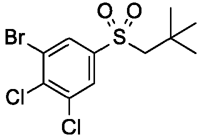

Formula Weight: 360,09

Molecular Formula: C₁₁H₁₃BrCl₂O₂S

Using **GP-2, I-072** was obtained as a white solid in 72% yield using **I-071** (308mg, 939µmol, 1 equiv.) and m-CPBA (70% wet, 509mg, 2.07mmol, 2.2 equiv.) in DCM (4.7mL) at RT for 1h. Purified by FC (cHex/EtOAc = 100/0 to 70/30). ¹H NMR (400 MHz, Chloroform-d) δ 8.08 (d, *J =* 2.0 Hz, 1H), 7.96 (d, *J* = 2.0 Hz, 1H), 3.04 (s, 2H), 1.23 (s, 9H). MS (ESI⁺): [M+H]⁺ 358.9/360.9.

### Synthesis of intermediates required for final product 036

### 1-bromo-3-chloro-5-iodo-2-methyl-benzene (I-073)

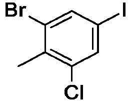

Formula Weight: 331,38

Molecular Formula: C₇H₅BrCII

3-bromo-5-chloro-4-methylaniline (363mg, 1.65mmol, 1 equiv.) was added to a solution of H₂SO₄ (0.26mL, 4.9mmol, 3 equiv.) in water (6.1mL). The mixture was stirred at 80°C for 10min before being cooled down to 5°C. A solution of NaNO₂ (114mg, 1.65mmol, 1 equiv.) in water (0.7mL) was added dropwise. The mixture was stirred at RT for 1h and a solution of KI (273mg, 1.65mmol, 1 equiv.) in water (0.7mL) was added dropwise. The resulting mixture was stirred at RT for 30min and at 40°C for 30min. EtOAc was added and the layers were separated. The aqueous phase was extracted with EtOAc. The combined organic extracts were dried (Na₂SO₄), filtered and concentrated under reduced pressure. The residue was purified by FC (cHex/EtOAc = 100/0 to 70/30) affording **I-073** in 32% yield. ¹H NMR (400 MHz, Chloroform-d) δ 7.80 (d, *J =* 1.7 Hz, 1H), 7.66 (d, *J* = 1.7 Hz, 1H), 2.47 (s, 3H).

### 1-bromo-3-chloro-5-(2,2-dimethylpropylsulfanyl)-2-methyl-benzene (1-074)

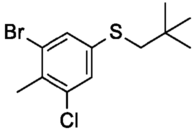

Formula Weight: 307,68

Molecular Formula: C₁₂H₁₆BrClS

A MW vial was charged with **I-073** (174mg, 0.525mmol, 1 equiv.) and XantPhos-Pd-G3 (50mg, 0.052mmol, 0.1 equiv.). The vial was flushed with Ar and degassed toluene (2.6mL) was added. iPr₂NEt (183µL, 1.05mmol, 2 equiv.) and 2,2-dimethylpropane-1-thiol (63.6µL, 0.525mmol, 1 equiv.) were added. The vial was sealed and the reaction was stirred at 100°C in a preheated heating-block for 1.5h. After cooling down to RT, the reaction mixture was concentrated under reduced pressure. The residue was purified by FC (cHex/EtOAc = 100/0 to 80/20) affording **I-074** in 58% yield. ¹H NMR (400 MHz, Chloroform-*d*) δ 7.43 (d, *J =* 1.9 Hz, 1H), 7.28 (d, *J =* 1.9 Hz, 1H), 2.86 (s, 2H), 2.46 (s, 3H), 1.05 (s, 9H).

### 1-bromo-3-chloro-5-(2,2-dimethylpropylsulfonyl)-2-methyl-benzene (1-075)

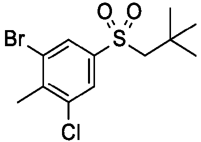

**Formula** Weight: 339,68

Molecular Formula: C₁₂H₁₆BrClO₂S

Using **GP-2, I-075** was obtained as a white solid in 67% yield using **I-074** (93.0mg, 302µmol, 1 equiv.) and m-CPBA (70% wet, 164mg, 0.665mmol, 2.2 equiv.) in DCM (1.5mL) at RT for 16h. Purified by FC (cHex/EtOAc = 100/0 to 70/30). ¹H NMR (400 MHz, Chloroform-d) δ 8.00 (d, *J =* 1.8 Hz, 1H), 7.86 (d, *J =* 1.8 Hz, 1H), 3.03 (s, 2H), 2.60 (s, 3H), 1.22 (s, 10H). MS (ESI⁺): [M+H]⁺ 339.0/341.0.

### Synthesis of final compounds

### Synthetic methods for final compounds

The synthetic protocols for the final compounds are presented on the following **Table 2.**

**Table 2**

| **Cpd** | **Synthetic protocols** | **Purity** |
|---|---|---|
| **001** | According to **GP-3, 001** was obtained in 87% yield using aryl bromide **I-012** (215mg, 633µmol, 1 equiv.), piperazine **I-028** (211mg, 736µmol, 1.2 equiv.), Cs₂CO₃ (311mg, 953µmol, 1.5 equiv.), Pd(OAc)₂ (7.1mg, 32µmol, 0.05 equiv.) and rac-BINAP (24mg, 38µmol, 0.06 equiv.) in Toluene (3.2mL) at reflux for 6h. Purification by FC (cHex/EtOAc = 95/5 to 0/100). | >95% |
| **002** | According to **GP-3, 002** was obtained in 80% yield using aryl bromide **I-013** (131mg, 410µmol, 1 equiv.), piperazine **I-028** (136mg, 493µmol, 1.2 equiv.), Cs₂CO₃ (201mg, 616µmol, 1.5 equiv.), Pd(OAc)₂ (4.6mg, 21 µmol, 0.05 equiv.) and rac-BINAP (15mg, 25µmol, 0.06 equiv.) in Toluene (2.1mL) at reflux for 2.5h. Purification by FC (cHex/EtOAc = 95/5 to 0/100). | >95% |
| **003** | According to **GP-3, 003** was obtained in 20% yield using aryl bromide **I-014** (76.0mg, 228µmol, 1 equiv.), piperazine **I-028** (78.0mg, 274µmol, 1.2 equiv.), Cs₂CO₃ (111mg, 342µmol, 1.5 equiv.), Pd(OAc)₂ (4.6mg, 21 µmol, 0.05 equiv.) and *rac*-BINAP (8.5mg, 14µmol, 0.06 equiv.) in Toluene (1.1mL) at reflux for 18h. Purification by FC (cHex/EtOAc = 95/5 to 50/50). | >95% |
| **004** | According to **GP-3, 004** was obtained in 47% yield using aryl bromide **I-013** (100mg, 313µmol, 1 equiv.), piperazine **I-030** (101mg, 376µmol, 1.2 equiv.), Cs₂CO₃ (153mg, 470µmol, 1.5 equiv.), Pd(OAc)₂ (4.6mg, 21 µmol, 0.05 equiv.) and rac-BINAP (12mg, 19µmol, 0.06 equiv.) in Toluene (1.1mL) at reflux for 5h. Purification by FC (cHex/EtOAc = 95/5 to 0/100). | >95% |
| **005** | According to **GP-3, 005** was obtained in 54% yield using aryl bromide **I-012** (100mg, 311µmol, 1 equiv.), piperazine **I-030** (100mg, 374µmol, 1.2 equiv.), Cs₂CO₃ (152mg, 467µmol, 1.5 equiv.), Pd(OAc)₂ (3.5mg, 16µmol, 0.05 equiv.) and *rac*-BINAP (12mg, 19µmol, 0.06 equiv.) in Toluene (1.5mL) at reflux for 12h. Purification by FC (cHex/EtOAc = 95/5 to 0/100). | >95% |
| **006** | According to **GP-3, 006** was obtained in 11% yield using aryl bromide **I-013** (300mg, 940µmol, 1 equiv.), piperazine **I-031** (290mg, 1.13mmol, 1.2 equiv.), Cs₂CO₃ (459mg, 1.41mmol, 1.5 equiv.), Pd(OAc)₂ (11mg, 47µmol, 0.05 equiv.) and *rac*-BINAP (35mg, 56µmol, 0.06 equiv.) in Toluene (4.7mL) at reflux for 48h. Purification by FC (cHex/EtOAc = 95/5 to 0/100). | >95% |
| **007** | According to **GP-3, 007** was obtained in 10% yield using aryl bromide **I-012** (300mg, 934µmol, 1 equiv.), piperazine **I-031** (288mg, 1.12mmol, 1.2 equiv.), Cs₂CO₃ (456mg, 1.40mmol, 1.5 equiv.), Pd(OAc)₂ (11mg, 47µmol, 0.05 equiv.) and *rac*-BINAP (35mg, 56µmol, 0.06 equiv.) in Toluene (4.7mL) at reflux for 24h. Purification by FC (cHex/EtOAc = 95/5 to 0/100). | >95% |
| **008** | According to **GP-3, 008** was obtained in 36% yield using aryl bromide **I-022** (45.0mg, 154µmol, 1 equiv.), piperazine **I-029** (45.0mg, 184µmol, 1.2 equiv.), Cs₂CO₃ (75.0mg, 230µmol, 1.5 equiv.), Pd(OAc)₂ (1.7mg, 7.7µmol, 0.05 equiv.) and *rac-*BINAP (5.7mg, 9.2µmol, 0.06 equiv.) in Toluene (0.8mL) at reflux for 16h. Purification by FC (cHex/EtOAc = 95/5 to 0/100). | >95% |
| **009** | According to **GP-3, 009** was obtained in 27% yield using aryl bromide **I-015** (110mg, 360µmol, 1 equiv.), piperazine **I-029** (105mg, 433µmol, 1.2 equiv.), Cs₂CO₃ (176mg, 541µmol, 1.5 equiv.), Pd(OAc)₂ (4.1mg, 18µmol, 0.05 equiv.) and *rac*-BINAP (14mg, 22µmol, 0.06 equiv.) in Toluene (1.8mL) at reflux for 16h. Purification by FC (cHex/EtOAc = 95/5 to 0/100) and PTLC (CHex/(EtOAc/EtOH = 3/1) = 1/1). | >95% |
| **010** | According to **GP-4, 010** was obtained in 79% yield using aryl bromide **I-016** (110mg, 360µmol, 1 equiv.), piperazine **I-030** (145mg, 541µmol, 1.5 equiv.), t-BuONa (104mg, 1.08mmol, 3 equiv.), XPhos-Pd-G3 (31mg, 36µmol, 0.1 equiv.) in Toluene (1.8mL) at reflux for 16h. Purification by FC (cHex/EtOAc = 95/5 to 0/100). | >95% |
| **011** | According to **GP-4, 011** was obtained in 34% yield using aryl bromide **I-017** (50.0mg, 163µmol, 1 equiv.), piperazine **I-029** (60.0mg, 244µmol, 1.5 equiv.), t-BuONa (47.0mg, 488µmol, 3 equiv.), XPhos-Pd-G3 (14mg, 16µmol, 0.1 equiv.) in Toluene (0.8mL) at reflux for 2h. Purification by FC (cHex/EtOAc = 95/5 to 0/100). | >95% |
| **012** | According to **GP-4, 012** was obtained in 29% yield using aryl bromide **I-018** (60.0mg, 188µmol, 1 equiv.), piperazine **I-029** (68.0mg, 282µmol, 1.5 equiv.), t-BuONa (54.0mg, 564µmol, 3 equiv.), XPhos-Pd-G3 (16mg, 19µmol, 0.1 equiv.) in Toluene (0.9mL) at reflux for 2h. Purification by FC (cHex/EtOAc = 95/5 to 0/100). | >95% |
| **013** | According to **GP-4, 013** was obtained in 29% yield using aryl bromide **I-019** (60.0mg, 180µmol, 1 equiv.), piperazine **I-029** (66mg, 270µmol, 1.5 equiv.), t-BuONa (52.0mg, 540µmol, 3 equiv.), XPhos-Pd-G3 (15mg, 18µmol, 0.1 equiv.) in Toluene (0.9mL) at reflux for 2h. Purification by FC (cHex/EtOAc = 95/5 to 0/100) and PTLC (DCM/MeOH = 95/05). | >95% |
| **014** | According to **GP-4, 014** was obtained in 90% yield using aryl bromide **I-020** (100mg, 293µmol, 1 equiv.), piperazine **I-030** (118mg, 440µmol, 1.5 equiv.), t-BuONa (85.0mg, 879µmol, 3 equiv.), XPhos-Pd-G3 (25mg, 29µmol, 0.1 equiv.) in Toluene (1.5mL) at reflux for 16h. Purification by FC (cHex/EtOAc = 95/5 to 0/100). | >95% |
| **015** | According to **GP-4, 015** was obtained in 32% yield using aryl bromide **I-021** (40mg, 125µmol, 1 equiv.), piperazine **I-030** (50.0mg, 187µmol, 1.5 equiv.), t-BuONa (36.0mg, 374µmol, 3 equiv.), XPhos-Pd-G3 (11mg, 13µmol, 0.1 equiv.) in Toluene (0.6mL) at reflux for 16h. Purification by FC (cHex/EtOAc = 95/5 to 0/100) and PTLC (DCM/MeOH = 90/10). | >95% |
| **016** | To a solution of 2-chloro-4-fluorobenzoic acid (38.5mg, 220µmol, 1.5 equiv.) in DMF (1.5mL) at RT, was added TBTU (70.7mg, 220µmol, 1.5 equiv.). The mixture was stirred 30min at RT, and a solution of piperazine **I-027** (50mg, 147µmol, 1.00 equiv ) and Et₃N (41µL, 294µmol, 2 equiv) in THF (1.5mL) was added dropwise. The mixture was stirred at RT for 16h. The reaction mixture was partitioned between EtOAc and aq. sat. NH₄Cl. The layers were separated and the aqueous phase was extracted with EtOAc. The combined organic extracts were washed (aq. sat. NH₄Cl, aq. sat. NaHCO₃, brine), dried (NaSO4), filtered and concentrated under reduced pressure. The residue was purified by PTLC (cHex/EtOAc = 50/50) and another PTLC (DCM/EtOAc = 90/10) affording **016** in 49% yield. | >95% |
| **017** | To a solution of 4-fluoro-2-(trifluoromethyl)benzoic acid (45.8mg, 220µmol, 1.5 equiv.) in DMF (1.5mL) at RT, was added TBTU (70.7mg, 220µmol, 1.5 equiv.). The mixture was stirred 30min at RT, and a solution of piperazine **I-027** (50mg, 147µmol, 1.00 equiv ) and triethylamine (41µL, 294µmol, 2 eq uiv) in THF (1.5mL) was added dropwise. The mixture was stirred at RT for 16h. The reaction mixture was partitioned between EtOAc and aq. sat. NH₄Cl. The layers were separated and the aqueous phase was extracted with EtOAc. The combined organic extracts were washed (aq. sat. NH₄Cl, aq. sat. NaHCO₃, brine), dried (NaSO4), filtered and concentrated under reduced pressure. The residue was purified by PTLC (cHex/EtOAc = 50/50) and another PTLC (DCM/EtOAc = 90/10) affording 017 in 58% yield. | >95% |
| **018** | To a solution of thioether **I-024** (105m, 223µmol, 1 equiv.) in THF/water (2/1, 2.2mL) at RT, was added oxone (137mg, 440µmol, 2 equiv.). After 2h, more oxone (68mg, 110µmol, 1 equiv.) was added and the mixture was stirred a further 2h at RT. | >95% |
| | Water was added and the product was extracted twice with EtOAc. The combined organics extracts were washed with brine, dried (MgSO₄), filtered and concentrated under reduced pressure. The residue was purified by FC (cHex/EtOAc = 95/5 to 0/100) affording **018** in 9% yield. | |
| **019** | According to **GP-4, 019** was obtained in 22% yield using aryl bromide **I-040** (73.0mg, 210µmol, 1 equiv.), piperazine **I-030** (67.8mg, 252µmol, 1.2 equiv.), t-BuONa (61mg, 0.63mmol, 3 equiv.), XPhos-Pd-G3 (8.9mg, 11µmol, 0.05 equiv.) in Toluene (1.1mL) at reflux for 2h. Purification by FC (cHex/EtOAc = 95/5 to 0/100). | >95% |
| **020** | According to **GP-4, 020** was obtained in 78% yield using aryl bromide **I-041** (40.0mg, 130µmol, 1 equiv.), piperazine **I-030** (45.5mg, 169µmol, 1.3 equiv.), t-BuONa (37mg, 0.39mmol, 3 equiv.), XPhos-Pd-G3 (11mg, 13µmol, 0.1 equiv.) in Toluene (0.7mL) at reflux for 5h. Purification by FC (cHex/EtOAc = 95/5 to 0/100). | >95% |
| **021** | According to **GP-4, 021** was obtained in 62% yield using aryl bromide **I-042** (185mg, 552µmol, 1 equiv.), piperazine **I-030** (193mg, 717µmol, 1.3 equiv.), t-BuONa (159mg, 1.65mmol, 3 equiv.), XPhos-Pd-G3 (47mg, 55µmol, 0.1 equiv.) in Toluene (2.8mL) at reflux for 5h. Purification by FC (cHex/EtOAc = 95/5 to 0/100). | >95% |
| **022** | According to **GP-4, 022** was obtained in 78% yield using aryl bromide **I-043** (40.0mg, 130µmol, 1 equiv.), piperazine **I-030** (45.5mg, 169µmol, 1.3 equiv.), t-BuONa (37.5mg, 0.391mmol, 3 equiv.), XPhos-Pd-G3 (11mg, 13µmol, 0.1 equiv.) in Toluene (0.7mL) at reflux for 5h. Purification by FC (cHex/EtOAc = 95/5 to 0/100). | >95% |
| **023** | According to **GP-4, 023** was obtained in 68% yield using aryl bromide **I-044** (98.0mg, 256µmol, 1 equiv.), piperazine **I-030** (82.4mg, 307µmol, 1.2 equiv.), t-BuONa (73.7mg, 0.767mmol, 3 equiv.), XPhos-Pd-G3 (11mg, 13µmol, 0.05 equiv.) in Toluene (2.6mL) at reflux for 5h. Purification by FC (cHex/EtOAc = 95/5 to 30/70). | >95% |
| **024** | According to **GP-4, 024** was obtained in 21% yield using aryl bromide **I-045** (75.0mg, 189µmol, 1 equiv.), piperazine **I-030** (60.9mg, 227µmol, 1.2 equiv.), t-BuONa (54.4mg, 0.566mmol, 3 equiv.), XPhos-Pd-G3 (8.0mg, 9.4µmol, 0.05 equiv.) in Toluene (1.9mL) at reflux for 16h. Purification by FC (cHex/EtOAc = 95/5 to 30/70). | >95% |
| **025** | According to **GP-4, 025** was obtained in 60% yield using aryl bromide **I-046** (100mg, 252µmol, 1 equiv.), piperazine **I-030** (81.1mg, 302µmol, 1.2 equiv.), t-BuONa (72.6mg, 0.755mmol, 3 equiv.), XPhos-Pd-G3 (11mg, 13µmol, 0.05 equiv.) in Toluene (2.5mL) at reflux for 2h. Purification by FC (cHex/EtOAc = 95/5 to 30/70). | >95% |
| **026** | According to **GP-3, 026** was obtained in 82% yield using aryl bromide **I-047** (25mg, 61µmol, 1 equiv.), piperazine **I-030** (20mg, 73µmol, 1.2 equiv.), Cs₂CO₃ (59.4mg, 182µmol, 3 equiv.), Pd(OAc)₂ (1.4mg, 6.1 µmol, 0.1 equiv.) and rac-BINAP (4.6mg, 7.3µmol, 0.12 equiv.) in Toluene (0.3mL) at reflux for 16h. Purification by PTLC (cHex/EtOAc = 70/30). | >95% |
| **027** | According to **GP-4, 027** was obtained in 40% yield using amine hydrochloride **I-050** (80.0mg, 135µmol, 1 equiv.), bromobenzene (21.3µL, 202µmol, 1.5 equiv.), t-BuONa (38.8mg, 0.403mmol, 3 equiv.), XPhos-Pd-G3 (11mg, 13µmol, 0.05 equiv.) in Toluene (0.7mL) at reflux for 16h. Purification by PTLC (cHex/EtOAc = 50/50). | >95% |
| **028** | To a solution of amine hydrochloride **I-050** (80.0mg, 135µmol, 1 equiv.) in DCM (0.7mL) at RT, were added phenylacetaldehyde (37mg, 0.31mmol, 2 equiv.) and NaBH(OAc)₃ (65mg, 0.31mmol, 2 equiv.). The mixture was stirred at RT for 2h. Sat. aq. NaHCO₃ and the layers were separated. The aqueous phase was extracted with DCM and the combined organic extracts were dried (Na₂SO₄), filtered and concentrated under reduced pressure. The residue was purified by PTLC (cHex/EtOAc = 50/50) affording **028** in 55% yield. | >95% |
| **029** | To a solution of amine hydrochloride **I-050** (80.0mg, 135µmol, 1 equiv.) in DCM (0.7mL) at RT, were added 2-(4-Fluorophenyl)acetaldehyde (42mg, 0.31mmol, 2 equiv.) and NaBH(OAc)₃ (65mg, 0.31mmol, 2 equiv.). The mixture was stirred at RT for 2h. Sat. aq. NaHCO₃ and the layers were separated. The aqueous phase was extracted with DCM and the combined organic extracts were dried (Na₂SO₄), filtered and concentrated under reduced pressure. The residue was purified by PTLC (cHex/EtOAc = 50/50) affording **029** in 41% yield. | >95% |
| **030** | To a solution of **I-056** (42mg, 67µmol, 1 equiv.) in DCM (0.7mL) at RT, was added TFA (0.10mL, 1.3mmol, 20 equiv.). | >95% |
| | The mixture was stirred at RT for 16h. The reaction mixture was concentrated under reduced pressure. The residue was purified by PTLC (CHCl₃/*i*PrOH = 97/3) affording **030** in 36% yield. | |
| **031** | To a solution of **I-057** (44mg, 67µmol, 1 equiv.) in DCM (0.7mL) at RT, was added TFA (0.10mL, 1.3mmol, 20 equiv.). | >95% |
| | The mixture was stirred at RT for 16h. The reaction mixture was concentrated under reduced pressure. The residue was purified by PTLC (DCM/MeOH = 95/5) affording **031** in 49% yield. | |
| **032** | To a solution of **I-063** (100mg, 181µmol, 1 equiv.) in DCM (0.9mL) at RT, was added TFA (0.28mL, 3.6mmol, 20 equiv.). | >95% |
| | The mixture was stirred at RT for 2h. The reaction mixture was concentrated under reduced pressure. The residue was purified by FC (cHex/EtOAc = 95/5 to 50/50) affording **032** in 11% yield. | |
| **033** | To a solution of **1-069** (200mg, 349µmol, 1 equiv.) in DCM (1.7mL) at RT, was added TFA (0.54mL, 7.0mmol, 20 equiv.). | >95% |
| | The mixture was stirred at RT for 2h. The reaction mixture was concentrated under reduced pressure. The residue was purified by FC (cHex/EtOAc = 95/5 to 50/50) affording **033** in 84% yield. | |
| **034** | A suspension of **033** (45mg, 85mol, 1 equiv.) 2-bromoethanol (15µL, 0.21mmol, 2.5 equiv.) and K₂CO₃ (35mg, 0.26mmol, 3 equiv.) in dry DMF (0.8mL) was stirred at 50°C for 16h. Water was added and the product was extracted twice with EtOAc. The combined organic layers were washed with water and brine, dried over Na₂SO₄, filtered and concentrated. The residue was purified by PTLC (cHex/EtOAc = 30/70) to afford **034** in 72% yield. | >95% |
| **035** | According to **GP-3, 035** was obtained in 58% yield using aryl bromide **1-072** (120mg, 333µmol, 1 equiv.), piperazine **I-030** (107mg, 400µmol, 1.2 equiv.), Cs₂CO₃ (326mg, 1.00mmol, 3 equiv.), Pd(OAc)₂ (7.5mg, 33µmol, 0.10 equiv.) and rac-BINAP (25mg, 40µmol, 0.12 equiv.) in Toluene (1.7mL) at reflux for 2h. Purification by FC (cHex/EtOAc = 92/8 to 20/80) and PTLC (cHex/EtOAc = 50/50). | >95% |
| **036** | According to **GP-3, 036** was obtained in 27% yield using aryl bromide **I-075** (69.0mg, 203µmol, 1 equiv.), piperazine **1-030** (65.5mg, 244µmol, 1.2 equiv.), Cs₂CO₃ (199mg, 0.609mmol, 3 equiv.), Pd(OAc)₂ (4.6mg, 20µmol, 0.10 equiv.) and rac-BINAP (15mg, 24µmol, 0.12 equiv.) in Toluene (1.0mL) at reflux for 2h. Purification by FC (cHex/EtOAc = 94/6 to 40/60) and PTLC (cHex/EtOAc = 70/30). | >95% |

### Analytical data for the final compounds

The analytical data for the final compounds are presented on the following **Table 3.**

**Table 3**

| **Cpd** | **Description** |
|---|---|
| **001** | White solid. ¹H NMR (400 MHz, Chloroform-d) δ 7.58 (dd, *J =* 8.5, 2.2 Hz, 1H), 7.46 (dd, *J =* 8.8, 2.5 Hz, 1H), 7.43-7.31 (m, 3H), 7.00 (d, *J =* 8.6 Hz, 1H), 4.05 (ddd, *J* = 13.3, 6.4, 3.4 Hz, 1H), 4.00-3.90 (m, 4H), 3.38 (t, *J* = 5.1 Hz, 2H), 3.22 (ddd, *J =* 10.1, 6.4, 3.5 Hz, 1H), 3.14 (ddd, *J =* 11.5, 7.1, 3.5 Hz, 1H), 3.08-2.93 (m, 2H), 2.77 (tt, *J* = 7.1, 4.8 Hz, 1H), 1.95-1.80 (m, 2H), 1.75-1.60 (m, 2H), 1.01 (t, *J* = 7.5 Hz, 6H). ¹⁹F NMR (376 MHz, Chloroform-*d*) δ -60.3, -109.2. MS (ESI⁺): [M+H]⁺ 517.2. |
| **002** | Brown solid. ¹H NMR (400 MHz, Chloroform-d) δ 7.61 (dd, *J =* 8.5, 2.2 Hz, 1H), 7.46 (dd, *J =* 8.8, 2.5 Hz, 1H), 7.43-7.31 (m, 3H), 7.00 (d, *J =* 8.6 Hz, 1H), 4.04 (ddd, *J =* 13.2, 6.4, 3.6 Hz, 1H), 4.00-3.92 (m, 4H), 3.57-3.42 (m, 1H), 3.38 (t, *J =* 5.1 Hz, 2H), 3.22 (ddd, *J =* 10.2, 6.5, 3.7 Hz, 1H), 3.14 (ddd, *J =* 11.5, 7.1, 3.6 Hz, 1H), 3.08-2.93 (m, 2H), 2.12-2.00 (m, 2H), 1.95-1.84 (m, 2H), 1.84-1.70 (m, 2H), 1.68-1.60 (m, 2H). ¹⁹F NMR (376 MHz, Chloroform-d) δ -60.2, -109.2. MS (ESI⁺): [M+H]⁺ 515.1. |
| **003** | White solid. ¹H NMR (400 MHz, Chloroform-d) δ 7.55 (dd, *J =* 8.5, 2.2 Hz, 1H), 7.44 (dd, *J* = 8.8, 2.5 Hz, 1H), 7.38 (m, 1H), 7.36-7.31 (m, 1H), 7.30 (d, *J =* 2.3 Hz, 1H), 6.98 (d, *J = 8.6* Hz, 1H), 4.06-3.90 (m, 5H), 3.36 (t, *J =* 5.1 Hz, 2H), 3.24-3.09 (m, 2H), 3.05-2.94 (m, 2H), 2.85 (tt, *J* = 12.2, 3.5 Hz, 1H), 2.05 (d, *J =* 12.4 Hz, 2H), 1.85 (d, *J =* 12.3 Hz, 2H), 1.43-1.34 (m, 2H), 1.29-1.11 (m, 4H). ¹⁹F NMR (376 MHz, Chloroform-d) δ -60.3, -109.2. MS (ESI⁺): [M+H]⁺ 529.1. |
| **004** | Yellow solid. ¹H NMR (400 MHz, Chloroform-d, *2 sets of rotamers)* δ 7.47 & 7.45 (t, *J =* 1.8 Hz, 1H), 7.37 & 7.21 (dd, *J =* 8.4, 6.0 Hz, 1H), 7.26-7.23 (m, 1H), 7.19-7.12 (m, 1H), 7.08 & 7.03 (td, *J* = 8.2, 2.5 Hz, 1H), 6.95 (d, *J* = 8.5 Hz, 1H), 4.55-4.45 (m, 1H), 4.30 (t, *J =* 8.0 Hz, 1H), 4.10-4.04 (m, 1H), 3.94 & 3.93 (s, 3H), 3.67 & 3.50 (d, *J =* 12.6, Hz, 1H), 3.47-3.38 (m, 1H), 3.30 & 3.27 (d, *J =* 4.9 Hz, 1H), 3.13 (t, *J =* 10.9 Hz, 1H), 2.10-1.79 (m, 8H), 1.79-1.67 (m, 2H), 1.67-1.57 (m, 2H). ¹⁹F NMR (376 MHz, Chloroform-d, *2 sets of rotamers)* δ -109.26, -109.38. MS (ESI⁺): [M+H]⁺ 507.1/509.1. |
| **005** | Yellow solid. ¹H NMR (400 MHz, Chloroform-d, *multiple sets of rotamers)* δ 7.47 (m, 1H), 7.45-7.15 (m, 3H), 7.08 (dtd, *J* = 18.7, 8.2, 2.4 Hz, 1H), 6.98 (d, *J =* 8.5 Hz, 1H), 4.55 & 4.51 (d, *J = 4.0* Hz, 1H), 4.38-4.28 (m, 1H), 4.14-4.04 (m, 1H), 3.96 & 3.95 (s, 3H), 3.70 & 3.53 (d, *J =* 12.0 Hz, 1H), 3.32 & 3.29 (d, *J =* 5.8 Hz, 1H), 3.19-3.12 (m, 1H), 2.80-2.72 (m, 1H), 2.16-1.60 (m, 8H), 1.02-0.98 (m, 6H). ¹⁹F NMR (376 MHz, Chloroform-d, *2 sets of rotamers)* δ -109.26, -109.37. MS (ESI⁺): [M+H]⁺ 509.1/511.1. |
| **006** | Yellow solid. ¹H NMR (400 MHz, Chloroform-d, *multiple sets of rotamers)* δ 7.64-7.52 (m, 1H), 7.47-7.28 (m, 2H), 7.23-7.14 (m, 1H), 7.12-7.02 (m, 1H), 7.01-6.90 (m, 1H), 4.23-3.67 (m, 6H), 3.58-2.75 (m, 5H), 2.12-1.59 (m, 8H), 1.06-0.76 (m, 3H). ¹⁹F NMR (376 MHz, Chloroform-d, *multiple sets of rotamers)* δ -109.27, -109.33, 109.34. MS (ESI⁺): [M+H]⁺ 495.0/497.0. |
| **007** | Yellow solid. ¹H NMR (400 MHz, Chloroform-d, *multiple sets of rotamers)* δ 7.63-7.54 (m, 1H), 7.45-7.29 (m, 2H), 7.19 (ddd, *J* = 11.1, 8.5, 2.4 Hz, 1H), 7.07 (tdt, *J* = 8.0, 4.6, 2.5 Hz, 1H), 6.97 (dd, *J =* 8.5, 4.8 Hz, 1H), 4.24-3.68 (m, 6H), 3.59-2.72 (m, 5H), 1.94-1.79 (m, 2H), 1.73-1.58 (m, 2H), 1.05-0.93 (m, 8H), 0.81 (t, *J =* 6.5 Hz, 1H). ¹⁹F NMR (376 MHz, Chloroform-d) δ - 109.27, -109.33, -109.35. MS (ESI⁺): [M+H]⁺ 497.2/499.1. |
| **008** | White solid. ¹H NMR (400 MHz, Chloroform-d) δ 7.57 (dd, *J =* 8.5, 2.2 Hz, 1H), 7.32 (dd, *J =* 5.4, 3.9 Hz, 2H), 7.18 (dd, *J =* 8.5, 2.5 Hz, 1H), 7.07 (td, *J =* 8.3, 2.5 Hz, 1H), 6.99 (d, *J =* 8.5 Hz, 1H), 3.99 (t, *J =* 5.1 Hz, 2H), 3.96 (s, 3H), 3.56-3.44 (m, 1H), 3.44-3.34 (m, 1H), 3.16 (d, 3H), 3.12-3.07 (m, 1H), 3.04-2.90 (m, 1H), 1.28 (d, *J* = 6.8 Hz, 6H). ¹⁹F NMR (376 MHz, Chloroform-d) δ -109.20. MS (ESI⁺): [M+H]⁺ 455.1/457.1. |
| **009** | Yellow solid. ¹H NMR (400 MHz, Chloroform-d) δ 7.62 (dd, *J* = 8.4, 2.1 Hz, 1H), 7.40 (d, *J =* 2.2 Hz, 1H), 7.32 (dd, *J =* 8.5, 5.8 Hz, 1H), 7.18 (dd, *J =* 8.5, 2.4 Hz, 1H), 7.07 (td, *J =* 8.3, 2.4 Hz, 1H), 6.99 (d, *J =* 8.5 Hz, 1H), 4.05-3.97 (m, 2H), 3.96 (s, 3H), 3.52-3.33 (m, 2H), 3.23-3.06 (m, 3H), 2.99 (d, *J* = 7.2 Hz, 3H), 1.01 (tt, *J =* 8.3, 5.0 Hz, 1H), 0.62-0.48 (m, 2H), 0.23-0.05 (m, 2H). ¹⁹F NMR (376 MHz, Chloroform-d) δ -109.18. MS (ESI⁺): [M+H]⁺ 467.1/469.1. |
| **010** | White solid. ¹H NMR (400 MHz, Chloroform-d, *2 sets of rotamers)* δ 7.46-7.41 (m, 1H), 7.36 (dd, *J =* 8.5, 5.8 Hz, 1H), 7.21 (p, *J =* 3.0 Hz, 1H), 7.19-7.12 (m, 1H), 7.06 (dtd, *J =* 18.8, 8.2, 2.4 Hz, 1H), 6.95 (d, *J =* 8.5 Hz, 1H), 4.55-4.45 (m, 1H), 4.34-4.24 (m, 1H), 4.06 (s, 1H), 3.93 (s, 3H), 3.75 (p, *J =* 8.3 Hz, 1H), 3.70-3.46 (m, 1H), 3.28 (dd, *J =* 13.1, 5.2 Hz, 1H), 3.13 (t, *J =* 11.0 Hz, 1H), 2.60-2.45 (m, 2H), 2.22-2.09 (m, 2H), 2.05-1.87 (m, 5H), 1.66-1.59 (m, 1H). ¹⁹F NMR (376 MHz, Chloroform-d, *2 sets of rotamers) δ -* 109.26, -109.37. MS (ESI⁺): [M+H]⁺ 493.1/495.1. |
| **011** | White solid. ¹H NMR (400 MHz, Chloroform-d) δ 7.59 (dd, *J =* 8.6, 2.2 Hz, 1H), 7.36-7.30 (m, 2H), 7.18 (dd, *J =* 8.5, 2.4 Hz, 1H), 7.07 (td, *J = 8.3,* 2.4 Hz, 1H), 7.00 (d, *J =* 8.5 Hz, 1H), 4.93 (t, *J =* 6.7 Hz, 2H), 4.78 (t, *J =* 7.7 Hz, 2H), 4.49-4.37 (m, 1H), 4.05-3.97 (m, 2H), 3.96 (s, 3H), 3.55-3.32 (m, 2H), 3.21-2.97 (m, 4H). ¹⁹F NMR (376 MHz, Chloroform-d) δ -109.10. MS (ESI⁺): [M+H]⁺ 469.0/479.0. |
| **012** | Yellow solid. ¹H NMR (400 MHz, Chloroform-d) δ 7.57 (dd, *J* = 8.4, 2.2 Hz, 1H), 7.37-7.29 (m, 2H), 7.18 (dd, *J* = 8.5, 2.4 Hz, 1H), 7.07 (td, *J =* 8.3, 2.5 Hz, 1H), 6.98 (d, *J =* 8.6 Hz, 1H), 4.05-3.97 (m, 2H), 3.95 (s, 3H), 3.44 (dd, *J =* 27.2, 7.2 Hz, 2H), 3.22-2.95 (m, 6H), 2.71 (p, *J =* 7.7 Hz, 1H), 2.05 (q, *J* = 8.9 Hz, 2H), 1.94-1.68 (m, 4H). ¹⁹F NMR (376 MHz, Chloroform-d) δ - 109.18. MS (ESI⁺): [M+H]⁺ 481.1/483.1. |
| **013** | White solid. ¹H NMR (400 MHz, Chloroform-d) δ 7.60 (dd, *J =* 8.5, 2.2 Hz, 1H), 7.36 (d, *J =* 2.2 Hz, 1H), 7.33 (dd, *J =* 8.5, 5.9 Hz, 1H), 7.18 (dd, *J =* 8.5, 2.5 Hz, 1H), 7.07 (td, *J =* 8.2, 2.5 Hz, 1H), 6.98 (d, *J =* 8.6 Hz, 1H), 4.03-3.96 (m, 2H), 3.96 (s, 3H), 3.52-3.34 (m, 2H), 3.24-2.96 (m, 6H), 2.25 (dq, *J =* 15.4, 7.7 Hz, 1H), 1.89 (dd, *J =* 12.5, 7.5 Hz, 2H), 1.68-1.57 (m, 3H), 1.25-1.15 (m, 3H). ¹⁹F NMR (376 MHz, Chloroform-d) δ -109.19. MS (ESI⁺): [M+H]⁺ 495.2/497.1. |
| **014** | Yellow solid. ¹H NMR (400 MHz, Chloroform-d, *2 sets of rotamers)* δ 7.37-7.27 (m, 3H), 7.26-7.01 (m, 6H), 6.89 (d, *J =* 8.5 Hz, 1H), 6.73-6.70 (m, 1H), 4.42 (dd, *J =* 12.9, 7.2 Hz, 1H), 4.28-4.25 (m, 2H), 4.09-3.97 (m, 1H), 3.91 (s, 3H), 3.90-3.81 (m, 1H), 3.59-3.35 (m, 1H), 3.15 (dd, *J =* 12.9, 5.7 Hz, 1H), 3.05 (t, *J* = 13.8 Hz, 1H), 1.85-1.64 (m, 4H). ¹⁹F NMR (376 MHz, Chloroform-d, *2 sets of rotamers)* δ -109.27, -109.36. MS (ESI⁺): [M+H]⁺ 529.1/531.1. |
| **015** | White solid. ¹H NMR (400 MHz, Chloroform-*d*, *2 sets of rotamers)* δ 7.49-7.45 (m, 1H), 7.36 (dd, *J =* 8.5, 5.9 Hz, 1H), 7.21 (dd, *J =* 8.5, 2.4 Hz, 1H), 7.18-7.13 (m, 1H), 7.06 (dtd, *J =* 18.8, 8.2, 2.4 Hz, 1H), 6.95 (d, *J =* 8.5 Hz, 1H), 4.56-4.46 (m, 1H), 4.38-4.28 (m, 1H), 4.08 (s, 1H), 3.93 (s, 3H), 3.70-3.47 (m, 1H), 3.29 (dd, *J =* 13.0, 5.9 Hz, 1H), 3.13 (t, *J =* 11.7 Hz, 1H), 3.00 (s, 2H), 2.10-1.83 (m, 4H), 1.15 (s, 9H). ¹⁹F NMR (376 MHz, Chloroform-*d, 2 sets of rotamers)* δ -109.27, -109.38. MS (ESI⁺): [M+H]⁺ 509.2/ 511.2. |
| **016** | White solid. ¹H NMR (400 MHz, Chloroform-*d*, *multiple sets of diastereoisomers*) δ 7.61-7.56 (m, 1H), 7.43-7.30 (m, 2H), 7.22-7.16 (m, 1H), 7.11-7.04 (m, 1H), 7.01-6.96 (m, 1H), 4.22-3.92 (m, 4H), 3.88-3.71 (m, 2H), 3.58-3.28 (m, 3H), 3.20-2.77 (m, 3H), 1.94-1.85 (m, 1H), 1.51-1.23 (m, 8H), 1.03-0.81 (m, 7H). ¹⁹F NMR (376 MHz, Chloroform-*d*, *multiple sets of diastereoisomers) δ* -109.27, -109.33, -109.34. MS (ESI⁺): [M+H]⁺ 497.0/499.0. |
| **017** | White solid. ¹H NMR (400 MHz, Chloroform-d, *multiple sets of diastereoisomers)* δ 7.64-7.57 (m, 1H), 7.46-7.29 (m, 4H), 7.01-6.96 (m, 1H), 4.35-3.90 (m, 4H), 3.85-3.41 (m, 2H), 3.39-3.25 (m, 2H), 3.22-2.75 (m, 3H), 1.94-1.84 (m, 1H), 1.50-1.23 (m, 7H), 1.00-0.78 (m, 7H).¹⁹F NMR (376 MHz, Chloroform-*d*, *multiple sets of diastereoisomers)* δ -60.30, -60.31, - 60.32, -60.49, -109.19, -109.23, -109.25, -109.30. MS (ESI⁺): [M+H]⁺ 531.1. |
| **018** | Brown solid. ¹H NMR (400 MHz, Chloroform-*d*) δ 7.60 (dd, *J =* 8.5, 2.2 Hz, 1H), 7.44 (dd, *J =* 8.8, 2.5 Hz, 1H), 7.42-7.29 (m, 3H), 6.98 (d, *J =* 8.6 Hz, 1H), 4.05-3.90 (m, 5H), 3.37 (t, *J =* 5.1 Hz, 2H), 3.24-3.10 (m, 2H), 3.05-2.92 (m, 4H), 2.23 (dp, *J =* 13.3, 6.7 Hz, 1H), 1.06 (d, *J =* 6.7 Hz, 6H). ¹⁹F NMR (376 MHz, Chloroform-*d*) δ -60.2, -109.2. MS (ESI⁺): [M+H]⁺ 503.1. |
| **019** | White solid. ¹H NMR (400 MHz, Chloroform-d, *2 sets of rotamers)* δ 7.47 (d, *J =* 8.0 Hz, 1H), 7.37 (dd, *J =* 8.5, 5.8 Hz, 1H), 7.25 - 7.20 (m, 1H), 7.18 - 7.12 (m, 1H), 7.11 - 7.01 (m, 1H), 6.97 (d, *J =* 8.5 Hz, 1H), 4.51 (t, *J =* 10.9 Hz, 1H), 4.36 - 4.27 (m, 1H), 4.07 (s, 1H), 3.94 (s, 3H), 3.70 - 3.48 (m, 1H), 3.29 (dd, *J =* 13.0, 6.1 Hz, 1H), 3.13 (t, *J =* 11.5 Hz, 1H), 3.08 - 2.99 (m, 2H), 2.11 - 1.87 (m, 4H), 1.80 - 1.61 (m, 6H), 1.62 - 1.57 (m, 2H), 1.49 - 1.43 (m, 1H), 1.11 - 0.97 (m, 2H).¹⁹F NMR (376 MHz, Chloroform-d, 2 *sets of rotamers)* δ -109.25, -109.35. MS (ESI⁺): [M+H]⁺ 535.1/537.1. |
| **020** | White solid. ¹H NMR (400 MHz, Chloroform-d, *2 sets of rotamers)* δ 7.49 - 7.44 (m, 1H), 7.37 (dd, *J =* 8.5, 5.9 Hz, 1H), 7.25 - 7.13 (m, 2H), 7.06 (dtd, *J=* 19.0, 8.3, 2.4 Hz, 1H), 6.96 (d*, J =* 8.5 Hz, 1H), 4.55 - 4.46 (m, 1H), 4.31 (dd, *J =* 12.8, 6.3 Hz, 1H), 4.07 (s, 1H), 3.93 (s, 3H), 3.71 - 3.47 (m, 1H), 3.29 (dd, *J =* 13.1, 6.9 Hz, 1H), 3.13 (t, *J =* 11.9 Hz, 1H), 3.08 - 3.00 (m, 2H), 2.11 - 1.87 (m, 4H), 1.69 - 1.61 (m, 2H), 1.37 (h, *J=* 7.3 Hz, 2H), 0.88 (t, *J =* 7.3 Hz, 3H). ¹⁹F NMR (376 MHz, Chloroform-d, *2 sets of rotamers)* δ -109.25, -109.36. MS (ESI⁺): [M+H]⁺ 495.1/497.1. |
| **021** | White solid. ¹H NMR (400 MHz, Chloroform-d, *2 sets of rotamers)* δ 7.47 (dt, *J =* 8.5, 1.8 Hz, 1H), 7.25 - 7.17 (m, 2H), 7.17 - 7.12 (m, 1H), 7.06 (dtd, *J* = 18.8, 8.3, 2.4 Hz, 1H), 6.96 *(d, J =* 8.5 Hz, 1H), 4.55 - 4.46 (m, 1H), 4.36 - 4.27 (m, 1H), 4.11 - 4.04 (m, 1H), 3.94 (s, 3H), 3.59 (dd, *J =* 64.1, 12.3 Hz, 1H), 3.29 (dd, *J =* 13.1, 6.9 Hz, 1H), 3.13 (t, *J =* 11.3 Hz, 1H), 2.98 *(d, J* = 5.9 Hz, 2H), 2.06 - 1.88 (m, 3H), 1.81 (p, *J =* 6.2 Hz, 1H), 1.67 - 1.59 (m, 1H), 1.49 - 1.35 (m, 4H), 0.80 (d, *J* = 8.0 Hz, 6H). ¹⁹F NMR (376 MHz, Chloroform-d, *2 sets of rotamers)* δ -109.25, -109.36. MS (ESI⁺): [M+H]⁺ 523.2/525.2. |
| **022** | White solid. ¹H NMR (400 MHz, Chloroform-*d*, *2 sets of rotamers)* δ 7.46 - 7.42 (m, 1H), 7.36 (dd, *J* = 8.5, 5.9 Hz, 1H), 7.21 (p, *J* = 2.9 Hz, 1H), 7.19 - 7.13 (m, 1H), 7.06 (dtd, *J =* 18.8, 8.3, 2.5 Hz, 1H), 6.96 (d, *J =* 8.5 Hz, 1H), 4.55 - 4.46 (m, 1H), 4.34 - 4.26 (m, 1H), 4.06 (s, 1H), 3.93 (s, 3H), 3.70 - 3.47 (m, 1H), 3.28 (dd, *J=* 13.1, 5.1 Hz, 1H), 3.13 (t, *J =* 10.9 Hz, 1H), 2.89 (dqd, *J =* 10.4, 6.8, 3.7 Hz, 1H), 2.08 - 1.89 (m, 4H), 1.66 - 1.59 (m, 1H), 1.46 - 1.34 (m, 1H), 1.24 (dd, *J =* 6.9, 2.6 Hz, 3H), 0.96 (t, *J =* 7.5, 1.4 Hz, 3H). ¹⁹F NMR (376 MHz, Chloroform-d, *2 sets of rotamers) δ* -109.26, - 109.37. MS (ESI⁺): [M+H]⁺ 495.1/497.1. |
| **023** | White solid. ¹H NMR (400 MHz, Chloroform-d) δ 7.47 - 7.35 (m, 1H), 7.31 - 7.00 (m, 9H), 6.97 (d, *J =* 8.4 Hz, 1H), 4.59 - 4.45 (m, 1H), 4.37 - 4.21 (m, 1H), 4.11 - 4.01 (m, 1H), 3.96 (s, 3H), 3.72 - 3.64 (m, 0.5H), 3.59 - 3.45 (m, 0.5H), 3.39 - 3.23 (m, 1H), 3.23 - 3.06 (m, 1H), 3.06 - 2.91 (m, 1H), 2.87 - 2.77 (m, 1H), 2.66 - 2.50 (m, 1H), 2.37 - 2.19 (m, 1H), 2.10 - 1.83 (m, 3.5H), 1.78 - 1.63 (m, 1.5H), 1.42 - 1.23 (m, 3H). ¹⁹F NMR (376 MHz, Chloroform-d, *2 sets of rotamers)* δ -109.25, -109.36. MS (ESI⁺): [M+H]⁺ 571.1/573.1. |
| **024** | White solid. ¹H NMR (400 MHz, Chloroform-d) δ 7.50 - 7.26 (m, 2H), 7.25 - 6.98 (m, 8H), 6.94 (d, *J =* 8.5 Hz, 1H), 4.60 - 4.44 (m, 1H), 4.39 - 4.18 (m, 1H), 4.04 (s, 1H), 3.94 & 3.93 (s, 3H), 3.78 - 3.60 (m, 0.5H), 3.58 - 3.46 (m, 0.5H), 3.37 - 3.21 (m, 1H), 3.21 - 3.05 (m, 1H), 3.05 - 2.89 (m, 1H), 2.70 - 2.46 (m, 2H), 2.13 - 1.83 (m, 3.5H), 1.83 - 1.50 (m, 3.5H), 1.51 - 1.29 (m, 1H), 1.30 - 1.18 (m, 3H). ¹⁹F NMR (376 MHz, Chloroform-d, 2 *sets of rotamers)* δ -109.25, -109.36. MS (ESI⁺): [M+H]⁺ 585.1/587.2. |
| **025** | White solid. ¹H NMR (400 MHz, Chloroform-d) δ 7.46 - 7.40 (m, 1H), 7.40 - 7.32 (m, 0.5H), 7.26 - 6.91 (m, 9.5H), 4.56 - 4.43 (m, 1H), 4.31 - 4.21 (m, 1H), 4.10 - 3.98 (m, 1H), 3.97 - 3.90 (m, 3H), 3.73 - 3.60 (m, 0.5H), 3.54 - 3.44 (m, 0.5H), 3.31 - 3.22 (m, 1H), 3.19 - 3.05 (m, 1H), 2.86 - 2.57 (m, 3H), 2.24 - 2.06 (m, 1H), 2.06 - 1.80 (m, 5.5H), 1.75 - 1.58 (m, 1.5H), 1.05 - 0.91 (m, 3H). ¹⁹F NMR (376 MHz, Chloroform-d, *2 sets of rotamers) δ -* 109.26, -109.37. MS (ESI⁺): [M+H]⁺ 585.1/587.2. |
| **026** | White solid. ¹H NMR (400 MHz, Chloroform-d) δ 7.42 - 7.33 (m, 1.5H), 7.26 - 6.98 (m, 8.5H), 6.93 (d, *J* = 8.5 Hz, 1H), 4.57 - 4.43 (m, 1H), 4.32 - 4.21 (m, 1H), 4.08 - 3.99 (m, 1H), 3.94 (s, 3H), 3.72 - 3.61 (m, 0.5H), 3.54 - 3.44 (m, 0.5H), 3.34 - 3.22 (m, 1H), 3.12 (t, *J =* 11.0 Hz, 1H), 2.84 - 2.74 (m, 1H), 2.63 - 2.50 (m, 2H), 2.12 - 1.58 (m, 10H), 0.99 - 0.87 (m, 3H). ¹⁹F NMR (376 MHz, Chloroform-d, *2 sets of rotamers) δ* -109.25, -109.36. MS (ESI⁺): [M+H]⁺ 599.2/601.2. |
| **027** | ¹H NMR (400 MHz, Chloroform-*d*, *2 sets of rotamers)* δ 7.48 *(d, J =* 8.5 Hz, 1H), 7.40 (dd, *J= 8.5,* 5.9 Hz, 0.5H), 7.28 - 7.22 (m, 3.5H), 7.22 - 7.14 (m, 1H), 7.14 - 7.02 (m, 1H), 7.00 (d, *J = 8.5* Hz, 1H), 6.94 - 6.84 (m, 3H), 4.54 (dd, *J =* 13.3, 6.3 Hz, 1H), 4.39 - 4.28 (m, 1H), 4.17 - 4.04 (m, 1H), 4.03 - 3.89 (m, 3H), 3.77 (d, *J =* 12.4 Hz, 2H), 3.72 - 3.51 (m, 1H), 3.32 (d, *J=* 13.6 Hz, 1H), 3.16 (*t, J =* 10.8 Hz, 1H), 3.08 - 2.94 (m, 1H), 2.75 - 2.62 (m, 2H), 2.17 - 1.62 (m, 8H). ¹⁹F NMR (376 MHz, Chloroform-d, *2 sets of rotamers*) δ -109.23, -109.35. MS (ESI⁺): [M+H]⁺ 598.3/600.2. |
| **028** | White solid. ¹H NMR (400 MHz, Chloroform-*d*, *2 sets of rotamers)* δ 7.39 - 7.33 (m, 1H), 7.30 (dd, *J =* 8.5, 5.8 Hz, 0.5H), 7.23 - 7.20 (m, 1H), 7.19 - 7.16 (m, 1H), 7.16 - 7.11 (m, 2.5H), 7.11 - 7.05 (m, 3H), 6.99 (dtd, *J* =18.8, 8.3, 2.5 Hz, 1H), 6.89 (dd, *J =* 8.6*,* 1.3 Hz, 1H), 4.53 - 4.39 (m, 1H), 4.28 - 4.17 (m, 1H), 4.08 - 3.97 (m, 1H), 3.93 - 3.81 (m, 3H), 3.65 - 3.37 (m, 1H), 3.29 - 3.15 (m, 1H), 3.12 - 3.04 (m, 1H), 3.04 - 2.94 (m, 2H), 2.85 - 2.73 (m, 1H), 2.72 - 2.61 (m, 2H), 2.57 - 2.42 (m, 2H), 2.02 - 1.54 (m, 10H). ¹⁹F NMR (376 MHz, Chloroform-d, *2 sets of rotamers) δ* -109.26, -109.38. MS (ESI⁺): [M+H]⁺ 626.3/628.3. |
| **029** | ¹H NMR (400 MHz, Chloroform-*d*, *2 sets of rotamers)* δ 7.39 - 7.33 (m, 1H), 7.30 (dd, *J=* 8.5, 5.8 Hz, 0.5H), 7.16 - 6.92 (m, 5.5H), 6.92 - 6.84 (m, 3H), 4.44 (dd, *J =* 13.0, 6.0 Hz, 1H), 4.28 - 4.18 (m, 1H), 4.06 - 3.95 (m, 1H), 3.92 - 3.80 (m, 3H), 3.66 - 3.39 (m, 1H), 3.22 (d, *J =* 12.8 Hz, 1H), 3.06 (t, *J=* 10.4 Hz, 1H), 3.02 - 2.91 (m, 2H), 2.78 (t, *J =* 12.3 Hz, 1H), 2.68 - 2.58 (m, 2H), 2.51 - 2.38 (m, 2H), 1.99 - 1.55 (m, 10H). ¹⁹F NMR (376 MHz, Chloroform-d, *2 sets of rotamers) δ* -109.24, -109.36, -117.22, - 117.23. MS (ESI⁺): [M+H]⁺ 644.3/646.3. |
| **030** | Off-white solid. ¹H NMR (400 MHz, Chloroform-d, *2 sets of rotamers) δ* 7.58 (dd, *J =* 8.4, 2.1 Hz, 1H), 7.42 - 7.34 & 7.26 - 7.15 (m, 5H), 7.14 - 7.01 (m, 2H), 6.97 - 6.80 (m, 4H), 4.64 (d, *J = 12.9* Hz, 1H), 3.89 - 3.71 (m, 3H), 3.70 - 3.61 & 3.57 - 3.41 (m, 2H), 3.34 - 3.17 (m, 2H), 3.06 - 2.89 (m, 1H), 2.67 (t, *J =* 12.0 Hz, 2H), 2.24 - 1.96 & 1.88 - 1.71 (m, 8H). ¹⁹F NMR (376 MHz, Chloroform-d, *2 sets of rotamers) δ* -108.72, -108.86. MS (ESI⁺): [M+H]⁺ 584.2/586.2. |
| **031** | White solid. ¹H NMR (400 MHz, Chloroform-*d*) δ 7.55 (dd, *J* = 8.4, 2.1 Hz, 1H), 7.41 - 7.32 & 7.25 - 7.23 (m, 3H), 7.30 - 7.27 (m, 1H), 7.23 - 7.03 (m, 6H), 6.92 *(br* s, 1H), 4.64 (d, *J =* 12.2 Hz, 1H), 3.84 - 3.74 (m, 1H), 3.70 - 3.59 & 3.57 - 3.41 (m, 2H), 3.33 - 3.21 (m, 2H), 3.07 (d, *J =* 11.4 Hz, 2H), 2.86 (t, *J =* 12.5 Hz, 1H), 2.79 - 2.67 (m, 2H), 2.62 - 2.48 (m, 2H), 2.25 - 1.91 & 1.77 - 1.60 (m, 10H). ¹⁹F NMR (376 MHz, Chloroform-*d*, *2 sets of rotamers)* δ -108.75, -108.89. MS (ESI⁺): [M+H]⁺ 612.2/614.1. |
| **032** | White solid. ¹H NMR (400 MHz, Chloroform-d, *2 sets of rotamers*) δ 7.49 - 7.46 (m, 1H), 7.40 - 7.36 (m, 0.5H), 7.30 - 7.28 (m, 0.5H), 7.25 - 6.99 (m, 4H), 4.67 - 4.59 (m, 1H), 3.75 - 3.69 (m, 1H), 3.66 - 3.43 (m, 2H), 3.30 - 3.22 (m, 2H), 2.97 (s, 2H), 2.31 (s, 3H), 2.17 - 1.98 (m, 3.5H), 1.75 - 1.68 (m, 0.5H), 1.16 (s, 9H). ¹⁹F NMR (376 MHz, Chloroform-d, *2 sets of rotamers)* δ -108.81, -108.94. MS (ESI⁺): [M+H]⁺ 509.0/511.0. |
| **033** | White solid. ¹H NMR (400 MHz, Chloroform-*d*, *2 sets of rotamers)* δ 7.59 & 7.58 (m, 1H), 7.43 - 7.36 (m, 0.5H), 7.27 - 7.15 (m, 2.5H), 7.14 - 7.03 (m, 1H), 6.68 (br s , 1H), 4.63 - 4.55 (m, 1H), 4.22 - 4.13 (m, 1H), 3.97 - 3.87 (m, 1H), 3.77 - 3.47 (m, 1H), 3.33 - 3.26 (m, 1H), 3.25 - 3.16 (m, 1H), 3.01 (s, 2H), 2.20 - 1.90 (m, 3.5H), 1.76 - 1.65 (m, 0.5H), 1.20 (s, 9H). ¹⁹F NMR (376 MHz, Chloroform-d, *2 sets of rotamers)* δ -108.87, -109.01. MS (ESI⁺): [M+H]⁺ 529.0/531.0. |
| **034** | White solid. ¹H NMR (400 MHz, Chloroform-*d*, *2 sets of rotamers)* δ 7.65 - 7.20 (m, 1H), 7.43 - 7.33 (m, 0.5H), 7.35 - 7.30 (m, 1H), 7.27 - 7.16 (m, 1.5H), 7.15 - 7.03 (m, 1H), 4.63 & 4.59 (s, 1H), 4.46 - 4.17 (m, 4H), 4.13 - 3.98 (m, 1H), 3.85 - 3.75 (m, 2H), 3.75 - 3.50 (m, 1H), 3.35 & 3.32 (s, 1H), 3.27 - 3.17 (m, 1H), 3.02 (s, 2H), 2.15 - 1.90 (m, 3.5H), 1.74 - 1.63 (m, 0.5H), 1.22 (s, 9H). ¹⁹F NMR (376 MHz, Chloroform-d, *2 sets of rotamers)* δ -108.80, -109.94. MS (ESI⁺): [M+H]⁺ 573.1/575.1/577.1 |
| **035** | White solid. ¹H NMR (400 MHz, Chloroform-*d*, *2 sets of rotamers)* δ 7.67 - 7.57 (m, 1H), 7.44 - 7.34 (m, 0.5H), 7.31 - 7.27 (m, 1H), 7.26 - 7.02 (m, 2.5H), 4.69 - 4.53 (m, 1H), 4.22 - 4.11 (m, 1H), 4.00 - 3.89 (m, 1H), 3.79 - 3.69 (m, 0.5H), 3.63 - 3.53 (m, 0.5H), 3.41 - 3.29 (m, 1H), 3.29 - 3.14 (m, 1H), 3.01 (s, 2H), 2.24 - 1.86 (m, 3.5H), 1.79 - 1.65 (m, 0.5H), 1.24 - 1.15 (m, 9H). ¹⁹F NMR (376 MHz, Chloroform-d, *2 sets of rotamers)* δ -108.96, -109.09,. MS (ESI⁺): [M+H]⁺ 547.1/549.1. |
| **036** | White solid. ¹H NMR (400 MHz, Chloroform-*d*, *2 sets of rotamers)* δ 7.60 - 7.54 (m, 1H), 7.43 - 7.33 (m, 0.5H), 7.26 - 7.00 (m, 3.5H), 4.68 - 4.50 (m, 1H), 3.98 - 3.83 (m, 1H), 3.76 - 3.58 (m, 1.5H), 3.58 - 3.47 (m, 0.5H), 3.37 - 3.15 (m, 2H), 3.00 (s, 2H), 2.55 - 2.40 (m, 3H), 2.16 - 1.87 (m, 3.5H), 1.75 - 1.62 (m, 0.5H), 1.19 (s, 9H). ¹⁹F NMR (376 MHz, Chloroform-d, *2 sets of rotamers)* δ -108.97, -109.11. MS (ESI⁺): [M+H]⁺ 527.1/529.1. |

### Example 2: Biological activity of the compounds

The purpose of this experiment was to evaluate the GFRα1-RET activity of the compounds **001-036** according to the invention.

### Materials and methods

The compounds were tested for their activating activity of Elk1 signaling using the previously developed reporter-gene-based system in cells expressing GFRα1-RET (MG87 murine fibroblast stably transfected with PathDetect Elk-1, GFRα1, and RET) disclosed in Sidorova, Y. A. et al.: "Persephin signaling through GFRα1: The potential for the treatment of Parkinson's disease." Molecular and Cellular Neuroscience, July 2010, Vol. 44, pp. 223-232. DOI: 10.1016/j.mcn.2010.03.009. For EC₅₀ determination, a dose-response test was performed using 6 concentrations of each tested compound. Dose-response curves were fitted using the sigmoidal dose-response (variable slope) analysis in GraphPad Prism program (Graph Pad Inc) and EC₅₀ of agonist / activator activity was calculated. Dose-response experiments were all performed in duplicate, two times independently.

### Results

The results are presented on **Table 4** below (* means "50 µM > EC₅₀ > 10µM", ** means "1µM ≤ EC₅₀ ≤ 10µM", *** means "EC₅₀ < 1µM").

**Table 4**

| **Cpd** | **Name** | **Luciferase assay EC₅₀** |
|---|---|---|
| **001** | [4-[5-(1-ethylpropylsulfonyl)-2-methoxy-phenyl]piperazin-1-yl]-[4-fluoro-2-(trifluoromethyl)phenyl]methanone | * |
| **002** | [4-(5-cyclopentylsulfonyl-2-methoxy-phenyl)piperazin-1-yl]-[4-fluoro-2-(trifluoromethyl)phenyl]methanone | * |
| **003** | [4-(5-cyclohexylsulfonyl-2-methoxy-phenyl)piperazin-1-yl]-[4-fluoro-2-(trifluoromethyl)phenyl]methanone | * |
| **004** | (2-chloro-4-fluoro-phenyl)-[(1S,5R)-8-(5-cyclopentylsulfonyl-2-methoxy-phenyl)-3,8-diazabicyclo[3.2.1]octan-3-yl]methanone | ** |
| **005** | (2-chloro-4-fluoro-phenyl)-[(1S,5R)-8-[5-(1-ethylpropylsulfonyl)-2-methoxy-phenyl]-3,8-diazabicyclo[3.2.1]octan-3-yl]methanone | ** |
| **006** | (2-chloro-4-fluoro-phenyl)-[rac-(3S)-4-(5-cyclopentylsulfonyl-2-methoxy-phenyl)-3-methyl-piperazin-1-yl]methanone | ** |
| **007** | (2-chloro-4-fluoro-phenyl)-[rac-(3 S)-4-[5-(1-ethylpropylsulfonyl)-2-methoxy-phenyl]-3-methyl-piperazin-1-yl]methanone | ** |
| **008** | (2-chloro-4-fluoro-phenyl)-[4-(5-isopropylsulfonyl-2-methoxy-phenyl)piperazin-1-yl]methanone | * |
| **009** | (2-chloro-4-fluoro-phenyl)-[4-[5-(cyclopropylmethylsulfonyl)-2-methoxy-phenyl]piperazin-1-yl]methanone | ** |
| **010** | (2-chloro-4-fluoro-phenyl)-[(1S,5R)-8-(5-cyclobutylsulfonyl-2-methoxy-phenyl)-3,8-diazabicyclo[3.2.1]octan-3-yl]methanone | ** |
| **011** | (2-chloro-4-fluoro-phenyl)-[4-[2-methoxy-5-(oxetan-3-ylsulfonyl)phenyl]piperazin-1-yl]methanone | * |
| **012** | (2-chloro-4-fluoro-phenyl)-[4-[5-(cyclobutylmethylsulfonyl)-2-methoxy-phenyl]piperazin-1-yl]methanone | * |
| **013** | (2-chloro-4-fluoro-phenyl)-[4-[5-(cyclopentylmethylsulfonyl)-2-methoxy-phenyl]piperazin-1-yl]methanone | ** |
| **014** | [(1S,5R)-8-(5-benzylsulfonyl-2-methoxy-phenyl)-3,8-diazabicyclo[3.2.1]octan-3-yl]-(2-chloro-4-fluoro-phenyl)methanone | * |
| **015** | (2-chloro-4-fluoro-phenyl)-[(1S,5R)-8-[5-(2,2-dimethylpropylsulfonyl)-2-methoxy-phenyl]-3,8-diazabicyclo[3.2.1]octan-3-yl]methanone | ** |
| **016** | (2-chloro-4-fluoro-phenyl)-[rac-(3S)-4-[2-methoxy-5-[rac-(1S)-1-methylpropyl]sulfonyl-phenyl]-3-methyl-piperazin-1-yl]methanone | ** |
| **017** | [4-fluoro-2-(trifluoromethyl)phenyl]-[rac-(3 S)-4-[2-methoxy-5-[rac-(1S)-1-methylpropyl]sulfonyl-phenyl]-3-methyl-piperazin-1-yl]methanone | ** |
| **018** | [4-fluoro-2-(trifluoromethyl)phenyl]-[4-(5-isobutylsulfonyl-2-methoxy-phenyl)piperazin-1-yl]methanone | * |
| **019** | (2-chloro-4-fluoro-phenyl)-[(1S,5R)-8-[5-(2-cyclopentylethylsulfonyl)-2-methoxy-phenyl]-3,8-diazabicyclo[3.2.1]octan-3-yl]methanone | ** |
| **020** | [(1S,5R)-8-(5-butylsulfonyl-2-methoxy-phenyl)-3,8-diazabicyclo[3.2.1]octan-3-yl]-(2-chloro-4-fluoro-phenyl)methanone | ** |
| **021** | (2-chloro-4-fluoro-phenyl)-[(1S,5R)-8-[5-(2-ethylbutylsulfonyl)-2-methoxy-phenyl]-3,8-diazabicyclo[3.2.1]octan-3-yl]methanone | ** |
| **022** | (2-chloro-4-fluoro-phenyl)-[(1S,5R)-8-[2-methoxy-5-[(1SR)-1-methylpropyl]sulfonyl-phenyl]-3,8-diazabicyclo[3.2.1]octan-3-yl]methanone | ** |
| **023** | (2-chloro-4-fluoro-phenyl)-[(1S,5R)-8-[2-methoxy-5-[(1 SR)-1-methyl-3-phenyl-propyl]sulfonyl-phenyl]-3,8-diazabicyclo[3.2.1]octan-3-yl]methanone | ** |
| **024** | (2-chloro-4-fluoro-phenyl)-[(1S,5R)-8-[2-methoxy-5-[(1SR)-1-methyl-4-phenyl-butyl]sulfonyl-phenyl]-3,8-diazabicyclo[3.2.1]octan-3-yl]methanone | ** |
| **025** | (2-chloro-4-fluoro-phenyl)-[(1 S,5R)-8-[5-[(1 SR)-1-ethyl-3-phenyl-propyl]sulfonyl-2-methoxy-phenyl]-3,8-diazabicyclo[3.2.1]octan-3-yl]methanone | ** |
| **026** | (2-chloro-4-fluoro-phenyl)-[(1 S,5R)-8-[5-[(1 SR)-1-ethyl-4-phenyl-butyl]sulfonyl-2-methoxy-phenyl]-3,8-diazabicyclo[3.2.1]octan-3-yl]methanone | ** |
| **027** | (2-chloro-4-fluoro-phenyl)-[(1S,5R)-8-[2-methoxy-5-[(1-phenyl-4-piperidyl)sulfonyl]phenyl]-3,8-diazabicyclo[3.2.1]octan-3-yl]methanone | *** |
| **028** | (2-chloro-4-fluoro-phenyl)-[(1S,5R)-8-[2-methoxy-5-[[1-(2-phenylethyl)-4-piperidyl]sulfonyl]phenyl]-3,8-diazabicyclo[3.2.1]octan-3-yl]methanone | ** |
| **029** | (2-chloro-4-fluoro-phenyl)-[(1S,5R)-8-[5-[[1-[2-(4-fluorophenyl)ethyl]-4-piperidyl]sulfonyl]-2-methoxy-phenyl]-3,8-diazabicyclo[3.2.1]octan-3-yl]methanone | ** |
| **030** | (2-chloro-4-fluoro-phenyl)-[(1S,5R)-8-[2-hydroxy-5-[(1-phenyl-4-piperidyl)sulfonyl]phenyl]-3,8-diazabicyclo[3.2.1]octan-3-yl]methanone | *** |
| **031** | (2-chloro-4-fluoro-phenyl)-[(1S,5R)-8-[2-hydroxy-5-[[1-(2-phenylethyl)-4-piperidyl]sulfonyl]phenyl]-3,8-diazabicyclo[3.2.1]octan-3-yl]methanone | ** |
| **032** | (2-chloro-4-fluoro-phenyl)-[(1S,5R)-8-[5-(2,2-dimethylpropylsulfonyl)-2-hydroxy-3-methyl-phenyl]-3,8-diazabicyclo[3.2.1]octan-3-yl]methanone | ** |
| **033** | [(1S,5R)-8-[3-chloro-5-(2,2-dimethylpropylsulfonyl)-2-hydroxy-phenyl]-3,8-diazabicyclo[3.2.1]octan-3-yl]-(2-chloro-4-fluoro-phenyl)methanone | *** |
| **034** | [(1S,5R)-8-[3-chloro-5-(2,2-dimethylpropylsulfonyl)-2-(2-hydroxyethoxy)phenyl]-3,8-diazabicyclo[3.2.1]octan-3-yl]-(2-chloro-4-fluoro-phenyl)methanone | ** |
| **035** | (2-chloro-4-fluoro-phenyl)-[(1S,5R)-8-[2,3-dichloro-5-(2,2-dimethylpropylsulfonyl)phenyl]-3,8-diazabicyclo[3.2.1]octan-3-yl]methanone | *** |
| **036** | [(1S,5R)-8-[3-chloro-5-(2,2-dimethylpropylsulfonyl)-2-methyl-phenyl]-3,8-diazabicyclo[3.2.1]octan-3-yl]-(2-chloro-4-fluoro-phenyl)methanone | *** |

The above results clearly evidence that the tested compounds 001-036 have significant GFRα1-RET activity. Thus, the compounds of the invention are useful as neuroprotective and neurorestorative agents. The tested compounds 001-036 are well-representing the class of the compounds of formula (I).

## Claims

1. Compound of formula (I) 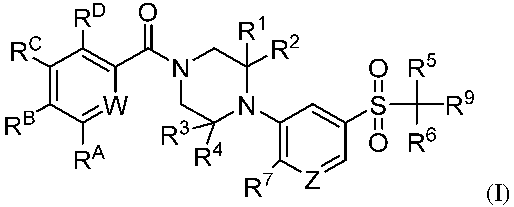 or a pharmaceutically acceptable salt and/or solvate thereof;
wherein
**W** represents CH or N;
**R^{A}**, **R^{B}**, **R^{C}** and **R^{D}** each independently represents hydrogen, F, Cl, CH₃, CF₃, CHF₂ or CH₂F,
*provided that* at least one among R^{A}, R^{B}, R^{C} and R^{D} does not represent hydrogen;
**R¹** represents hydrogen or (C₁-C₈) alkyl, wherein the alkyl is optionally substituted by at least one OH, (C₁-C₃) alkoxy or F; and **R²**, **R³** and **R**⁴ represents hydrogen;
or **R**¹ and **R**⁴ form together -CH₂-O-CH₂- or -CH₂-CH₂-, wherein the -CH₂-CH₂- is optionally substituted by at least one F, OH or OCH₃; and **R**² and **R**³ each represents hydrogen;
**R**⁷ represents hydrogen, OH, halogen, (C₁-C₈) alkyl, cycloalkyl, (C₁-C₈) alkyl-O-, cycloalkyl-O-, cycloalkyl-(C₁-C₈) alkyl-O-, heterocycloalkyl-O-, R¹¹O-(C₁-C₈) alkyl-O-, R¹¹R¹²N-(C₁-C₈) alkyl-O-, (R¹¹O)(R¹²)N-(C₁-C₈) alkyl-O-, R¹¹R¹²N-(C₁-C₈) alkyl-, R¹¹O-(C₁-C₈) alkyl-, NR¹¹R¹², CN, CO₂H, CO₂R¹¹, CONH₂, CON(R¹¹)H, heterocycloalkyl, or heteroaryl; wherein **R¹¹** and **R¹²** each independently represents hydrogen or (C₁-C₈) alkyl;
wherein the alkyl or cycloalkyl in **R**⁷ is optionally substituted by at least one F, Cl, OH, =O, (C₁-C₈) alkyl, (C₁-C₈) alkyl-O-, heterocycloalkyl, aryl or heteroaryl;
wherein the heterocycloalkyl, aryl or heteroaryl is optionally substituted by at least one F, Cl, (C₁-C₈) alkyl, CF₃, CHF₂, CH₂F, OCF₃, CN, OH, =O, →O, (C₁-C₈) alkoxy, NR¹³R¹⁴, R¹³R¹⁴N-(C₁-C₈) alkyl-, R¹³O₂C(C₁-C₈) alkyl-, CO₂H, R¹³R¹⁴N-C(O)-, R¹³O-NR¹⁴-, or (C₁-C₈) alkyl-CO₂-; wherein **R**¹³ and **R**¹⁴ each independently represents hydrogen or (C₁-C₈) alkyl;
**Z** represents C-H, C-**R⁸** or N;
wherein **R⁸** represents (C₁-C₄) alkyl, F, Cl, CF₃, CHF₂, CH₂F, OCF₃, CN, OH or (C₁-C₄) alkoxy;
or **Z** represents C-**R⁸** and **R**⁷ and **R⁸** form together with the carbon atoms to which they are bound a cycloalkyl or heterocycloalkyl,
wherein the cycloalkyl or heterocycloalkyl is optionally substituted by at least one F, OH, =O, →O, (C₁-C₈) alkyl, CF₃, HO₂C-CH₂-, (C₁-C₄) alkyl-CO₂-CH₂-, R¹⁵R¹⁶N-CH₂-, aryl, or aryl-(C₁-C₈) alkyl-, wherein **R¹⁵** and **R¹⁶** each independently represents hydrogen or (C₁-C₈) alkyl;
**R⁵** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
wherein the alkyl or cycloalkyl in **R⁵** is optionally substituted by at least one F, Cl, (C₁-C₈) alkyl, CF₃, OCF₃, CN, OH, =O, (C₁-C₈) alkoxy, NR¹⁷R¹⁸, CO₂H, R¹⁷R¹⁸N-C(O)-, R¹⁷O-NR¹⁸-, heterocycloalkyl, aryl or heteroaryl; wherein **R¹⁷** and **R¹⁸** each independently represents hydrogen or (C₁-C₈) alkyl;
wherein the heterocycloalkyl, aryl or heteroaryl is optionally substituted by at least one F, Cl, (C₁-C₈) alkyl, CF₃, CHF₂, CH₂F, OCF₃, CN, OH, =O, →O, (C₁-C₈) alkoxy, NR¹⁹R²⁰, CO₂H, R¹⁹R²⁰N-C(O)-, R¹⁹O-NR²⁰-, (C₁-C₈) alkyl-CO₂-, R¹⁹R²⁰N-(C₁-C₈) alkyl-, R¹⁹O₂C-(C₁-C₈) alkyl-, heterocycloalkyl, heteroaryl, aryl, or aryl-(C₁-C₈) alkyl-; wherein **R¹⁹** and **R²⁰** each independently represents hydrogen or (C₁-C₈) alkyl;
**R⁶** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
wherein the alkyl or cycloalkyl in **R⁶** is optionally substituted by at least one F, Cl, (C₁-C₈) alkyl, CF₃, OCF₃, CN, OH, =O, (C₁-C₈) alkoxy, NR²¹R²², CO₂H, R²¹R²²N-C(O)-, R²¹O-NR²²-, heterocycloalkyl, aryl or heteroaryl; wherein **R²¹** and **R²²** each independently represents hydrogen or (C₁-C₈) alkyl;
wherein the heterocycloalkyl, aryl or heteroaryl is optionally substituted by at least one F, Cl, (C₁-C₈) alkyl, CF₃, CHF₂, CH₂F, OCF₃, CN, OH, =O, →O, (C₁-C₈) alkoxy, NR²³R²⁴, CO₂H, R²³R²⁴N-C(O)-, R²³O-NR²⁴-, (C₁-C₈) alkyl-CO₂-, R²³R²⁴N-(C₁-C₈) alkyl-, R²³O₂C-(C₁-C₈) alkyl-, heterocycloalkyl, heteroaryl, aryl, or aryl-(C₁-C₈) alkyl-; wherein **R²³** and **R²⁴** each independently represents hydrogen or (C₁-C₈) alkyl;
or **R⁵** and **R⁶** form together with the carbon atom to which they are bound a cycloalkyl or heterocycloalkyl,
wherein the cycloalkyl or heterocycloalkyl is optionally substituted by at least one F, Cl, (C₁-C₈) alkyl, CF₃, OCF₃, CN, OH, =O, →O, (C₁-C₈) alkoxy, NR²⁵R²⁶, CO₂H, (C₁-C₈) alkyl-CO₂-, R²⁵R²⁶N-C(O)-, R²⁵O-NR²⁶-, R²⁵R²⁶N-(C₁-C₈) alkyl-, R²⁵O₂C-(C₁-C₈) alkyl-, cycloalkyl, heterocycloalkyl, aryl, heteroaryl, cycloalkyl-(C₁-C₈) alkyl-, heterocycloalkyl-(C₁-C₈) alkyl-, aryl-(C₁-C₈) alkyl-, heteroaryl-(C₁-C₈) alkyl-, aryl-cycloalkyl-, cycloalkyl-O-, heterocycloalkyl-O-, aryl-O-, heteroaryl-O-, cycloalkyl-(C₁-C₈) alkyl-O-, heterocycloalkyl-(C₁-C₈) alkyl-O-, aryl-(C₁-C₈) alkyl-O-, heteroaryl-(C₁-C₈) alkyl-O-, cycloalkyl-NR²⁵-, heterocycloalkyl-NR²⁵-, aryl-NR²⁵-, heteroaryl-NR²⁵-, cycloalkyl-(C₁-C₈) alkyl-NR²⁵-, heterocycloalkyl-(C₁-C₈) alkyl-NR²⁵-, aryl-(C₁-C₈) alkyl-NR²⁵-, heteroaryl-(C₁-C₈) alkyl-NR²⁵-, benzylidene, heteroarylidene, aryl-(C₁-C₈) alkyl-ylidene- or heteroaryl-(C₁-C₈) alkyl-ylidene-;
wherein **R²⁵** and **R²⁶** each independently represents hydrogen or (C₁-C₈) alkyl;
wherein the heterocycloalkyl, aryl, heteroaryl, benzylidene or heteroarylidene is optionally substituted at least one F, Cl, (C₁-C₈) alkyl, CF₃, CHF₂, CH₂F, OCF₃, CN, OH, =O, →O, (C₁-C₈) alkoxy, NR²⁷R²⁸, CO₂H, R²⁷R²⁸N-C(O)-, R²⁷O-NR²⁸-, (C₁-C₈) alkyl-CO₂-, R²⁷R²⁸N-(C₁-C₈) alkyl-, R²⁷O₂C-(C₁-C₈) alkyl-, heterocycloalkyl, heteroaryl, aryl, or aryl-(C₁-C₈) alkyl-; wherein **R²⁷** and **R²⁸** each independently represents hydrogen or (C₁-C₈) alkyl;
**R⁹** represents hydrogen, (C₁-C₈) alkyl, CH₃ substituted by one to three (C₁-C₈) alkyl groups, cycloalkyl, heterocycloalkyl, aryl, or cycloalkyl-(C₁-C₈) alkyl;
wherein the alkyl or cycloalkyl in **R⁹** is optionally substituted by at least one F, Cl, (C₁-C₈) alkyl, CF₃, OCF₃, CN, OH, =O, (C₁-C₈) alkoxy, NR²⁹R³⁰, CO₂H, R²⁹R³⁰N-C(O)-, R²⁹O-NR³⁰-, heterocycloalkyl, aryl or heteroaryl; wherein **R²⁹** and **R³⁰** each independently represents hydrogen or (C₁-C₈) alkyl;
wherein the heterocycloalkyl, aryl or heteroaryl is optionally substituted by at least one F, Cl, (C₁-C₈) alkyl, CF₃, OCF₃, CN, OH, =O, →O, (C₁-C₈) alkoxy, NR³¹R³², CO₂H, R³¹R³²N-C(O)-, R³¹O-NR³²-, (C₁-C₈) alkyl-CO₂-, R²³R²⁴N-(C₁-C₈) alkyl-, R²³O₂C-(C₁-C₈) alkyl-, heterocycloalkyl, heteroaryl, aryl, or aryl-(C₁-C₈) alkyl-; wherein **R³¹** and **R¹²** each independently represents hydrogen or (C₁-C₈) alkyl.

2. The compound according to claim **1,** wherein at least one among **R^{A}, R^{B}, R^{C}** and **R^{D}** represents hydrogen, preferably at least one among **R^{A}** and **R^{C}** represents hydrogen, more preferably **R^{C}** represents hydrogen.

3. The compound according to claim **1** or claim **2,** wherein at least one among **R^{A}, R^{B}** and **R^{D}** represents F or Cl, preferably at least one among **R^{B}** and **R^{D}** represents F or Cl; more preferably **R^{B}** and **R^{D}** each independently represents F or Cl.

4. The compound according to any one of claims **1** to **3,** wherein **R¹** and **R⁴** form together -CH₂-O-CH₂- or -CH₂-CH₂, preferably **R¹** and **R⁴** form together -CH₂-CH₂-; wherein the -CH₂-CH₂- is optionally substituted by at least one F, OH or OCH₃.

5. The compound according to any one of claims **1** to **4,** wherein **R¹** represents methyl, ethyl, CF₃ or CH₃OCH₂-.

6. The compound according to any one of claims **1** to **5,** wherein **R⁷** represents hydrogen, OH or halogen, preferably **R⁷** represents OH or Cl.

7. The compound according to any one of claims **1** to **6,** wherein **R⁷** represents (C₁-C₈) alkyl-O- or cycloalkyl-O-, wherein the alkyl or cycloalkyl is optionally substituted by at least one F, Cl, OH, (C₁-C₈) alkoxy or aryl; preferably **R⁷** represents OCH₃ or HO-CH₂-CH₂-O.

8. The compound according to any one of claims **1** to **7,** wherein **R⁵** and **R⁶** each independently represents (C₁-C₈) alkyl, aryl-(C₁-C₈) alkyl-, and **R⁹** represents hydrogen, preferably **R⁵** and **R⁶** each independently represents methyl, ethyl, *n-*propyl, 2-phenylethyl- or 3-phenylpropyl-.

9. The compound according to any one of claims **1** to **8,** wherein **R⁶** represents (C₁-C₈) alkyl, cycloalkyl-(C₁-C₈)-alkyl or aryl and **R⁵** and **R⁹** each represents hydrogen, preferably **R⁶** represents i-propyl, n-propyl, *tert-butyl,* i-pentyl, cyclopropyl, cyclobutyl, cyclopentyl, cyclopentyl-CH₂- or phenyl.

10. The compound according to any one of claims **1** to **8,** wherein **R⁵** and **R⁶** form together with the carbon atom to which they are bound a cycloalkyl or heterocycloalkyl and **R⁹** represents hydrogen; preferably **R⁵** and **R⁶** form together with the carbon atom to which they are bound a cyclobutyl, cyclopentyl, cyclohexyl, oxetane, 1-phenyl-4-piperidyl, 1-(2-phenylethyl)-4-piperidyl or 1-[2-(4-fluorophenyl)ethyl]-4-piperidyl.

11. The compound according to any one of claims **1** to **10,** wherein said compound is selected from:
| | |
|---|---|
| **001** | [4-[5-(1-ethylpropylsulfonyl)-2-methoxy-phenyl]piperazin-1-yl]-[4-fluoro-2-(trifluoromethyl)phenyl]methanone |
| **002** | [4-(5-cyclopentylsulfonyl-2-methoxy-phenyl)piperazin-1-yl]-[4-fluoro-2-(trifluoromethyl)phenyl]methanone |
| **003** | [4-(5-cyclohexylsulfonyl-2-methoxy-phenyl)piperazin-1-yl]-[4-fluoro-2-(trifluoromethyl)phenyl]methanone |
| **004** | (2-chloro-4-fluoro-phenyl)-[(1S,5R)-8-(5-cyclopentylsulfonyl-2-methoxy-phenyl)-3,8-diazabicyclo[3.2.1]octan-3-yl]methanone |
| **005** | (2-chloro-4-fluoro-phenyl)-[(1S,5R)-8-[5-(1-ethylpropylsulfonyl)-2-methoxy-phenyl]-3,8-diazabicyclo[3.2.1]octan-3-yl]methanone |
| **006** | (2-chloro-4-fluoro-phenyl)-[rac-(3S)-4-(5-cyclopentylsulfonyl-2-methoxy-phenyl)-3-methyl-piperazin-1-yl]methanone |
| **007** | (2-chloro-4-fluoro-phenyl)-[rac-(3S)-4-[5-(1-ethylpropylsulfonyl)-2-methoxy-phenyl]-3-methyl-piperazin-1-yl]methanone |
| **008** | (2-chloro-4-fluoro-phenyl)-[4-(5-isopropylsulfonyl-2-methoxy-phenyl)piperazin-1-yl]methanone |
| **009** | (2-chloro-4-fluoro-phenyl)-[4-[5-(cyclopropylmethylsulfonyl)-2-methoxy-phenyl]piperazin-1-yl]methanone |
| **010** | (2-chloro-4-fluoro-phenyl)-[(1S,5R)-8-(5-cyclobutylsulfonyl-2-methoxy-phenyl)-3,8-diazabicyclo[3.2.1]octan-3-yl]methanone |
| **011** | (2-chloro-4-fluoro-phenyl)-[4-[2-methoxy-5-(oxetan-3-ylsulfonyl)phenyl]piperazin-1-yl]methanone |
| **012** | (2-chloro-4-fluoro-phenyl)-[4-[5-(cyclobutylmethylsulfonyl)-2-methoxy-phenyl]piperazin-1-yl]methanone |
| **013** | (2-chloro-4-fluoro-phenyl)-[4-[5-(cyclopentylmethylsulfonyl)-2-methoxy-phenyl]piperazin-1-yl]methanone |
| **014** | [(1S,5R)-8-(5-benzylsulfonyl-2-methoxy-phenyl)-3,8-diazabicyclo[3.2.1]octan-3-yl]-(2-chloro-4-fluoro-phenyl)methanone |
| **015** | (2-chloro-4-fluoro-phenyl)-[(1S,5R)-8-[5-(2,2-dimethylpropylsulfonyl)-2-methoxy-phenyl]-3,8-diazabicyclo[3.2.1]octan-3-yl]methanone |
| **016** | (2-chloro-4-fluoro-phenyl)-[rac-(3S)-4-[2-methoxy-5-[rac-(1S)-1-methylpropyl]sulfonyl-phenyl]-3-methyl-piperazin-1-yl]methanone |
| **017** | [4-fluoro-2-(trifluoromethyl)phenyl]-[rac-(3S)-4-[2-methoxy-5-[rac-(1S)-1-methylpropyl]sulfonyl-phenyl]-3-methyl-piperazin-1-yl]methanone |
| **018** | [4-fluoro-2-(trifluoromethyl)phenyl]-[4-(5-isobutylsulfonyl-2-methoxy-phenyl)piperazin-1-yl]methanone |
| **019** | (2-chloro-4-fluoro-phenyl)-[(1S,5R)-8-[5-(2-cyclopentylethylsulfonyl)-2-methoxy-phenyl]-3,8-diazabicyclo[3.2.1]octan-3-yl]methanone |
| **020** | [(1S,5R)-8-(5-butylsulfonyl-2-methoxy-phenyl)-3,8-diazabicyclo[3.2.1]octan-3-yl]-(2-chloro-4-fluoro-phenyl)methanone |
| **021** | (2-chloro-4-fluoro-phenyl)-[(1S,5R)-8-[5-(2-ethylbutylsulfonyl)-2-methoxy-phenyl]-3,8-diazabicyclo[3.2.1]octan-3-yl]methanone |
| **022** | (2-chloro-4-fluoro-phenyl)-[(1S,5R)-8-[2-methoxy-5-[(1SR)-1-methylpropyl]sulfonyl-phenyl]-3,8-diazabicyclo[3.2.1]octan-3-yl]methanone |
| **023** | (2-chloro-4-fluoro-phenyl)-[(1S,5R)-8-[2-methoxy-5-[(1SR)-1-methyl-3-phenyl-propyl]sulfonyl-phenyl]-3,8-diazabicyclo[3.2.1]octan-3-yl]methanone |
| **024** | (2-chloro-4-fluoro-phenyl)-[(1S,5R)-8-[2-methoxy-5-[(1SR)-1-methyl-4-phenyl-butyl]sulfonyl-phenyl]-3,8-diazabicyclo[3.2.1]octan-3-yl]methanone |
| **025** | (2-chloro-4-fluoro-phenyl)-[(1S,5R)-8-[5-[(1SR)-1-ethyl-3-phenyl-propyl]sulfonyl-2-methoxy-phenyl]-3,8-diazabicyclo[3.2.1]octan-3-yl]methanone |
| **026** | (2-chloro-4-fluoro-phenyl)-[(1S,5R)-8-[5-[(1SR)-1-ethyl-4-phenyl-butyl]sulfonyl-2-methoxy-phenyl]-3,8-diazabicyclo[3.2.1]octan-3-yl]methanone |
| **027** | (2-chloro-4-fluoro-phenyl)-[(1S,5R)-8-[2-methoxy-5-[(1-phenyl-4-piperidyl)sulfonyl]phenyl]-3,8-diazabicyclo[3.2.1]octan-3-yl]methanone |
| **028** | (2-chloro-4-fluoro-phenyl)-[(1S,5R)-8-[2-methoxy-5-[[1-(2-phenylethyl)-4-piperidyl]sulfonyl]phenyl]-3,8-diazabicyclo[3.2.1]octan-3-yl]methanone |
| **029** | (2-chloro-4-fluoro-phenyl)-[(1S,5R)-8-[5-[[1-[2-(4-fluorophenyl)ethyl]-4-piperidyl]sulfonyl]-2-methoxy-phenyl]-3,8-diazabicyclo[3.2.1]octan-3-yl]methanone |
| **030** | (2-chloro-4-fluoro-phenyl)-[(1S,5R)-8-[2-hydroxy-5-[(1-phenyl-4-piperidyl)sulfonyl]phenyl]-3,8-diazabicyclo[3.2.1]octan-3-yl]methanone |
| **031** | (2-chloro-4-fluoro-phenyl)-[(1S,5R)-8-[2-hydroxy-5-[[1-(2-phenylethyl)-4-piperidyl]sulfonyl]phenyl]-3,8-diazabicyclo[3.2.1]octan-3-yl]methanone |
| **032** | (2-chloro-4-fluoro-phenyl)-[(1S,5R)-8-[5-(2,2-dimethylpropylsulfonyl)-2-hydroxy-3-methyl-phenyl]-3,8-diazabicyclo[3.2.1]octan-3-yl]methanone |
| **033** | [(1S,5R)-8-[3-chloro-5-(2,2-dimethylpropylsulfonyl)-2-hydroxyphenyl]-3,8-diazabicyclo[3.2.1]octan-3-yl]-(2-chloro-4-fluoro-phenyl)methanone |
| **034** | [(1S,5R)-8-[3-chloro-5-(2,2-dimethylpropylsulfonyl)-2-(2-hydroxyethoxy)phenyl]-3,8-diazabicyclo[3.2.1]octan-3-yl]-(2-chloro-4-fluoro-phenyl)methanone |
| **035** | (2-chloro-4-fluoro-phenyl)-[(1S,5R)-8-[2,3-dichloro-5-(2,2-dimethylpropylsulfonyl)phenyl]-3,8-diazabicyclo[3.2.1]octan-3-yl]methanone |
| **036** | [(1S,5R)-8-[3-chloro-5-(2,2-dimethylpropylsulfonyl)-2-methyl-phenyl]-3,8-diazabicyclo[3.2.1]octan-3-yl]-(2-chloro-4-fluoro-phenyl)methanone |
and pharmaceutically acceptable salts and/or solvates thereof.

12. Pharmaceutical composition comprising a compound according to any one of claims **1** to **11** and at least one pharmaceutically acceptable carrier.

13. The compound according to any one of claims **1** to **11** or the pharmaceutical composition according to claim **12** for use as a medicament.

14. The compound or the pharmaceutical composition according to claim **13** for use in the treatment of a neurological disorder.

15. Process for manufacturing a compound according to any one of claims **1** to **11,** wherein said process comprises a step of reaction of:
a compound of formula (II) 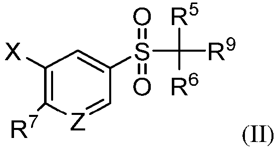 wherein
**Z, R⁵, R⁶, R⁷** and R⁹ are as defined in claim 1, and
**X** represents halide or -CF₃SO₃,
with a compound of formula (III) 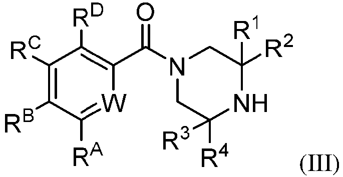 wherein **W, R^{A}-R^{D}** and **R¹-R⁴** are as defined in claim **1,**
in presence of a base and a metal catalyst;
thereby obtaining the compound of formula (I) or the pharmaceutically acceptable salt and/or solvate thereof.

## Patentansprüche

1. Verbindung der Formel (I) 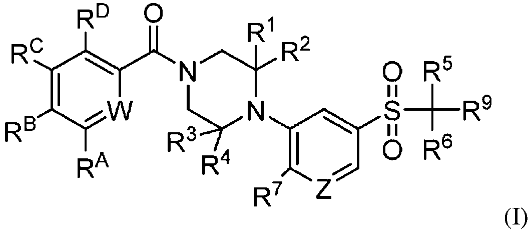 oder ein pharmazeutisch akzeptables Salz und/oder Solvat davon;
wobei
**W** CH oder N darstellt;
**R^{A}, R^{B}, R^{C}** und **R^{D}** jeweils unabhängig Wasserstoff, F, Cl, CH₃, CF₃, CHF₂ oder CH₂F darstellen,
*vorausgesetzt, dass* mindestens einer aus R^{A}, R^{B}, R^{C} und R^{D} keinen Wasserstoff darstellt;
**R¹** Wasserstoff oder (C₁-C₈) Alkyl darstellt, wobei das Alkyl optional durch mindestens ein OH-, (C₁-C₃)-Alkoxy oder F substituiert ist; und **R², R³** und **R⁴** Wasserstoff darstellen;
oder **R¹** und **R⁴** zusammen -CH₂-O-CH₂- oder -CH₂-CH₂- bilden, wobei das -CH₂-CH₂- optional durch mindestens ein F, OH oder OCH₃ substituiert ist; und **R²** und **R³** jeweils Wasserstoff darstellen;
**R⁷** Wasserstoff, OH, Halogen, (C₁-C₈) Alkyl, Cycloalkyl, (C₁-C₈) Alkyl-O-, Cycloalkyl-O-, Cycloalkyl-(C₁-C₈) Alkyl-O-, Heterocycloalkyl-O-, R¹¹O-(C₁-C₈) Alkyl-O-, R¹¹R¹²N-(C₁-C₈) Alkyl-O-, (R¹¹O)(R¹²)N-(C₁-C₈) Alkyl-O-, R¹¹R¹²N-(C₁-C₈) Alkyl-, R¹¹O-(C₁-C₈) Alkyl-, NR¹¹R¹², CN, CO₂H, CO₂R¹¹, CONH₂, CON(R¹¹)H, Heterocycloalkyl, oder Heteroaryl darstellt;
wobei **R¹¹** und **R¹²** jeweils unabhängig Wasserstoff oder (C₁-C₈) Alkyl darstellen;
wobei das Alkyl oder Cycloalkyl in **R⁷** optional durch mindestens ein F, Cl, OH, =O, (C₁-C₈) Alkyl, (C₁-C₈) Alkyl-O-, Heterocycloalkyl, Aryl oder Heteroaryl substituiert ist;
wobei das Heterocycloalkyl, Aryl oder Heteroaryl optional durch mindestens ein F, Cl, (C₁-C₈) Alkyl, CF₃, CHF₂, CH₂F, OCF₃, CN, OH, =O, →O, (C₁-C₈) Alkoxy, NR¹³R¹⁴, R¹³R¹⁴N-(C₁-C₈) Alkyl-, R¹³O₂C(C₁-C₈) Alkyl-, CO₂H, R¹³R¹⁴N-C(O)-, R¹³O-NR¹⁴-, oder (C₁-C₈) Alkyl-CO₂- substituiert ist; wobei **R¹³** und **R¹⁴** jeweils unabhängig Wasserstoff oder (C₁-C₈) Alkyl darstellen;
Z C-H, C-**R⁸** oder N darstellt;
wobei **R⁸** (C₁-C₄) Alkyl, F, Cl, CF₃, CHF₂, CH₂F, OCF₃, CN, OH oder (C₁-C₄) Alkoxy darstellt;
oder **Z** C-**R⁸** darstellt und **R⁷** und **R⁸** zusammen mit den Kohlenstoffatomen, an die sie gebunden sind, ein Cycloalkyl- oder Heterocycloalkyl bilden,
wobei das Cycloalkyl oder Heterocycloalkyl optional durch mindestens ein F, OH, =O, →O, (C₁-C₈) Alkyl, CF₃, HO₂C-CH₂-, (C₁-C₄) Alkyl-CO₂-CH₂-, R¹⁵R¹⁶N-CH₂-, Aryl oder Aryl-(C₁-C₈) Alkyl- substituiert ist, wobei **R¹⁵** und **R¹⁶** jeweils unabhängig Wasserstoff oder (C₁-C₈) Alkyl darstellen;
**R⁵** Wasserstoff, (C₁-C₈) Alkyl, durch ein bis drei (C₁-C₈) Alkylgruppen substituiertes CH₃, Cycloalkyl, Heterocycloalkyl, Aryl oder Cycloalkyl-(C₁-C₈) Alkyl darstellt;
wobei das Alkyl oder Cycloalkyl in **R⁵** optional durch mindestens ein F, Cl, (C₁-C₈) Alkyl, CF₃, OCF₃, CN, O^{H}, =O, (C₁-C₈) Alkoxy, NR¹⁷R¹⁸, CO₂H, R¹⁷R¹⁸N-C(O)-, R¹⁷O-NR¹⁸-, Heterocycloalkyl, Aryl oder Heteroaryl substituiert ist; wobei **R¹⁷** und **R¹⁸** jeweils unabhängig Wasserstoff oder (C₁-C₈) Alkyl darstellen;
wobei das Heterocycloalkyl, Aryl oder Heteroaryl optional durch mindestens ein F, Cl, (C₁-C₈) Alkyl, CF₃, CHF₂, CH₂F, OCF₃, CN, OH, =O, →O, (C₁-C₈) Alkoxy, NR¹⁹R²⁰, CO₂H, R¹⁹R²⁰N-C(O)-, R¹⁹O-NR²⁰-, (C₁-C₈) Alkyl-CO₂-, R¹⁹R²⁰N-(C₁-C₈) Alkyl-, R¹⁹O₂C-(C₁-C₈) Alkyl-, Heterocycloalkyl, Heteroaryl, Aryl oder Aryl-(C₁-C₈) Alkyl- substituiert ist; wobei **R¹⁹** und **R²⁰** jeweils unabhängig voneinander Wasserstoff oder (C₁-C₈) Alkyl darstellen;
**R⁶** Wasserstoff, (C₁-C₈) Alkyl, durch ein bis drei (C₁-C₈) Alkylgruppen substituiertes CH₃, Cycloalkyl, Heterocycloalkyl, Aryl oder Cycloalkyl-(C₁-C₈) Alkyl darstellt;
wobei das Alkyl oder Cycloalkyl in **R⁶** optional durch mindestens ein F, Cl, (C₁-C₈) Alkyl, CF₃, OCF₃, CN, OH, =O, (C₁-C₈) Alkoxy, NR²¹R²², CO₂H, R²¹R²²N-C(O)-, R²¹O-NR²²-, Heterocycloalkyl, Aryl oder Heteroaryl substituiert ist; wobei **R²¹** und **R²²** jeweils unabhängig Wasserstoff oder (C₁-C₈) Alkyl darstellen;
wobei das Heterocycloalkyl, Aryl oder Heteroaryl optional durch mindestens ein F, Cl, (C₁-C₈) Alkyl, CF₃, CHF₂, CH₂F, OCF₃, CN, OH,=O, →O, (C₁-C₈) Alkoxy, NR²³R²⁴, CO₂H, R²³R²⁴N-C(O)-, R²³O-NR²⁴-, (C₁-C₈) Alkyl-CO₂-, R²³R²⁴N-(C₁-C₈) Alkyl-, R₂₃O₂C-(C₁-C₈) Alkyl-, Heterocycloalkyl, Heteroaryl, Aryl oder Aryl-(C₁-C₈) Alkyl- substituiert ist; wobei **R²³** und **R²⁴** jeweils unabhängig Wasserstoff oder (C₁-C₈) Alkyl darstellen;
oder **R⁵** und **R⁶** zusammen mit dem Kohlenstoffatom, an das sie gebunden sind, ein Cycloalkyl- oder Heterocycloalkyl bilden,
wobei das Cycloalkyl oder Heterocycloalkyl optional durch mindestens ein F, Cl, (C₁-C₈) Alkyl, CF₃, OCF₃, CN, OH, =O, →O, (C₁-C₈) Alkoxy, NR²⁵R²⁶, CO₂H, (C₁-C₈) Alkyl-CO₂-, R²⁵R²⁶N-C(O)-, R²⁵O-NR²⁶-,
R²⁵R²⁶N-(C₁-C₈) Alkyl-, R²⁵O₂C-(C₁-C₈) Alkyl-, Cycloalkyl, Heterocycloalkyl, Aryl, Heteroaryl, Cycloalkyl-(C₁-C₈) Alkyl-, Heterocycloalkyl-(C₁-C₈) Alkyl-, Aryl-(C₁-C₈) Alkyl-, Heteroaryl-(C₁-C₈) Alkyl-, Aryl-Cycloalkyl-, Cycloalkyl-O-, Heterocycloalkyl-O-, Aryl-O-, Heteroaryl-O-, Cycloalkyl-(C₁-C₈) Alkyl-O-, Heterocycloalkyl-(C₁-C₈) Alkyl-O-, Aryl-(C₁-C₈) Alkyl-O-, Heteroaryl-(C₁-C₈) Alkyl-O-, Cycloalkyl-NR²⁵-, Heterocycloalkyl-NR²⁵-, Aryl-NR²⁵-, Heteroaryl-NR²⁵-, Cycloalkyl-(C₁-C₈) Alkyl-NR²⁵-, Heterocycloalkyl-(C₁-C₈) Alkyl-NR²⁵-, Aryl-(C1-C8) Alkyl-NR²⁵-, Heteroaryl-(C₁-C₈) Alkyl-NR²⁵-, Benzyliden, Heteroaryliden, Aryl-(C₁-C₈) Alkyl-yliden- oder Heteroaryl-(C₁-C₈) Alkyl-ylidensubstituiert ist;
wobei **R²⁵** und **R²⁶** jeweils unabhängig Wasserstoff oder (C₁-C₈) Alkyl darstellen;
wobei das Heterocycloalkyl, Aryl, Heteroaryl, Benzyliden oder Heteroaryliden optional durch mindestens ein F, Cl, (C₁-C₈) Alkyl, CF₃, CHF₂, CH₂F, OCF₃, CN, OH, =O, →O, (C₁-C₈) Alkoxy, NR²⁷R²⁸, CO₂H, R²⁷R²⁸N-C(O)-, R²⁷O-NR²⁸-, (C₁-C₈) Alkyl-CO₂-, R²⁷R²⁵N-(C₁-C₈) Alkyl-, R²⁷O₂C-(C₁-C₈) Alkyl-, Heterocycloalkyl, Heteroaryl, Aryl oder Aryl-(C₁-C₈) Alkyl- substituiert ist; wobei **R²⁷** und **R²⁸** jeweils unabhängig Wasserstoff oder (C₁-C₈) Alkyl darstellen;
**R⁹** Wasserstoff, (C₁-C₈) Alkyl, durch ein bis drei (C₁-C₈) Alkylgruppen substituiertes CH₃, Cycloalkyl, Heterocycloalkyl, Aryl oder Cycloalkyl-(C₁-C₈) Alkyl darstellt;
wobei das Alkyl oder Cycloalkyl in **R⁹** optional durch mindestens ein F, Cl, (C₁-C₈) Alkyl, CF₃, OCF₃, CN, OH, =O, (C₁-C₈)-Alkoxy, NR²⁹R³⁰, CO₂H, R²⁹R³⁰N-C(O)-, R²⁹O-NR³⁰-, Heterocycloalkyl, Aryl oder Heteroaryl substituert ist; wobei **R²⁹** und **R³⁰** jeweils unabhängig voneinander Wasserstoff oder (C₁-C₈) Alkyl darstellen;
wobei das Heterocycloalkyl, Aryl oder Heteroaryl optional durch mindestens ein F, Cl, (C₁-C₈) Alkyl, CF₃, OCF₃, CN, OH, =O, →O, (C₁-C₈)-Alkoxy, NR³¹R³², CO₂H, R³¹R³²N-C(O)-, R³¹O-NR³²-, (C₁-C₈) Alkyl-CO₂-, R²³R²⁴N-(C₁-C₈) Alkyl-, R²³O₂C-(C₁-C₈) Alkyl-, Heterocycloalkyl, Heteroaryl, Aryl oder Aryl-(C₁-C₈) Alkyl- substituiert ist; wobei **R³¹** und **R³²** jeweils unabhängig Wasserstoff oder (C₁-C₈) Alkyl darstellen.

2. Verbindung nach Anspruch **1,** wobei mindestens eines von **R^{A}, R^{B}, R^{C}** und **R^{D}** Wasserstoff darstellt, vorzugsweise mindestens eines von **R^{A}** und **R^{C}** Wasserstoff darstellt, bevorzugter **R^{C}** Wasserstoff darstellt.

3. Verbindung nach Anspruch **1** oder Anspruch **2,** wobei mindestens eines von **R^{A}, R^{B}** und **R^{D}** F oder Cl darstellt, vorzugsweise mindestens eines von **R^{B}** und **R^{D}** F oder Cl darstellt; bevorzugter **R^{B}** und **R^{D}** jeweils unabhängig F oder Cl darstellen.

4. Verbindung nach einem der Ansprüche **1** bis **3,** wobei **R¹** und **R⁴** zusammen - CH₂-O-CH₂- oder -CH₂-CH₂ bilden, vorzugsweise **R¹** und **R⁴** zusammen -CH₂-CH₂- bilden; wobei das -CH₂-CH₂- optional durch mindestens ein F, OH oder OCH₃ substituiert ist.

5. Verbindung nach einem der Ansprüche **1** bis **4,** wobei **R¹** Methyl, Ethyl, CF₃ oder CH₃OCH₂- darstellt.

6. Verbindung nach einem der Ansprüche **1** bis **5,** wobei **R⁷** Wasserstoff, OH oder Halogen darstellt, wobei vorzugsweise **R⁷** OH oder Cl darstellt.

7. Verbindung nach einem der Ansprüche **1** bis **6,** wobei **R⁷** (C₁-C₈) Alkyl-O- oder Cycloalkyl-O- darstellt, wobei das Alkyl oder Cycloalkyl optional durch mindestens ein F, Cl, OH, (C₁-C₈) Alkoxy oder Aryl substituiert ist; vorzugsweise **R⁷** OCH₃ oder HO-CH₂-CH₂-O darstellt.

8. Verbindung nach einem der Ansprüche **1** bis **7,** wobei **R⁵** und **R⁶** jeweils unabhängig (C₁-C₈) Alkyl, Aryl-(C₁-C₈) Alkyl- darstellen und **R⁹** Wasserstoff darstellt, vorzugsweise **R⁵** und **R⁶** jeweils unabhängig Methyl, Ethyl, n-Propyl, 2-Phenylethyl- oder 3-Phenylpropyl- darstellen.

9. Verbindung nach einem der Ansprüche **1** bis **8,** wobei **R⁶** (C₁-C₈) Alkyl, Cycloalkyl-(C₁-C₈) Alkyl oder Aryl darstellt und **R⁵** und **R⁹** jeweils Wasserstoff darstellen, wobei **R⁶** vorzugsweise *i*-Propyl, *n*-Propyl, *tert-Butyl, i*-Pentyl, Cyclopropyl, Cyclobutyl, Cyclopentyl, Cyclopentyl-CH₂- oder Phenyl darstellt.

10. Verbindung nach einem der Ansprüche **1** bis **8,** wobei **R⁵** und **R⁶** zusammen mit dem Kohlenstoffatom, an das sie gebunden sind, ein Cycloalkyl oder Heterocycloalkyl bilden und **R⁹** Wasserstoff darstellen; vorzugsweise **R⁵** und **R⁶** zusammen mit dem Kohlenstoffatom, an das sie gebunden sind, ein Cyclobutyl, Cyclopentyl, Cyclohexyl, Oxetan, 1-Phenyl-4-piperidyl, 1-(2-Phenylethyl)-4-piperidyl oder 1-[2-(4-Fluorphenyl)ethyl]-4-piperidyl bilden.

11. Verbindung nach einem der Ansprüche **1** bis **10,** wobei die Verbindung ausgewählt ist aus:
| | |
|---|---|
| **001** | [4-[5-(1-Ethylpropylsulfonyl)-2-methoxy-phenyl]piperazin-1-yl]-[4-fluor-2-(trifluormethyl)phenyl]methanon |
| **002** | [4-(5-Cyclopentylsulfonyl-2-methoxy-phenyl)piperazin-1-yl]-[4-fluor-2-(trifluormethyl)phenyl]methanon |
| **003** | [4-(5-Cyclohexylsulfonyl-2-methoxy-phenyl)piperazin-1-yl]-[4-fluor-2-(trifluormethyl)phenyl]methanon |
| **004** | (2-Chlor-4-fluor-phenyl)-[(1S,5R)-8-(5-cyclopentylsulfonyl-2-methoxy-phenyl)-3,8-diazabicyclo [3.2.1 ]octan-3-yl]methanon |
| **005** | (2-Chlor-4-fluor-phenyl)-[(1S,5R)-8-[5-(1-ethylpropylsulfonyl)-2-methoxy-phenyl]-3,8-diazabicyclo[3.2.1]octan-3-yl]methanon |
| **006** | (2-Chlor-4-fluor-phenyl)-[rac-(3S)-4-(5-cyclopentylsulfonyl-2-methoxy-phenyl)-3-methyl-piperazin-1-yl]methanon |
| **007** | (2-Chlor-4-fluor-phenyl)-[rac-(3S)-4-[5-(1-ethylpropylsulfonyl)-2-methoxy-phenyl]-3-methyl-piperazin-1-yl]methanon |
| **008** | (2-Chlor-4-fluor-phenyl)-[4-(5-isopropylsulfonyl-2-methoxy-phenyl)piperazin-1-yl]methanon |
| **009** | (2-Chlor-4-fluor-phenyl)-[4-[5-(cyclopropylmethylsulfonyl)-2-methoxy-phenyl]piperazin-1-yl]methanon |
| **010** | (2-Chlor-4-fluor-phenyl)-[(1S,5R)-8-(5-Cyclobutylsulfonyl-2-methoxy-phenyl)-3,8-diazabicyclo[3.2.1]octan-3-yl]methanon |
| **011** | (2-Chlor-4-fluor-phenyl)-[4-[2-methoxy-5-(oxetan-3-ylsulfonyl)phenyl]piperazin-1-yl]methanon |
| **012** | (2-Chlor-4-fluor-phenyl)-[4-[5-(cyclobutylmethylsulfonyl)-2-methoxy-phenyl]piperazin-1-yl]methanon |
| **013** | (2-Chlor-4-fluor-phenyl)-[4-[5-(cyclopentylmethylsulfonyl)-2-methoxy-phenyl]piperazin-1-yl]methanon |
| **014** | [(1S,5R)-8-(5-Benzylsulfonyl-2-methoxy-phenyl)-3,8-diazabicyclo[3.2.1]octan-3-yl]-(2-chlor-4-fluor-phenyl)methanon |
| **015** | (2-Chlor-4-fluor-phenyl)-[(1S,5R)-8-[5-(2,2-dimethylpropylsulfonyl)-2-methoxy-phenyl]-3,8-diazabicyclo[3.2.1]octan-3-yl]methanon |
| **016** | (2-Chlor-4-fluor-phenyl)-[rac-(3S)-4-[2-methoxy-5-[rac-(1S)-1-methylpropy1]sulfony1-phenyl]-3-methy1-piperazin-1-yl]methanon |
| **017** | [4-Fluor-2-(trifluormethyl)phenyl]-[rac-(3S)-4-[2-methoxy-5-[rac-(1S)-1-methylpropyl]sulfonyl-phenyl]-3-methyl-piperazin-1-yl]methanon |
| **018** | [4-Fluor-2-(trifluormethyl)phenyl]-[4-(5-isobutylsulfonyl-2-methoxy-phenyl)piperazin-1-yl]methanon |
| **019** | (2-Chlor-4-fluor-phenyl)-[(1S,5R)-8-[5-(2-cyclopentylethylsulfonyl)-2-methoxy-phenyl]-3,8-diazabicyclo[3.2.1]octan-3-yl]methanon |
| **020** | [(1S,5R)-8-(5-Butylsulfonyl-2-methoxy-phenyl)-3,8-diazabicyclo[3.2.1]octan-3-yl]-(2-chlor-4-fluor-phenyl)methanon |
| **021** | (2-Chlor-4-fluor-phenyl)-[(1S,5R)-8-[5-(2-ethylbutylsulfonyl)-2-methoxy-phenyl]-3,8-diazabicyclo[3.2.1]octan-3-yl]methanon |
| **022** | (2-Chlor-4-fluor-phenyl)-[(1S,5R)-8-[2-methoxy-5-[(1SR)-1-methylpropyl]sulfonyl-phenyl]-3,8-diazabicyclo[3.2.1]octan-3-yl]methanon |
| **023** | (2-Chlor-4-fluor-phenyl)-[(1S,5R)-8-[2-methoxy-5-[(1SR)-1-methyl-3-phenyl-propyl]sulfonyl-phenyl]-3,8-diazabicyclo[3.2.1]octan-3-yl]methanon |
| **024** | (2-Chlor-4-fluor-phenyl)-[(1S,5R)-8-[2-methoxy-5-[(1SR)-1-methyl-4-phenyl-butyl]sulfonyl-phenyl]-3,8-diazabicyclo[3.2.1]octan-3-yl]methanon |
| **025** | (2-Chlor-4-fluor-phenyl)-[(1S,5R)-8-[5-[(1SR)-1-ethyl-3-phenyl-propyl]sulfonyl-2-methoxy-phenyl]-3,8-diazabicyclo[3.2.1]octan-3-yl]methanon |
| **026** | (2-Chlor-4-fluor-phenyl)-[(1S,5R)-8-[5-[(1SR)-1-ethyl-4-phenyl-butyl]sulfonyl-2-methoxy-phenyl]-3,8-diazabicyclo[3.2.1]octan-3-yl]methanon |
| **027** | (2-Chlor-4-fluor-phenyl)-[(1S,5R)-8-[2-methoxy-5-[(1-phenyl-4-piperidyl)sulfonyl]phenyl]-3,8-diazabicyclo[3.2.1]octan-3-yl]methanon |
| **028** | (2-Chlor-4-fluorphenyl)-[(1S,5R)-8-[2-methoxy-5-[[1-(2-phenylethyl) -4-piperidyl]sulfonyl]phenyl]-3,8-diazabicyclo[3.2.1]octan-3-yl]methanon |
| **029** | (2-Chlor-4-fluor-phenyl)-[(1S,5R)-8-[5-[[1-[2-(4-fluorphenyl)ethyl]-4-piperidyl]sulfonyl]-2-methoxy-phenyl]-3,8-diazabicyclo[3.2.1]octan-3-yl]methanon |
| **030** | (2-Chlor-4-fluor-phenyl)-[(1S,5R)-8-[2-hydroxy-5-[(1-phenyl-4-piperidyl)sulfonyl]phenyl]-3,8-diazabicyclo[3.2.1]octan-3-yl]methanon |
| **031** | (2-Chlor-4-fluor-phenyl)-[(1S,5R)-8-[2-hydroxy-5-[[1-(2-phenylethyl)-4-piperidyl]sulfonyl]phenyl]-3,8-diazabicyclo[3.2.1]octan-3-yl]methanon |
| **032** | (2-Chlor-4-fluor-phenyl)-[(1S,5R)-8-[5-(2,2-dimethylpropylsulfonyl)-2-hydroxy-3-methyl-phenyl]-3,8-diazabicyclo[3.2.1]octan-3-yl]methanon |
| **033** | [(1S,5R)-8-[3-Chlor-5-(2,2-dimethylpropylsulfonyl)-2-hydroxy-phenyl]-3,8-diazabicyclo[3.2.1]octan-3-yl]-(2-chlor-4-fluor-phenyl)methanon |
| **034** | [(1S,5R)-8-[3-Chlor-5-(2,2-dimethylpropylsulfonyl)-2-(2-hydroxyethoxy)phenyl]-3,8-diazabicyclo[3.2.1]octan-3-yl]-(2-chlor-4-fluor-phenyl)methanon |
| **035** | (2-Chlor-4-fluor-phenyl)-[(1S,5R)-8-[2,3-dichlor-5-(2,2-dimethylpropylsulfonyl)phenyl]-3,8-diazabicyclo[3.2.1]octan-3-yl]methanon |
| **036** | [(1S,5R)-8-[3-Chlor-5-(2,2-dimethylpropylsulfonyl)-2-methylphenyl]-3,8-diazabicyclo[3.2.1]octan-3-yl]-(2-chlor-4-fluor-phenyl)methanon |
und pharmazeutisch verträglichen Salzen und/oder Solvaten davon.

12. Pharmazeutische Zusammensetzung, umfassend eine Verbindung gemäß einem der Ansprüche **1** bis **11** und mindestens einen pharmazeutisch verträglichen Träger.

13. Verbindung nach einem der Ansprüche **1** bis **11** oder pharmazeutische Zusammensetzung nach Anspruch **12** zur Verwendung als Arzneimittel.

14. Verbindung oder pharmazeutische Zusammensetzung nach Anspruch **13** zur Verwendung bei der Behandlung einer neurologischen Störung.

15. Verfahren zur Herstellung einer Verbindung nach einem der Ansprüche **1** bis **11,** wobei das Verfahren einen Reaktionsschritt umfasst von:
einer Verbindung der Formel (II), 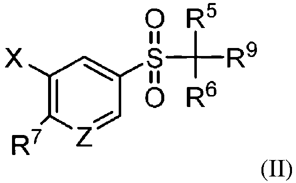 wobei
**Z, R⁵, R⁶, R⁷** und **R⁹** wie in Anspruch 1 definiert sind, und
**X** Halogen oder -CF₃SO₃ darstellt,
mit einer Verbindung der Formel (III), 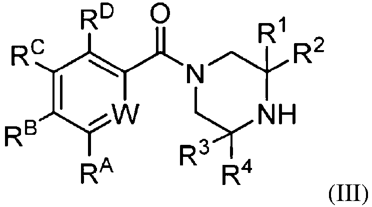 wobei **W, R^{A}-R^{D}** und **R¹-R⁴** wie in Anspruch 1 definiert sind,
in Gegenwart einer Basis und eines Metallkatalysators;
wodurch die Verbindung der Formel (I) oder ein pharmazeutisch verträgliches Salz und/oder Solvat davon erhalten wird.

## Revendications

1. Composé de formule (I) 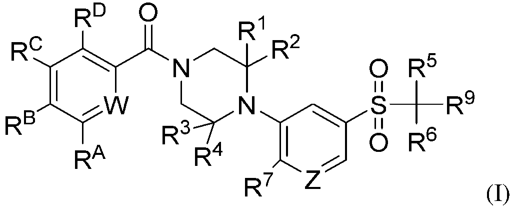 ou un sel et/ou un solvate pharmaceutiquement acceptable de celui-ci ;
dans lequel
**W** représente CH ou N ;
**R^{A}, R^{B}, R^{C}** et **R^{D}** chacun représente indépendamment un hydrogène, F, Cl, CH₃, CF₃, CHF₂ ou CH₂F,
*à condition* qu'au moins l'un parmi **R^{A}, R^{B}, R^{C}** et **R^{D}** ne représente pas un hydrogène ;
**R¹** représente un hydrogène ou un alkyle en C₁-C₈, dans lequel l'alkyle est optionnellement substitué par au moins un OH, un alcoxyle en C₁-C₃ ou F ; et **R², R³** et **R⁴** représentent un hydrogène ;
ou **R¹** et **R⁴** forment ensemble -CH₂-O-CH₂- ou -CH₂-CH₂-, dans lequel -CH₂-CH₂- est optionnellement substitué par au moins un F, OH ou OCH₃ ; et **R²** et **R³** représentent chacun un hydrogène ;
**R⁷** représente un hydrogène, OH, un halogène, un alkyle en C₁-C₈, un cycloalkyle, un (alkyle en C₁-C₈)-O-, un cycloalkyle-O-, un cycloalkyle(alkyle en C₁-C₈)-O-, un hétérocycloalkyle-O-, R¹¹O-(alkyle en C₁-C₈)-O-, R¹¹R¹²N(alkyle en C₁-C₈)-O-, (R¹¹O)(R¹²)N-(alkyle en C₁-C₈)-O-, R¹¹R¹²N-(alkyle en C₁-C₈)-, R¹¹O-(alkyle en C₁-C₈)-, NR¹¹R¹², CN, CO₂H, CO₂R¹¹, CONH₂, CON(R¹¹)H, un hétérocycloalkyle, ou un hétéroaryle; dans lequel **R¹¹** et **R¹²** représentent chacun indépendamment un hydrogène ou un alkyle en C₁-C₈ ;
dans lequel l'alkyle ou le cycloalkyle de **R⁷** est optionnellement substitué par au moins un F, Cl, OH, =O, un alkyle en C₁-C₈, un (alkyle en C₁-C₈)-O-, un hétérocycloalkyle, un aryle ou un hétéroaryle ;
dans lequel l'hétérocycloalkyle, l'aryle ou l'hétéroaryle est optionnellement substitué par au moins un F, Cl, un alkyle en C₁-C₈, CF₃, CHF₂, CH₂F, OCF₃, CN, OH, =O, →O, un alcoxyle en C₁-C₈, NR¹³R¹⁴, R¹³R¹⁴N-(alkyle en C₁-C₈)-, R¹³O₂C-(alkyle en C₁-C₈)-, CO₂H, R¹³R¹⁴N-C(O)-, R¹³O-NR¹⁴-, ou -(alkyle en C₁-C₈)-CO₂- ; dans lequel **R¹³** et **R¹⁴** représentent chacun indépendamment un hydrogène ou un alkyle en C₁-C₈ ;
**Z** représente C-H, C-**R⁸** ou N ;
dans lequel **R⁸** représente un alkyle en C₁-C₄, F, Cl, CF₃, CHF₂, CH₂F, OCF₃, CN, OH ou un alcoxyle en C₁-C₄ ;
ou **Z** représente C-**R⁸** et **R⁷** et **R⁸** forment, avec les atomes de carbone auxquels ils sont liés, un cycloalkyle ou un hétérocycloalkyle,
dans lequel le cycloalkyle ou l'hétérocycloalkyle est optionnellement substitué par au moins un F, OH, =O, →O, un alkyle en C₁-C₈, CF₃, HO₂C-CH₂-, (alkyle en C₁-C₄)-CO₂-CH₂-, R¹⁵R¹⁶N-CH₂-, un aryle, ou un aryle-(alkyle en C₁-C₈)-, dans lequel **R¹⁵** et **R¹⁶** représentent chacun indépendamment un hydrogène ou un alkyle en C₁-C₈ ;
**R⁵** représente un hydrogène, un alkyle en C₁-C₈, un CH₃ substitué par un à trois groupes alkyles en C₁-C₈, un cycloalkyle, un hétérocycloalkyle, un aryle, ou un cycloalkyle-(alkyle en C₁-C₈) ;
dans lequel l'alkyle ou le cycloalkyle de **R⁵** est optionnellement substitué par au moins un F, Cl, un alkyle en C₁-C₈, CF₃, OCF₃, CN, OH, =O, un alcoxyle en C₁-C₈, NR¹⁷R¹⁸, CO₂H, R¹⁷R¹⁸N-C(O)-, R¹⁷O-NR¹⁸-, un hétérocycloalkyle, un aryle ou un hétéroaryle ; dans lequel **R¹⁷** et **R¹⁸** représentent chacun indépendamment un hydrogène ou un alkyle en C₁-C₈ ;
dans lequel l'hétérocycloalkyle, l'aryle ou l'hétéroaryle est optionnellement substitué par au moins un F, Cl, un alkyle en C₁-C₈, CF₃, CHF₂, CH₂F, OCF₃, CN, OH, =O, →O, un alcoxyle en C₁-C₈, NR¹⁹R²⁰, CO₂H, R¹⁹R²⁰N-C(O)-, R¹⁹O-NR²⁰-, (alkyle en C₁-C₈)-CO₂-, R¹⁹R²⁰N-(alkyle en C₁-C₈)-, R¹⁹O₂C-(alkyle en C₁-C₈)-, un hétérocycloalkyle, un hétéroaryle, un aryle, ou un aryle-(alkyle en C₁-C₈)-; dans lequel **R¹⁹** et **R²⁰** représentent chacun indépendamment un hydrogène ou un alkyle en C₁-C₈ ;
**R⁶** représente un hydrogène, un alkyle en C₁-C₈, un CH₃ substitué par un à trois groupes alkyles en C₁-C₈, un cycloalkyle, un hétérocycloalkyle, un aryle, ou un cycloalkyle-(alkyle en C₁-C₈) ;
dans lequel l'alkyle ou le cycloalkyle de **R⁶** est optionnellement substitué par au moins un F, Cl, un alkyle en C₁-C₈, CF₃, OCF₃, CN, OH, =O, un alcoxyle en C₁-C₈, NR²¹R²², CO₂H, R²¹R²²N-C(O)-, R²¹O-NR²²-, un hétérocycloalkyle, un aryle ou un hétéroaryle ; dans lequel **R²¹** et **R²²** représentent chacun indépendamment un hydrogène ou un alkyle en C₁-C₈ ;
dans lequel l'hétérocycloalkyle, l'aryle ou l'hétéroaryle est optionnellement substitué par au moins un F, Cl, un alkyle en C₁-C₈, CF₃, CHF₂, CH₂F, OCF₃, CN, OH, =O, →O, un alcoxyle en C₁-C₈, NR²³R²⁴, CO₂H, R²³R²⁴N-C(O)-, R²³O-NR²⁴-, (alkyle en C₁-C₈)-CO₂-, R²³R²⁴N-(alkyle en C₁-C₈)-, R²³O₂C-(alkyle en C₁-C₈)-, un hétérocycloalkyle, un hétéroaryle, un aryle, ou un aryle-(alkyle en C₁-C₈)- ; dans lequel **R²³** et **R²⁴** représentent chacun indépendamment un hydrogène ou un alkyle en C₁-C₈ ;
ou **R⁵** et **R⁶** forment, avec les atomes de carbone auxquels ils sont liés, un cycloalkyle ou un hétérocycloalkyle
dans lequel le cycloalkyle ou l'hétérocycloalkyle est optionnellement substitué par au moins un F, Cl, un alkyle en C₁-C₈, CF₃, OCF₃, CN, OH, =O, →O, un alcoxyle en C₁-C₈, NR²⁵R²⁶, CO₂H, (alkyle en C₁-C₈)-CO₂-, R²⁵R²⁶N-C(O)-, R²⁵O-NR²⁶-, R²⁵R²⁶N-(alkyle en C₁-C₈)-, R²⁵O₂C-(alkyle en C₁-C₈)-, un cycloalkyle, un hétérocycloalkyle, un aryle, un hétéroaryle, un cycloalkyle-(alkyle en C₁-C₈)-, un hétérocycloalkyle-(alkyle en C₁-C₈)-, un aryle-(alkyle en C₁-C₈)-, un hétéroaryle-(alkyle en C₁-C₈)-, un aryle-cycloalkyle-, un cycloalkyle-O-, un hétérocycloalkyle-O-, un aryle-O-, un hétéroaryle-O-, un cycloalkyle-(alkyle en C₁-C₈)-O-, un hétérocycloalkyle-(alkyle en C₁-C₈)-O-, un aryle-(alkyle en C₁-C₈)-O-, un hétéroaryle-(alkyle en C₁-C₈)-O-, un cycloalkyle-NR²⁵-, un hétérocycloalkyle-NR²⁵-, un aryle-NR²⁵-, un hétéroaryle-NR²⁵-, un cycloalkyle-(alkyle en C₁-C₈)-NR²⁵-, un hétérocycloalkyle-(alkyle en C₁-C₈)-NR²⁵-, un aryle-(alkyle en C₁-C₈)-NR²⁵-, un hétéroaryle-(alkyle en C₁-C₈)-NR²⁵-, un benzylidène, un hétéroarylidène, un aryle-(alkyle en C₁-C₈)-ylidène- ou un hétéroaryle-(alkyle en C₁-C₈)-ylidène-;
dans lequel **R²⁵** et **R²⁶** représentent chacun indépendamment un hydrogène ou un alkyle en C₁-C₈ ;
dans lequel l'hétérocycloalkyle, l'aryle, l'hétéroaryle, le benzylidène ou l'hétéroarylidène est optionnellement substitué par au moins un F, Cl, un alkyle en C₁-C₈, CF₃, CHF₂, CH₂F, OCF₃, CN, OH, =O, →O, un alcoxyle en C₁-C₈, NR²⁷R²⁸, CO₂H, R²⁷R²⁸N-C(O)-, R²⁷O-NR²⁸-, (alkyle en C₁-C₈)-CO₂-, R²⁷R²⁸N-(alkyle en C₁-C₈)-, R²⁷O₂C-(alkyle en C₁-C₈)-, un hétérocycloalkyle, un hétéroaryle, un aryle, ou un aryle-(alkyle en C₁-C₈)-; dans lequel **R²⁷** et **R²⁸** représentent chacun indépendamment un hydrogène ou un alkyle en C₁-C₈ ;
**R⁹** représente un hydrogène, un alkyle en C₁-C₈, un CH₃ substitué par un à trois groupes alkyles en C₁-C₈, un cycloalkyle, un hétérocycloalkyle, un aryle, ou un cycloalkyle-(alkyle en C₁-C₈) ;
dans lequel l'alkyle ou le cycloalkyle de **R⁹** est optionnellement substitué par au moins un F, Cl, (C₁-C₈) alkyl, CF₃, OCF₃, CN, OH, =O, un alcoxyle en C₁-C₈, NR²⁹R³⁰, CO₂H, R²⁹R³⁰N-C(O)-, R²⁹O-NR³⁰-, un hétérocycloalkyle, un aryle ou un hétéroaryle ; dans lequel **R²⁹** et **R³⁰** représentent chacun indépendamment un hydrogène ou un alkyle en C₁-C₈ ;
dans lequel l'hétérocycloalkyle, l'aryle ou l'hétéroaryle est optionnellement substitué par au moins un F, Cl, un alkyle en C₁-C₈, CF₃, OCF₃, CN, OH, =O, →O, un alcoxyle en C₁-C₈, NR³¹R³², CO₂H, R³¹R³²N-C(O)-, R³¹O-NR³²-, (alkyle en C₁-C₈)-CO₂-, R²³R²⁴N-(alkyle en C₁-C₈)-, R²³O₂C-(alkyle en C₁-C₈)-, un hétérocycloalkyle, un hétéroaryle, un aryle, ou un aryle-(alkyle en C₁-C₈)- ; dans lequel **R³¹** et **R³²** représentent chacun indépendamment un hydrogène ou un alkyle en C₁-C₈.

2. Le composé selon la revendication **1,** dans lequel au moins l'un parmi **R^{A}, R^{B}, R^{C}** et **R^{D}** représente un hydrogène, de préférence au moins l'un parmi **R^{A}** et **R^{C}** représente un hydrogène, plus préférentiellement **R^{C}** représente un hydrogène.

3. Le composé selon la revendication **1** ou la revendication **2,** dans lequel au moins l'un parmi **R^{A}, R^{B}** et **R^{D}** représente F ou Cl, de préférence au moins l'un parmi **R^{B}** et **R^{D}** représente F ou Cl ; plus préférentiellement **R^{B}** et **R^{D}** représentent chacun indépendamment F ou Cl.

4. Le composé selon l'une quelconque des revendications **1** à **3,** dans lequel **R¹** et **R⁴** forment ensemble -CH₂-O-CH₂- ou -CH₂-CH₂, de préférence **R¹** et **R⁴** forment ensemble -CH₂-CH₂- ; dans lequel le -CH₂-CH₂- est optionnellement substitué par au moins un F, OH ou OCH₃.

5. Le composé selon l'une quelconque des revendications **1** à **4,** dans lequel **R¹** représente un méthyle, un éthyle, CF₃ ou CH₃OCH₂-.

6. Le composé selon l'une quelconque des revendications **1** à **5,** dans lequel **R⁷** représente un hydrogène, OH ou un halogène, de préférence **R⁷** représente OH ou Cl.

7. Le composé selon l'une quelconque des revendications **1** à **6,** dans lequel **R⁷** représente (alkyle en C₁-C₈)-O- ou un cycloalkyle-O-, dans lequel l'alkyle ou le cycloalkyle est optionnellement substitué par au moins un F, Cl, OH, un alcoxyle en C₁-C₈ ou un aryle ; de préférence **R⁷** représente OCH₃ ou HO-CH₂-CH₂-O.

8. Le composé selon l'une quelconque des revendications **1** à **7,** dans lequel **R⁵** et **R⁶** représentent chacun indépendamment un alkyle en C₁-C₈, aryle-(alkyle en C₁-C₈)-, et **R⁹** représente un hydrogène, de préférence **R⁵** et **R⁶** représentent chacun indépendamment un méthyle, un éthyle, n-propyle, 2-phényléthyle- ou 3-phénylpropyle-.

9. Le composé selon l'une quelconque des revendications **1** à **8,** dans lequel **R⁶** représente un alkyle en C₁-C₈, un cycloalkyle-(alkyle en C₁-C₈) ou un aryle et **R⁵** et **R⁹** représentent chacun un hydrogène, de préférence **R⁶** représente i-propyle, *n*-propyle, tert-butyle, i-pentyle, cyclopropyle, cyclobutyle, cyclopentyle, cyclopentyle-CH₂- ou un phényle.

10. Le composé selon l'une quelconque des revendications **1** à **8,** dans lequel **R⁵** et **R⁶** forment, avec les atomes de carbone auxquels ils sont liés, un cycloalkyle ou hétérocycloalkyle et **R⁹** représente un hydrogène ; de préférence **R⁵** et **R⁶** forment, avec les atomes de carbone auxquels ils sont liés, un cyclobutyle, cyclopentyle, cyclohexyle, oxétane, 1-phényl-4-piperidyle, 1-(2-phényléthyl)-4-piperidyle ou 1-[2-(4-fluorophényl)éthyl]-4-piperidyle.

11. Le composé selon l'une quelconque des revendications **1** à **10,** dans lequel ledit composé est choisi parmi :
| | |
|---|---|
| **001** | [4-[5-(1-éthylpropylsulfonyl)-2-méthoxy-phényl]pipérazin-1-yl]-[4-fluoro-2-(trifluoromethyl)phenyl]methanone |
| **002** | [4-(5-cyclopentylsulfonyl-2-méthoxy-phényl)pipérazin-1-yl]-[4-fluoro-2-(trifluoromethyl)phenyl]methanone |
| **003** | [4-(5-cyclohexylsulfonyl-2-méthoxy-phényl)pipérazin-1-yl]-[4-fluoro-2-(trifluoromethyl)phenyl]methanone |
| **004** | (2-chloro-4-fluoro-phenyl)-[(1S,5R)-8-(5-cyclopentylsulfonyl-2-methoxy-phenyl)-3,8-diazabicyclo[3.2.1]octan-3-yl]methanone |
| **005** | (2-chloro-4-fluoro-phenyl)-[(1S,5R)-8-[5-(1-ethylpropylsulfonyl)-2-methoxy-phenyl]-3,8-diazabicyclo[3.2.1]octan-3-yl]methanone |
| **006** | (2-chloro-4-fluoro-phenyl)-[rac-(3S)-4-(5-cyclopentylsulfonyl-2-méthoxy-phényl)-3-méthyl-pipérazin-1-yl]méthanone |
| **007** | (2-chloro-4-fluoro-phényl)-[rac-(3S)-4-[5-(1-éthylpropylsulfonyl)-2-méthoxy-phényl]-3-méthyl-pipérazin-1-yl]méthanone |
| **008** | (2-chloro-4-fluoro-phényl)-[4-(5-isopropylsulfonyl-2-méthoxy-phényl)pipérazin-1-yl]méthanone |
| **009** | (2-chloro-4-fluoro-phényl)-[4-[5-(cyclopropylméthylsulfonyl)-2-méthoxy-phényl]pipérazin-1-yl]méthanone |
| **010** | (2-chloro-4-fluoro-phényl)-[(1S,5R)-8-(5-cyclobutylsulfonyl-2-methoxy-phenyl)-3,8-diazabicyclo[3.2.1]octan-3-yl]methanone |
| **011** | (2-chloro-4-fluoro-phényl)-[4-[2-méthoxy-5-(oxetan-3-ylsulfonyl)phényl]pipérazin-1-yl]méthanone |
| **012** | (2-chloro-4-fluoro-phényl)-[4-[5-(cyclobutylméthylsulfonyl)-2-méthoxy-phényl]pipérazin-1-yl]méthanone |
| **013** | (2-chloro-4-fluoro-phényl)-[4-[5-(cyclopentylméthylsulfonyl)-2-méthoxy-phényl]pipérazin-1-yl]méthanone |
| **014** | [(1S,5R)-8-(5-benzylsulfonyl-2-méthoxy-phényl)-3,8-diazabicyclo[3.2.1]octan-3-yl]-(2-chloro-4-fluoro-phényl)méthanone |
| **015** | (2-chloro-4-fluoro-phényl)-[(1S,5R)-8-[5-(2,2-diméthylpropylsulfonyl)-2-méthoxy-phényl]-3,8-diazabicyclo[3.2.1]octan-3-yl]méthanone |
| **016** | (2-chloro-4-fluoro-phényl)-[rac-(3S)-4-[2-méthoxy-5-[rac-(1S)-1-méthylpropyl]sulfonyl-phényl]-3-méthyl-pipérazin-1-yl]méthanone |
| **017** | [4-fluoro-2-(trifluorométhyl)phényl]-[rac-(3S)-4-[2-méthoxy-5-[rac-(1S)-1-méthylpropyl]sulfonyl-phényl]-3-méthyl-pipérazin-1-yl]méthanone |
| **018** | [4-fluoro-2-(trifluorométhyl)phényl]-[4-(5-isobutylsulfonyl-2-méthoxy-phényl)pipérazin-1-yl]méthanone |
| **019** | (2-chloro-4-fluoro-phényl)-[(1S,5R)-8-[5-(2-cyclopentylethylsulfonyl)-2-methoxy-phenyl]-3,8-diazabicyclo[3.2.1]octan-3-yl]méthanone |
| **020** | [(1S,5R)-8-(5-butylsulfonyl-2-méthoxy-phényl)-3,8-diazabicyclo[3.2.1]octan-3-yl]-(2-chloro-4-fluoro-phényl)méthanone |
| **021** | (2-chloro-4-fluoro-phényl)-[(1S,5R)-8-[5-(2-éthylbutylsulfonyl)-2-methoxy-phenyl]-3,8-diazabicyclo[3.2.1]octan-3-yl]methanone |
| **022** | (2-chloro-4-fluoro-phenyl)-[(1S,5R)-8-[2-methoxy-5-[(1SR)-1-methylpropyl]sulfonyl-phenyl]-3,8-diazabicyclo[3.2.1]octan-3-yl]méthanone |
| **023** | (2-chloro-4-fluoro-phenyl)-[(1S,SR)-8-[2-methoxy-5-[(1SR)-1-methyl-3-phenyl-propyl]sulfonyl-phenyl]-3,8-diazabicyclo[3.2.1]octan-3-yl]méthanone |
| **024** | (2-chloro-4-fluoro-phényl)-[(1S,5R)-8-[2-méthoxy-5-[(1SR)-1-méthyl-4-phényl-butyl]sulfonyl-phényl]-3,8-diazabicyclo[3.2.1]octan-3-yl]méthanone |
| **025** | (2-chloro-4-fluoro-phényl)-[(1S,5R)-8-[5-[(1SR)-1-éthyl-3-phenyl-propyl]sulfonyl-2-methoxy-phenyl]-3,8-diazabicyclo[3.2.1]octan-3-yl]méthanone |
| **026** | (2-chloro-4-fluoro-phényl)-[(1S,5R)-8-[5-[(1SR)-1-éthyl-4-phényl-butyl]sulfonyl-2-méthoxy-phényl]-3,8-diazabicyclo[3.2.1]octan-3-yl]méthanone |
| **027** | (2-chloro-4-fluoro-phényl)-[(1S,5R)-8-[2-méthoxy-5-[(1-phényl-4-piperidyl)sulfonyl]phenyl]-3,8-diazabicyclo[3.2.1]octan-3-yl]méthanone |
| **028** | (2-chloro-4-fluoro-phényl)-[(1S,5R)-8-[2-méthoxy-5-[[1-(2-phényléthyl)-4-piperidyl]sulfonyl]phényl]-3,8-diazabicyclo[3.2.1]octan-3-yl]méthanone |
| **029** | (2-chloro-4-fluoro-phényl)-[(1S,5R)-8-[5-[[1-[2-(4-fluorophenyl)ethyl]-4-piperidyl]sulfonyl]-2-methoxy-phenyl]-3,8-diazabicyclo[3.2.1]octan-3-yl]méthanone |
| **030** | (2-chloro-4-fluoro-phényl)-[(1S,5R)-8-[2-hydroxy-5-[(1-phényl-4-piperidyl)sulfonyl]phenyl]-3,8-diazabicyclo[3.2.1]octan-3-yl]méthanone |
| **031** | (2-chloro-4-fluoro-phényl)-[(1S,5R)-8-[2-hydroxy-5-[[1-(2-phényléthyl)-4-piperidyl]sulfonyl]phényl]-3,8-diazabicyclo[3.2.1]octan-3-yl]méthanone |
| **032** | (2-chloro-4-fluoro-phényl)-[(1S,5R)-8-[5-(2,2-dimethylpropylsulfonyl)-2-hydroxy-3-methyl-phenyl]-3,8-diazabicyclo[3.2.1]octan-3-yl]méthanone |
| **033** | [(1S,5R)-8-[3-chloro-5-(2,2-diméthylpropylsulfonyl)-2-hydroxy-phényl]-3,8-diazabicyclo[3.2.1]octan-3-yl]-(2-chloro-4-fluoro-phényl)méthanone |
| **034** | [(1S,5R)-8-[3-chloro-5-(2,2-diméthylpropylsulfonyl)-2-(2-hydroxyethoxy)phenyl]-3,8-diazabicyclo[3.2.1]octan-3-yl]-(2-chloro-4-fluoro-phenyl)methanone |
| **035** | (2-chloro-4-fluoro-phényl)-[(1S,5R)-8-[2,3-dichloro-5-(2,2-dimethylpropylsulfonyl)phenyl]-3,8-diazabicyclo[3.2.1]octan-3-yl]méthanone |
| **036** | [(1S,5R)-8-[3-chloro-5-(2,2-diméthylpropylsulfonyl)-2-méthyl-phényl]-3,8-diazabicyclo[3.2.1]octan-3-yl]-(2-chloro-4-fluoro-phényl)méthanone |
et leurs sels et/ou solvates pharmaceutiquement acceptables.

12. Composition pharmaceutique comprenant un composé selon l'une quelconque des revendications 1 à 11 et au moins un excipient pharmaceutiquement acceptable.

13. Le composé selon l'une quelconque des revendications 1 à 11 ou la composition pharmaceutique selon la revendication 12, pour utilisation en tant que médicament.

14. Le composé ou la composition pharmaceutique selon la revendication 13, pour utilisation dans le traitement d'un trouble neurologique.

15. Procédé de fabrication d'un composé selon l'une quelconque des revendications 1 à 11, dans lequel ledit procédé comprend une étape de réaction :
d'un composé de formule (II) 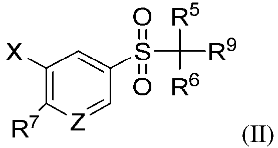 dans lequel
Z, R⁵, R⁶, R⁷ et R⁹ sont tels que définis dans la revendication 1, et
X représente un halogénure ou -CF₃SO₃,
avec un composé de formule (III) 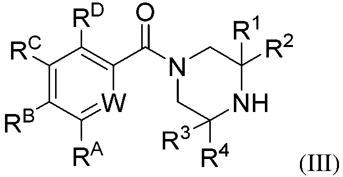 dans lequel W, R^{A}-R^{D} et R¹-R⁴ sont tels que définis dans la revendication 1, en présence d'une base et d'un catalyseur métallique ;
permettant ainsi d'obtenir le composé de formule (I) ou son sel et/ou son solvate pharmaceutiquement acceptable.
